# EUROPEAN PATENT SPECIFICATION

(11) **EP 2 565 275 B1**
(45) Date of publication and mention of the grant of the patent: **20.04.2016**
(21) Application number: 12174786.9
(22) Date of filing: 29.06.2009
(51) Int. Cl.: C12Q 1/26, A61P 3/10, A61P 9/00, A61K 39/00, A61K 38/00, A61K 48/00, G01N 33/53, G01N 33/68

(54) **Method of treatment of vascular complications using modulators of TRX and TRXNIP**
Verfahren zur Behandlung vaskulärer Komplikationen mittels modulatoren von TRX und TRXNIP
Procédé de traitement de complications vasculaires en utilisant des modulateurs de TRX et TRXNIP

(30) Priority: 27.06.2008 US 76550 P; 01.07.2008 US 77261 P
(43) Date of publication of application: 06.03.2013
(62) Divisional of application: 09768642.2
(73) Proprietor: THE HEART RESEARCH INSTITUTE LIMITED, Camperdown, NSW 2050 (AU)
(72) Inventor: Ng, Martin Kean Chong, Mosman, New South Wales NSW 2088 (AU); Dunn, Louise Lorraine, Randwick, New South Wales NSW 2031 (AU); Buckle, Andrew, Berowra, New South Wales NSW 2081 (AU)
(74) Representative: Harrison, Susan Joan

(56) References cited:
- WO-A2-2006/078853
- US-A1- 2003 199 464
- SCHULZE P CHRISTIAN ET AL: "Hyperglycemia promotes oxidative stress through inhibition of thioredoxin function by thioredoxin-interacting protein", JOURNAL OF BIOLOGICAL CHEMISTRY, vol. 279, no. 29, 16 July 2004 (2004-07-16), pages 30369-30374, XP002654196, ISSN: 0021-9258
- DUNN L ET AL: "Rescue of Diabetes-related Impairment of Angiogenesis by Gene Silencing of Thioredoxin-interacting Protein", HEART, LUNG AND CIRCULATION, ELSEVIER, vol. 17, 1 January 2008 (2008-01-01), pages S3-S4, XP022851958, ISSN: 1443-9506, DOI: DOI:10.1016/J.HLC.2008.05.005 [retrieved on 2008-01-01]
- BUCKLE ET AL: "Hyperglycaemia Inhibits Thioredoxin-Mediated Angiogenesis: Implications for Impairment of Neovascularisation in Diabetes Mellitus", HEART, LUNG AND CIRCULATION, ELSEVIER, vol. 16, 1 January 2007 (2007-01-01), pages S214-S215, XP022198499, ISSN: 1443-9506, DOI: DOI:10.1016/J.HLC.2007.06.512
- DUNN L ET AL: "Type 1 Diabetes Mellitus is Associated with Impairment of Late Outgrowth Endothelial Progenitor Cell Function", HEART, LUNG AND CIRCULATION, ELSEVIER, vol. 17, 1 January 2008 (2008-01-01), page S138, XP022852280, ISSN: 1443-9506, DOI: DOI:10.1016/J.HLC.2008.05.327 [retrieved on 2008-01-01]
- DUNN L L ET AL: "Gene silencing of thioredoxin interacting protein dramatically rescues diabetes-related impairment of angiogenesis in vitro and in vivo", HEART, LUNG AND CIRCULATION, ELSEVIER, vol. 18, 1 January 2009 (2009-01-01), page S243, XP026304064, ISSN: 1443-9506, DOI: DOI:10.1016/J.HLC.2009.05.601 [retrieved on 2009-01-01]
- DATABASE BIOSIS [Online] BIOSCIENCES INFORMATION SERVICE, PHILADELPHIA, PA, US; 1994, CONRAD KEVIN ET AL: "Effect of verapamil and captopril on endothelial injury in the diabetic hypertensive rat", XP009165174, Database accession no. PREV199497453243 & AMERICAN JOURNAL OF HYPERTENSION, vol. 7, no. 7 PART 1, 1994, pages 629-636, ISSN: 0895-7061
- WILLIAM B WHITE ET AL: "Management of Patients with Hypertension and Diabetes Mellitus: Advances in the Evidence for Intensive Treatment", THE AMERICAN JOURNAL OF MEDICINE, vol. 108, no. 3, 15 February 2000 (2000-02-15), pages 238-245, XP055045709,
- XIANG GUOSHENG ET AL: "Catalytic degradation of vitamin D up-regulated protein 1 mRNA enhances cardiomyocyte survival and prevents left ventricular remodeling after myocardial ischemia", JOURNAL OF BIOLOGICAL CHEMISTRY, AMERICAN SOCIETY FOR BIOCHEMISTRY AND MOLECULAR BIOLOGY, US, vol. 280, no. 47, 1 November 2005 (2005-11-01), pages 39394-39402, XP009121913, ISSN: 0021-9258, DOI: 10.1074/JBC.M502966200
- M. K.C. NG ET AL: "A Central Role for Nicotinic Cholinergic Regulation of Growth Factor-Induced Endothelial Cell Migration", ARTERIOSCLEROSIS, THROMBOSIS, AND VASCULAR BIOLOGY, vol. 27, no. 1, 1 January 2007 (2007-01-01), pages 106-112, XP055045844, ISSN: 1079-5642, DOI: 10.1161/01.ATV.0000251517.98396.4a
- TSUTOMU KOBAYASHI ET AL: "Vitamin D3 up-regulated protein-1 regulates collagen expression in mesangial cells", KIDNEY INTERNATIONAL, vol. 64, 1 November 2003 (2003-11-01), pages 1632-1642, XP055045853,
- XIANG GUOSHENG ET AL: "Protection against cardiomyocyte apoptosis and functional cardiac recovery by downregulating VDUP1 expression", FASEB JOURNAL, FED. OF AMERICAN SOC. FOR EXPERIMENTAL BIOLOGY, US, vol. 18, no. 8, Supp. S, 1 May 2004 (2004-05-01), page C216, XP009105695, ISSN: 0892-6638
- SCHULZE P CHRISTIAN ET AL: "Vitamin D3-upregulated protein-1 (VDUP-1) regulates redox-dependent vascular smooth muscle cell proliferation through interaction with thioredoxin", CIRCULATION RESEARCH, AMERICAN HEART ASSOCIATION, INC, US, vol. 91, no. 8, 18 October 2002 (2002-10-18), pages 689-695, XP009121909, ISSN: 1524-4571, DOI: 10.1161/01.RES.0000037982.55074.F6
- CHRISTIAN SCHULZE P ET AL: "Hyperglycemia Promotes Oxidative Stress through Inhibition of Thioredoxin Function by Thioredoxin-interacting Protein", JOURNAL OF BIOLOGICAL CHEMISTRY, AMERICAN SOCIETY FOR BIOCHEMISTRY AND MOLECULAR BIOLOGY, US, vol. 279, no. 29, 16 July 2004 (2004-07-16), pages 30369-30374, XP002654196, ISSN: 0021-9258, DOI: 10.1074/JBC.M400549200 [retrieved on 2004-05-05]
- J. Yoshioka ET AL: "Targeted Deletion of Thioredoxin-Interacting Protein Regulates Cardiac Dysfunction in Response to Pressure Overload", Circulation Research, vol. 101, no. 12, 7 December 2007 (2007-12-07), pages 1328-1338, XP055187506, ISSN: 0009-7330, DOI: 10.1161/CIRCRESAHA.106.160515
- S. S. Sheth: "Thioredoxin-interacting protein deficiency disrupts the fasting-feeding metabolic transition", The Journal of Lipid Research, vol. 46, no. 1, 16 November 2004 (2004-11-16), pages 123-134, XP055187509, ISSN: 0022-2275, DOI: 10.1194/jlr.M400341-JLR200

## Description

### Field of the invention

The present invention relates to the field of therapy and prophylaxis of vascular complications and/or vascular disease associated with diabetes mellitus and other conditions associated with impaired glucose tolerance and/or hyperglycemia.

### Background of the invention

### Diabetes and vascular complications thereof

Diabetes afflicts an estimated 194 million people worldwide e.g., approximately 7.9% of Americans and approximately 7.8% of Europeans. Effective clinical management of diabetes is recognized as a growing problem, especially in societies where diet contributes to the growth of Type 2 diabetes e.g., Nesto, Rev. Card. Med.4, S11-S18 (2003). Between about 85% and 95% of all diabetics suffer from Type 2 diabetes, although nearly 5 million people worldwide suffer from Type 1 diabetes, affecting an estimated 1.27 million people in Europe and about 1.04 million people in the United States.

Type 1 diabetic patients and Type 2 diabetic patients are each at higher risk than non-diabetic subjects for a wide array of vascular complications including microvascular and macrovascular complications. Microvascular complications include, for example, neuropathy, retinopathy e.g., diabetic retinopathy, nephropathy e.g., diabetic kidney disease, impotence, and impaired or slow wound healing e.g., of ulcers. Macrovascular complications include, for example, extracranial and intracranial vascular disease e.g., contributing to ischemia and/or stroke, coronary heart disease, and peripheral vascular disease.

Neuropathy results in a loss of sensation in the peripheral limbs e.g., feet, exposing patients to undue, sudden or repetitive stress. Neuropathy can cause a lack of awareness of damage to limbs, and/or a risk of fissures, creating a potential entry for bacteria and increased risk of infection.

The peripheral vasculature of a diabetic patient may result in diabetic foot that affects the ability of a patient to walk and/or stand. Diabetic feet are at risk for a wide range of pathologies, including peripheral vascular disease, microcirculatory changes, neuropathy, ulceration, infection, deep tissue destruction and metabolic complications. The development of an ulcer in the diabetic foot is commonly a result of a break in the barrier between the dermis of the skin and the subcutaneous fat that cushions the foot during ambulation. This, in turn, can lead to increased pressure on the dermis, resulting in tissue ischemia and eventual necrosis and/or apoptosis, and ulceration. Progressive peripheral vascular disease in diabetic subjects may result in infection leading to gangrene, which is generally treated surgically by amputation. A foot ulcer precedes roughly 85% of all lower extremity amputations in diabetic patients, and about 15% of all diabetic patients will develop a foot ulcer during the course of their lifetimes. Infected and/or ischemic diabetic foot ulcers account for about 25% of all hospital days among people with diabetes, and the costs of foot disorder diagnosis and management are estimated at several billion dollars annually.

Diabetic retinopathy is an eye disease associated with diabetes wherein damage is caused to the blood vessels feeding the retina. Every person with diabetes will develop some degree of diabetic retinopathy which, without treatment may cause loss of vision and ultimately, blindness. Diabetic subjects are about 25 times more likely to lose vision or go blind than non-diabetic subjects. Temporal progression of diabetes increases the risk of diabetic retinopathy, however there may be no obvious symptoms of retinopathy in the early stages of diabetes e.g., in juveniles. In the early stages of diabetic retinopathy, there are also no apparent symptoms, and vision may not become impaired until the disease has progressed. There are two forms of retinopathy that can affect sight: (a) maculopathy, that develops when some of the small blood vessels around the macula become occluded, thereby causing other blood vessels to leak such that fluids build up around the macula causing swelling and loss of sight; and (b) proliferative retinopathy, that develops when blood vessels develop and/or grow abnormally on the surface of the retina e.g., into the centre of the eye, wherein the abnormal blood vessels may bleed, thereby impairing vision. In proliferative retinopathy, healing of blood vessels that have bled may result in scar tissue formation, thereby increasing surface tension of the retina resulting in retinal detachment, loss of vision or blindness.

Diabetic nephropathy is characterized by hyperproteinurea and it is believed that uncontrolled high blood sugar leads to the development of kidney damage. High blood sugar levels may damage the multiple tiny blood vessels or nephrons of the kidney, which help remove waste from the body. This may cause the nephrons to thicken and become scarred, and gradually they may become destroyed. Eventually this damage may cause the kidneys to leak and protein, including albumin, begins to pass into the urine.

It has been estimated that about 35-75% of men with diabetes may experience at least some degree of erectile dysfunction, or impotence, during their lifetime, which may be developed 10 to 15 years earlier than in healthy non-diabetic men. Erectile dysfunction in men with diabetes is likely to be caused by impairments in nerve, blood vessel and muscle function. To get an erection, men need healthy blood vessels, nerves, male hormones, and a desire to be sexually stimulated. However, diabetes may lead to damage of the blood vessels and nerves that control erection. Therefore, diabetic males may not be able to achieve a firm erection, even if an adequate amounts of male hormones and sexual stimulation are present.

Patients with diabetes often have wounds that are difficult to heal. Hyperglycemia, or an increased blood glucose level, is often the initial barrier to wound healing, such as ulcers, including foot ulcers, in diabetic subjects. The increased blood glucose level, may cause the cell walls to become rigid, impairing blood flow through the critical small vessels at the wound surface and impeding red blood cell permeability and flow. This impairs hemoglobin release of oxygen, which results in oxygen and nutrient deficits in the wound. Persistently elevated blood glucose levels may lead to compromise chemotaxis and phagocytosis immune functions, which also contributes to poor wound healing in subjects suffering from hyperglycemia. Chemotaxis is the process by which white cells are attracted to the site of an infection, and phagocytosis is the ingestion of bacteria by white cells. Both chemotaxis and phagocytosis are important in controlling wound infections. Diabetic infections take longer to heal because of delayed macrophage introduction and diminished leukocyte migration, which causes a prolonged inflammatory phase in the wound healing cascade.

The macrovascular complications of diabetes mellitus are characterized by accelerated atherosclerosis, leading to more aggressive coronary artery disease, cerebrovascular disease and peripheral vascular disease. Coronary heart disease involves a failure of coronary circulation to supply adequate circulation to cardiac muscle and surrounding tissue. Peripheral vascular disease (PAD) refers to diseases of blood vessels outside the heart and brain. It's often a narrowing of vessels that carry blood to the legs, arms, stomach or kidneys. Peripheral vascular disease may be short-term effects that may not involve defects in blood vessels' structure, or may be caused by structural changes in the blood vessels, such as inflammation and tissue damage. People with peripheral vascular disease often have atherosclerosis of other parts of the vasculature including the heart and brain. Because of this association, most people with PAD have a higher risk of death from heart attack and stroke. Chronic hyperglycemia, such as that in diabetic subjects, increases the risk macrovascular complications.

Notwithstanding the associations between diabetes and increased risks of atherosclerosis and poor outcomes following vascular occlusion, the precise mechanisms underlying impaired vascular function are poorly understood.

### Accepted animal models of endothelial cell dysfunction and/or diabetes

Accepted animal models of diabetes, atherosclerosis and/or endothelial cell dysfunction include mice fed a high fat diet, and various genetic murine models of the disease e.g., an animal comprising a genetic background that confers an obesity or diabetes syndrome such as animals having a genetic background selected from the group consisting of:
(i) yellow obese (*Ay*/*a*), a dominant mutation causes the ectopic, ubiquitous expression of the agouti protein, resulting in a condition similar to adult-onset obesity and non-insulin-dependent diabetes mellitus (Michaud et al., Proc Natl Acad. Sci USA 91: 2562-2566, 1994);
(ii) Obese (*ob*/*ob*) having a mutation in the gene encoding leptin (Zhang et al., Nature 372: 425-432, 1994);
(iii) diabetes (*db*/*db*) having a mutation in the gene encoding the leptin receptor (Tartaglia et al., Cell 83: 1263-1271, 1995);
(iv) adipose (*cpe*/*cpe*) having a mutation in the gene encoding carboxypeptidase E (Naggert et al., Nat. Genet. 10: 135-142, 1995);
(v) tubby (*tub*/*tub*) having a mutation in a member of a new family of genes encoding tubby-like proteins (Kleyn et al., Cell 85: 281-290, 1996; Noben-Trauth et al., Nature 380: 534-548, 1996);
(vi) Apolipoprotein E (ApoE) knockout mice fed a high fat diet, which are an accepted model of atherosclerosis;
(vii) Low density lipoprotein (LDL) receptor knockout mice fed a high fat diet, which are an accepted model of atherosclerosis
(viii) Tissue kallikrein (TK) knockout mice, which are accepted as a model for endothelial cell dysfunction; and
(ix) Angiotensin I converting enzyme (ACE) over-expressing mice, which are an accepted model of diabetic nephropathy.

Other animal models of diabetes mellitus involve pharmacological induction of diabetes by administration of agents such as streptozotocin, alloxan, vacor, dithizone or 8-hydrozyquinolone.

### Accepted animal models of vascular complications in diabetes

Any of the foregoing animal models of diabetes can provide models of vascular complications in diabetes by virtue of inducing hindlimb ischemia therein by art-recognized method(s).

Alternatively, or in addition, any one or more of the following experimental models may be employed:
(i) a permanent coronary occlusion;
(ii) coronary ischemia-reperfusion;
(iii) atherosclerosis plaque rupture;
(iv) wound healing in diabetic mice, wherein the wound is induced e.g., by dorsal skin puncture, dorsal incision, dorsal flap, etc;
(v) cardiomyopathy e.g., Non-Obese Diabetic (NOD) or Akita mice;
(vi) vein graft atherosclerosis model;
(vii) transplant atherosclerosis model;
(viii) aortic ring vascular reactivity *ex vivo*;
(ix) mesenteric microcirculation vascular reactivity *in vivo;* and
(x) flow-mediated dilation of arteries e.g., in rats.
(xi) animal models of angiogenesis e.g., matrigel plug, disc angiogenesis, atherosclerosis plaque/adventitial neovascularization and/or ischemia-mediated neovascularization such as hindlimb ischemia and/or myocardial infarction.

It follows that there are a large number of models for assessing diabetes and vascular complications thereof known in the art.

### Hyperglycemia and vascular complications thereof

Blood glucose concentration typically is maintained within a narrow range. An elevation in blood glucose concentration normally leads to an increased release of insulin, which then acts on target cells to increase glucose uptake.

Dysregulation of blood glucose homeostasis can lead to persistent elevated blood glucose concentration, or hyperglycemia. Some individuals with hyperglycemia may develop Type 2 diabetes.

Chronic hyperglycemia may result in diverse complications including any one or more of the vascular complications of diabetes *supra.* Effective clinical management of hyperglycemia is recognized as a growing problem, especially in societies where diet contributes to the growth of Type 2 diabetes e.g., Nesto, Rev. Card. Med.4, S11-S18 (2003).

### Treatment of vascular complications

US Pat. No. 6,608,094 to Torrent Pharmaceuticals Ltd., and US Pat. Publication No. 20010018524, US Pat. Publication No. 20030032660 and 20030092744, each disclose the use of specific pyridinium compounds for the treatment of vascular disease generally, wherein the compounds break cross-linkages in advanced glycation end (AGE) products.

US Pat. No. 4,590,200 to Pfizer, Inc. discloses the use of 2-(1-imidazolylmethyl)-3-methylbenzo[b]thiophene-5-carboxylic acid, and 3-methyl-2-(3-pyridylmethyl) benzo[b]thiophene-5-carboxylic acid as selective inhibitors of thromboxane synthetase for the treatment of vascular disease generally.

Schulze et al (J. Biol. Chem. Vol. 279, No. 29, pp. 30369-30374, 2004) and Yoshioka et al (Circ. Res. 2007; 101; 1328-1338) discuss the role of reactive oxygen species (ROS) in vascular disease. Yoshioka et al concludes that TXNIP plays a role in myocardial energy homeostasis rather than redox regulation.

There is a need in the art for compositions of matter that are selective for the treatment and prevention of vascular complications associated with diabetes and more generally hyperglycemia, in humans and other mammals, including those subjects suffering from or at risk of developing an indication selected from hypertriglyceridemia, hypercholesteremia, mixed dyslipidemia, vascular disease, artherosclerotic disease, obesity, insulin resistance, hyperglycemia, Type 1 diabetes, Type 2 diabetes, neuropathy, retinopathy, nephropathy, impotence and impaired/slow wound healing and combinations thereof.

### General

Conventional techniques of molecular biology, microbiology, virology, recombinant DNA technology, peptide synthesis and immunology are described, for example, in the following texts:
- Sambrook, Fritsch & Maniatis, Molecular Cloning: A Laboratory Manual, Cold Spring Harbor Laboratories, New York, Second Edition (1989), whole of Vols I, II, and III;
- DNA Cloning: A Practical Approach, Vols. I and II (D. N. Glover, ed., 1985), IRL Press, Oxford, whole of text;
- Oligonucleotide Synthesis: A Practical Approach (M. J. Gait, ed., 1984) IRL Press, Oxford, whole of text, and particularly the papers therein by Gait, pp1-22; Atkinson *et al.,* pp35-81; Sproat *et al.,* pp 83-115; and Wu *et al.,* pp 135-151;
- Animal Cell Culture: Practical Approach, Third Edition (John R.W. Masters, ed., 2000), ISBN 0199637970, whole of text;
- Perbal, B., A Practical Guide to Molecular Cloning (1984);
- Methods In Enzymology (S. Colowick and N. Kaplan, eds., Academic Press, Inc.), whole of series;
- J.F. Ramalho Ortigão, "The Chemistry of Peptide Synthesis" In: Knowledge database of Access to Virtual Laboratory website (Interactiva, Germany);
- Barany, G. and Merrifield, R.B. (1979) in The Peptides (Gross, E. and Meienhofer, J. eds.), vol. 2, pp. 1-284, Academic Press, New York.
- Bodanszky, M. (1984) Principles of Peptide Synthesis, Springer-Verlag, Heidelberg.
- Bodanszky, M. & Bodanszky, A. (1984) The Practice of Peptide Synthesis, Springer-Verlag, Heidelberg.
- Bodanszky, M. (1985) Int. J. Peptide Protein Res. 25, 449-474.
- Golemis (2002) Protein-Protein Interactions: A Molecular Cloning Manual(Illustrated), Cold Spring Harbor Laboratory, New York, ISBN 0879696281.
- Smith et al., (2002) Short Protocols in Molecular Biology: A Compendium of Methods from Current Protocols in Molecular Biology, 5th Edition (Illustrated), John Wiley & Sons Inc., ISBN 0471250929.
- Sambrook and Russell (2001) Molecular Cloning, Cold Spring Harbor Laboratory, New York, ISBN 0879695773.

### Summary of the invention

### 1. Introduction

In work leading up to the present invention, the inventors sought to elucidate the molecular mechanisms involved in vascular complications of hyperglycemia and diabetes. The inventors demonstrate that expression and/or activity and/or level of the redox regulatory protein thioredoxin ("TRX"; e.g., SEQ ID NO: 2) is implicated in pathogenesis of vascular disease e.g., vascular complications in hyperglycemic or diabetic subjects, and that a reduced expression and/or activity and/or level of TRX is regulated by increased expression and/or activity and/or level of its cognate binding partner, the thioredoxin interacting protein ("TXNIP"; e.g., SEQ ID NO: 4), thereby leading to pathogenesis e.g., in hyperglycemic and/or diabetic subjects. Proceeding on this basis, the inventors reasoned that such an impairment explains, at least in part, impaired vascular function of subjects suffering from diabetes and/or impaired glucose tolerance and/or hyperglycemia. The inventors reasoned further that a sub-population of endothelial progenitor cells (EPCs) known as late outgrowth endothelial cells (hereinafter "OEC") have impaired angiogenic activity and/or impaired neovascularization activity in diabetic and/or hyperglycemic subjects, thereby contributing to pathogenesis of vascular complications.

As exemplified herein, the present inventors have demonstrated that by reducing expression of TXNIP in isolated cells, many aspects of hyperglycemia-mediated endothelial cell dysfunction are alleviated e.g., endothelial cell function including their ability to migrate, proliferation, undergo hyperglycemia-induced cell death, and tubulogenesis/angiogenesis. The inventors have also demonstrated that, in accepted animal models of diabetes and ischemia it is possible to restore impaired blood flow in response to an ischemic event in a diabetic animal by reducing TXNIP expression.

The data provided herein demonstrate that TXNIP and the thioredoxin system are key regulators of cardiovascular homeostasis; that TXNIP is a glucose-regulated gene; that one or more vascular complications of diabetes are, in significant part, caused by hyperglycemia-mediated dysregulation of TXNIP; and that interruption to this hyperglycemia-mediated dysregulation of TXNIP provides an effective prophylactic and/or therapeutic for vascular complications of diabetes. As will be known to the skilled artisan, determination of one or more parameters of endothelial cell function or dysfunction provides a model for pathogenesis of vascular complications in diabetes, because endothelial cell function is a key component of vascular development and endothelial cell dysfunction and/or impaired functioning of the vascular endothelium are early indicators of vascular complications.

Without detracting from the general applicability of the invention to therapy and prophylaxis of vascular disease/complications, the data presented herein also clearly demonstrate that hyperglycemia induces a concentration-dependent inhibition of key angiogenic and/or neovascularization processes, including proliferation, migration and tubulogenesis of endothelial cells. For example, knockdown of TRX expression e.g., using siRNA that specifically targets TRX-encoding nucleic acid inhibits angiogenesis. Conversely, the inventors have also demonstrated that knockdown of TXNIP expression e.g., using siRNA that specifically targets TXNIP-encoding nucleic acid stimulates angiogenesis. Thus, the present inventors have also demonstrated that the thioredoxin system plays a key role in angiogenesis and neovascularization processes, and that knockdown of TXNIP expression and/or activity and/or level has utility in preventing one or more vascular complications from arising or developing and/or reducing the severity of one or more vascular complications and/or enhancing vascular repair e.g., by promoting, inducing or enhancing angiogenesis and neovascularization processes. The exemplification provided herein also supports a method of ameliorating the impairment of angiogenesis induced by hyperglycemia comprising reducing the expression of TXNIP in a cell e.g., e.g., using siRNA that specifically targets TXNIP-encoding nucleic acid. The exemplification provided herein also supports a method of reducing, delaying or suppressing hyperglycemia-induced apoptosis comprising reducing the expression of TXNIP in a cell e.g., using siRNA that specifically targets TXNIP-encoding nucleic acid.

The inventors also provide a demonstration that agents such as siRNA that specifically target TXNIP expression can alleviate i.e., lessen hyperglycemia-induced impairment of vascular endothelial growth factor (VEGF) production and/or function.

Also without detracting form the general applicability of the invention to therapy and prophylaxis of vascular disease/complications, the data presented herein also clearly demonstrate that, in diabetic subjects e.g., male subjects having insulin replacement therapy, the OEC sub-population exhibits markedly different morphology and/or proliferative potential or activity compared to non-diabetic subjects, and that this may also indicate reduced migration and/or tubulogenic potential or activity. Thus, isolated OECs isolated from diabetic subjects do not proliferate under standard EPC culture conditions to the same extent as OECs from non-diabetic subjects. These data indicate that OECs from juvenile Type 1 diabetes mellitus patients display aberrant morphology and reduced proliferative potential or activity compared to OECs from non-diabetic subjects, suggesting similar effects in hyperglycemic and Type 2 diabetic subjects.

Accordingly, the data herein are consistent with therapeutic function of TXNIP and/or TRX modulatory compounds e.g., TXNIP antagonists, inhibitory molecules or repressor molecules and/or TRX agonists, inducers or activators, in the prophylactic and/or therapeutic intervention of one or more vascular complications associated with diabetes and/or impaired glucose tolerance and/or hyperglycemia. The data herein are also consistent with the use of such modulatory compounds to produce formulations, e.g., topical, injectable and oral formulations comprising the modulatory compounds and/or cells, for prophylactic and/or therapeutic intervention in vascular complications of diabetes and/or impaired glucose tolerance and/or hyperglycemia and/or for promoting vascular repair.

Accordingly, in one example the present invention provides a composition for use in preventing or alleviating one or more vascular complication(s) in a subject suffering from diabetes and/or impaired glucose tolerance and/or hyperglycemia, wherein the composition comprises SiRNA that specifically targets a thioredoxin-interacting protein (TXNIP)-encoding nucleic acid and reduces expression of TXNIP. In another aspect, the present invention provides use of an SiRNA that specifically targets a thioredoxin-interacting protein (TXNIP)-encoding nucleic acid and reduces expression of TXNIP in the preparation of a medicament for the treatment of one or more vascular complications of diabetes mellitus and/or impaired glucose tolerance and/or hyperglycemia.

Also described herein is a method of prevention or treatment of one or more vascular complication(s) in a subject at risk of developing diabetes and/or impaired glucose tolerance and/or hyperglycemia or suffering from diabetes and/or impaired glucose tolerance and/or hyperglycemia, said method comprising administering to the subject an amount of a composition effective to induce, enhance or otherwise increase expression and/or activity and/or level of a thioredoxin (TRX) thereby preventing or alleviating one or more vascular complication(s) in the subject.

Also without detracting from the general applicability of the invention to therapy and prophylaxis of vascular disease/complications, the data presented herein are consistent with a prophylactic or therapeutic function of TXNIP and/or TRX modulatory compounds e.g., TXNIP antagonists, inhibitory molecules or repressor molecules and/or TRX agonists, inducers or activators, for mobilizing cells involved in vascular repair e.g., by mobilizing cells from a site at which they are administered to a site where they can participate in vascular repair. Alternatively, or in addition, in subjects suffering from myocardial infarct, treatment may enhance repair of the vasculature e.g., by enhancing angiogenesis and/or enhancing neovascularization.

Accordingly, in another example, described herein is a method of vascular repair in a subject comprising administering to the subject an amount of a composition effective to inhibit, repress, delay or otherwise reduce expression and/or activity and/or level of TXNIP thereby enhancing the ability of a cell to develop or proliferate into a functional endothelial cell (EC) or contribute to the formation of functional endothelium or blood vessels or otherwise promote vascular repair or enhance vascular repair.

In another example, described herein is a method of vascular repair in a subject comprising administering to the subject an amount of a composition effective to induce, enhance or otherwise increase expression and/or activity and/or level of TRX thereby enhancing the ability of a cell to develop or proliferate into a functional endothelial cell (EC) or contribute to the formation of functional endothelium or blood vessels or otherwise promote vascular repair or enhance vascular repair.

Also described herein is a method of promoting vascular repair in a subject comprising:
(i) administering to the subject an amount of a composition effective to inhibit, repress, delay or otherwise reduce TXNIP expression and/or activity and/or level; and
(ii) administering to the subject an amount of a composition effective to induce, enhance or otherwise increase expression and/or activity and/or level of TRX,
thereby enhancing the ability of a cell to develop or proliferate into a functional endothelial cell (EC) or contribute to the formation of functional endothelium or blood vessels or otherwise promote vascular repair or enhance vascular repair.

The therapeutic of the invention clearly extends to therapy of subjects that are asymptomatic for one or more vascular complications, albeit have one or more risk factors for one or more vascular complications of diabetes and/or impaired glucose tolerance and/or hyperglycemia and/or impaired vascular repair ability.

The present invention clearly also extends to therapy of a previously-diagnosed subject. For example, the therapeutic of the invention also extends to therapy of subjects having aberrant outgrowth endothelial cell (OEC) morphology and/or aberrant OEC proliferation potential and/or aberrant OEC proliferation activity and/or impaired endothelial cell function, and more particularly, adult and juvenile subjects suffering from Type 1 diabetes.

It is within the scope of the invention for a person to provide or obtain or recommend an amount of a therapeutic composition of the invention according to any example hereof.

It is also within the scope of the invention for a person to perform one or more of the following:
(i) identifying a subject suffering from diabetes and/or impaired glucose tolerance and/or hyperglycemia and/or one or more vascular complications thereof or is at risk of developing one or more vascular complications associated with diabetes and/or impaired glucose tolerance and/or hyperglycemia and/or having impaired vascular repair ability;
(ii) obtaining an amount of a composition of the invention according to any example hereof;
(iii) formulating a composition of the invention according to any example hereof with a suitable carrier and/or excipient, e.g., for oral administration, topical administration, inhalation, injection or infusion, wherein said composition is in an amount sufficient to alleviate or prevent one or more vascular complication(s) and/or in an amount sufficient to inhibit, repress, delay or otherwise reduce expression and/or activity and/or level of TXNIP and/or to induce, enhance or otherwise increase expression and/or activity and/or level of TRX;
(iv) administering a composition of the invention according to any example hereof to a subject; and/or
(v) recommending administration of a composition of the invention according to any example hereof.

Also described herein are cell-based and animal-based screens to identify and/or isolate therapeutics compounds and other compositions of matter.

In one example, the present application describes a method for identifying or isolating a compound or other composition of matter for the treatment or prophylaxis of one or more vascular complications in subjects with diabetes and/or impaired glucose tolerance and/or hyperglycemia and/or for promoting vascular repair and/or for enhancing vascular repair, said method comprising:
(i) providing a compound or other composition of matter that inhibits, represses, delays or otherwise reduces expression and/or activity and/or level of TXNIP and/or induces, enhances or otherwise increases expression and/or activity and/or level of TRX;
(ii) providing a cell to a cell that is capable of developing or proliferating into a functional EC or contributing to the formation of functional endothelium or blood vessels or participating in angiogenesis or neovascularisation or vascular repair under non-hyperglycemic conditions, wherein the compound;
(iii) rendering the cell at (ii) hyperglycemic in the presence and absence of the compound or other composition of matter at (i) and determining an ability of the compound to alleviate or attenuate impaired hyperglycemia-mediated function of the cell under hyperglycemic conditions; and
(iv) selecting a cell at (iii) having a less-impaired function under hyperglycemic conditions in the presence of the compound or other composition of matter at (iii) than a cell in the absence of the compound or other composition of matter at (iii); and
(v) isolating or identifying the compound or other composition of matter in the selected cell at (iv), thereby isolating or identifying a compound or other composition of matter for the treatment or prophylaxis of one or more vascular complications diabetes and/or impaired glucose tolerance and/or hyperglycemia and/or for promoting vascular repair and/or for enhancing vascular repair.

In another example, the application describes a method for identifying a compound or other composition of matter for the treatment or prophylaxis of one or more vascular complications in subjects with diabetes and/or impaired glucose tolerance and/or hyperglycemia and/or for promoting vascular repair and/or for enhancing vascular repair, said method comprising:
(i) identifying a compound or other composition of matter capable of inhibiting, repressing, delaying or otherwise reducing expression and/or activity and/or level of TXNIP and/or capable of inducing, enhancing or otherwise increasing expression and/or activity and/or level of TRX in a cell;
(ii) providing the compound or other composition of matter to a cell having impaired ability to develop or proliferate into a functional endothelial cell (EC) or contribute to the formation of functional endothelium or blood vessels or participate in angiogenesis or neovascularization or vascular repair, wherein said cell is capable of expressing TXNIP and/or TRX;
(iii) comparing an ability of the cells at (ii) to an ability of a cell to which the compound or other composition of matter has not been provided, wherein said ability is an ability to develop or proliferate into a functional EC or contribute to the formation of functional endothelium or blood vessels or participate in angiogenesis or neovascularisation or vascular repair; and
(iv) selecting a compound or other composition of matter that enhances an ability of cells to develop or proliferate into a functional EC or contribute to the formation of functional endothelium or blood vessels or participate in angiogenesis or neovascularisation or vascular repair, thereby identifying a compound or other composition of matter for the treatment or prophylaxis of one or more vascular complications diabetes and/or impaired glucose tolerance and/or hyperglycemia and/or for promoting vascular repair and/or for enhancing vascular repair.

In another example, the application describes a method for identifying a compound or other composition of matter for the treatment or prophylaxis of one or more vascular complications in subjects with diabetes and/or impaired glucose tolerance and/or hyperglycemia and/or for promoting vascular repair or enhancing vascular repair, said method comprising:
(i) identifying a compound or other composition of matter capable of inhibiting, repressing, delaying or otherwise reducing expression and/or activity and/or level of TXNIP and/or capable of inducing, enhancing or otherwise increasing expression and/or activity and/or level of TRX in a cell;
(ii) administering the compound or other composition of matter to an animal having impaired endothelial cell (EC) function and/or impaired ability to form functional endothelium or blood vessels and/or impaired vascular repair capability and/or suffering from one or more vascular complications of diabetes and/or impaired glucose tolerance and/or hyperglycemia or is at risk of suffering from one or more vascular complications of diabetes and/or impaired glucose tolerance and/or hyperglycemia or is otherwise in need thereof;
(iii) comparing a parameter indicative of endothelial cell proliferation or development or function and/or vascular endothelium development or function and/or vascular network growth or development at (ii) to the level of the parameter in an animal to which the compound or other composition of matter has not been administered; and
(iv) selecting a compound or other composition of matter that enhances endothelial cell proliferation or development or function and/or vascular endothelium development or function and/or vascular network growth or development, thereby identifying a compound or other composition of matter for the promoting vascular repair and/or enhancing vascular repair and/or treatment and/or prophylaxis of one or more vascular complications in subjects with diabetes and/or impaired glucose tolerance and/or hyperglycemia.

In another example, the application describes a method for isolating a compound or other composition of matter for the treatment or prophylaxis of one or more vascular complications in subjects with diabetes and/or impaired glucose tolerance and/or hyperglycemia and/or for promoting vascular repair or enhancing vascular repair, said method comprising:
(i) identifying a mixture of compounds or other compositions of matter capable of inhibiting, repressing, delaying or otherwise reducing expression and/or activity and/or level of TXNIP and/or capable of inducing, enhancing or otherwise increasing expression and/or activity and/or level of TRX in a cell or identifying a library comprising one or more compound or other compositions of matter capable of inhibiting, repressing, delaying or otherwise reducing expression and/or activity and/or level of TXNIP and/or capable of inducing, enhancing or otherwise increasing expression and/or activity and/or level of TRX in a cell;
(ii) providing said mixture or said one or more compounds or other compositions of matter identified at (i) to a cell having impaired ability to develop or proliferate into a functional EC or contribute to the formation of functional endothelium or blood vessels or participate in angiogenesis or neovascularisation or vascular repair, wherein said cell is capable of expressing TXNIP and/or TRX;
(iii) comparing an ability in the cells at (ii) to the ability in a cell to which the mixture or one or more compound or other compositions of matter has not been provided, said ability being an ability to develop or proliferate into a functional EC or contribute to the formation of functional endothelium or blood vessels or participate in angiogenesis or neovascularisation or vascular repair;
(iv) identifying a mixture or a plurality of compounds or other compositions of matter that enhances an ability of the cell to develop or proliferate into a functional EC or contribute to the formation of functional endothelium or blood vessels or participate in angiogenesis or neovascularisation or vascular repair; and
(v) separating from the mixture or plurality a compound or other composition of matter that enhances the ability to develop or proliferate into a functional EC or contribute to the formation of functional endothelium or blood vessels or participate in angiogenesis or neovascularisation or vascular repair, thereby isolating a compound or other composition of matter for the treatment or prophylaxis of one or more vascular complications in hyperglycemic and/or diabetic subjects and/or for promoting or enhancing vascular repair.

In another example, the application describes a method for isolating a compound or other composition of matter for the treatment or prophylaxis of one or more vascular complications in subjects with diabetes and/or impaired glucose tolerance and/or hyperglycemia and/or for promoting vascular repair or enhancing vascular repair, said method comprising:
(i) identifying a mixture of compound or other compositions of matter capable of inhibiting, repressing, delaying or otherwise reducing expression and/or activity and/or level of TXNIP and/or capable of inducing, enhancing or otherwise increasing expression and/or activity and/or level of TRX in a cell, or identifying a library comprising a plurality of compounds or other compositions of matter wherein at least one compound or other composition of matter is capable of inhibiting, repressing, delaying or otherwise reducing expression and/or activity and/or level of TXNIP and/or capable of inducing, enhancing or otherwise increasing expression and/or activity and/or level of TRX in a cell;
(ii) administering the mixture or plurality to an animal having impaired endothelial cell (EC) function and/or impaired ability to form functional endothelium or blood vessels and/or impaired vascular repair capability and/or suffering from one or more vascular complications of diabetes and/or impaired glucose tolerance and/or hyperglycemia or is at risk of suffering from one or more vascular complications of diabetes and/or impaired glucose tolerance and/or hyperglycemia or is otherwise in need thereof;
(iii) comparing a parameter indicative of endothelial cell proliferation or development or function and/or vascular endothelium development or function and/or vascular network growth or development at (ii) to the level of the parameter in an animal to which the mixture or plurality has not been administered;
(iv) identifying a mixture or a plurality of compounds or other compositions of matter that modulates a parameter indicative of endothelial cell proliferation or development or function and/or vascular endothelium development or function and/or vascular network growth or development in the animal to which the mixture or plurality is administered relative to an animal to which the mixture or plurality has not been administered; and
(v) separating a compound or other composition of matter from the mixture or plurality of compounds or other compositions of matter that enhances endothelial cell proliferation or development or function and/or vascular endothelium development or function and/or vascular network growth or development, thereby identifying a compound or other composition of matter for the promoting vascular repair and/or enhancing vascular repair and/or treatment and/or prophylaxis of one or more vascular complications in subjects with diabetes and/or impaired glucose tolerance and/or hyperglycemia.

The application further describes the direct product of a screen as described according to any example hereof e.g., an antagonist or inverse agonist of TXNIP activity or expression.

For prophylaxis and/or therapy, a subject for treatment will generally be identified by one or more risk factors including e.g., ischemia, heart attack, myocardial infarct, hypertriglyceridemia, hypercholesteremia, mixed dyslipidemia, obesity, insulin resistance, hyperglycemia, Type 1 diabetes, Type 2 diabetes, elevated TXNIP expression and/or activity and/or level, elevated TRX:TXNIP protein complexes, and reduced TRX expression and/or activity and/or level. For prophylactic and/or therapeutic interventions of existing vascular complications, the subject may also be identified by one or more risk factors *supra* if the vascular complication(s) exist at an early stage that is not readily apparent or other means e.g., impaired angiogenic function and/or impaired neovascularization, aberrant OEC morphology, aberrant OEC proliferative ability or combination thereof. For therapeutic intervention of existing vascular complications that are apparent in a subject may be identified by the vascular complication *per se* and/or one or more risk factors or other means *supra.*

The data herein also indicate the utility of one or more integers as prognostic indicators to identify subjects at risk of developing one or more vascular complications, wherein the one or more integers are selected from: (i) reduced TRX expression and/or activity and/or level; (ii) elevated TXNIP expression and/or activity and/or level; (iii) elevated TXNIP:TRX complex formation and/or level; (iv) elevated non-functional EPC count; (v) elevated count of impaired EPCs; and (vi) aberrant EPC morphology.

### 2. Further examples

In another example, the present application describes a method of promoting vascular repair in a subject comprising administering to the subject an amount of a composition effective to inhibit, repress, delay or otherwise reduce expression and/or activity and/or level of TXNIP thereby enhancing the ability of a cell to participate in vascular repair. For example, the ability of a cell to participate in vascular repair is enhanced by virtue of the composition enhancing mobilization of a cell to a site where it can participate in vascular repair and/or enhancing an angiogenic process or neovascularization process in the subject.

In accordance with this example, the composition may comprise an inverse agonist or antagonist of TXNIP expression and/or activity and/or level. In one example, the composition comprises a compound or plurality of compounds e.g., one or more peptides (e.g., aptamers, conformationally-constrained peptides, dominant-negative mutants and combinations thereof) and/or proteins and/or nucleic acids (e.g., antisense molecule, ribozyme, RNAi, shRNA or siRNA and mixtures thereof) and/or antibodies (e.g., monoclonal antibodies, polyclonal antibodies, recombinant antibodies including humanized recombinant antibodies and combinations thereof) and/or small molecules (e.g., chemical compounds that are TXNIP antagonists).

Also in accordance with this example, the composition may comprise a sufficient dosage of the inverse agonist or antagonist to promote vascular repair in a subject and/or to prevent damage to the vasculature in a subject. Alternatively, multiple doses may be administered to achieve therapeutic or prophylactic benefit.

Alternatively, or in addition, the composition of this example may comprise a cell capable of participating in vascular repair, including a cell that is capable of differentiation into a vascular cell, including a monopotent stem cell or multipotent stem cell or pluripotent stem cell. Endothelial progenitor cells (EPCs) may also be employed. The cell is functional with respect to vascular repair e.g., by virtue of being functional in nature, or by virtue of having been genetically-modified or genetically-repaired so as to be capable of participating in one or more vascular repair processes e.g., because it can migrate and proliferate to form blood vessels such as by angiogenesis and/or neovascularization.

For example, the composition may comprise a cell that comprises and/or expresses a modulator of TXNIP expression and/or activity and/or level such as: (i) a cell that has been produced by transfection or transduction or transformation with nucleic acid encoding a peptide or polypeptide modulator of TXNIP expression and/or activity and/or level e.g., an aptamer inhibiting TXNIP activity, a conformationally-constrained peptide inhibitor, a dominant-negative mutant of the wild-type TXNIP protein; (ii) a cell that has been produced by transfection or transduction or transformation with nucleic acid that modulates TXNIP expression e.g., an antisense molecule, ribozyme, RNAi, shRNA or siRNA.

In accordance with this example, described herein is one or both of (i) treating a cell to render it capable of participating in vascular repair or enhancing the ability of the cell to participate in vascular repair; and (ii) introducing the cell to the subj ect.

Alternatively, or in addition, the application describes isolating a cell capable of participating in vascular repair from a subject.

Cells, such as stem cells and/or endothelial progenitor cells (EPCs) administered to a subject are generally, but not necessarily, allogeneic (i.e., from the same species as a subject to be treated). For example, the cells may be isolated from a healthy i.e., non-diabetic subject or subject having no apparent impaired glucose tolerance or hyperglycemia, optionally expanded, and administered to a subject in need thereof. Allogeneic cells may also be autologous i.e., from the subject to be treated. If the cells are autologous (i.e., derived from the subject to which they are administered), they may be isolated from the subject, genetically-repaired or otherwise treated *ex vivo* as described herein to render them functional and re-introduced to the subject. Alternatively, functional cells are isolated from a subject to be treated, expanded *ex vivo,* and re-introduced to the subject. On the other hand, if the cells are allogenic, but not autologous, it is preferred for the cells to be from a subject of a similar tissue type e.g. have a similar MHC/HLA haplotype as the subject to whom they are to be administered.

The use of xenogeneic cells is also contemplated, particularly the use of porcine or primate e.g., orang-utan EPCs in humans.

Cells are preferably administered to a subject, including any re-introduction of autologous cells, in a de-differentiated state. The present application further describes incubating isolated cells capable of participating in vascular repair or enhancing one or more vascular repair processes for a time and under conditions sufficient to initiate one or more processes required for their differentiation into blood vessels. By "initiate" in this context is meant a sufficient number of biological steps to programme the cells or commit the cells to vascular differentiation. Such initiation may not exclude cell division.

In one example, the composition comprises a liquid suspension of the cells comprising or expressing a modulator of TXNIP expression and/or activity and/or level. The liquid suspension may be a suspension of the cells in a medium that contains appropriate compounds to promote or enhance vascular repair or one or more vascular repair processes, or to initiate their differentiation into an endothelial cell type capable of proliferating to form blood vessels e.g., in the formation of a vascular network or in vascular repair, such as by angiogenesis and/or neovascularization. Alternatively, or in addition, the suspension may comprise one or more compounds that discourage the differentiation of the cells into a cell type that is not useful.

Also in accordance with this example, a cellular composition may comprises a sufficient dosage of the cells to reduce, delay or prevent one or more vascular complications in a subject and/or to induce and/or enhance vascular repair in a subject. Alternatively, multiple doses may be administered to achieve therapeutic or prophylactic benefit.

The present application also describes a method of promoting vascular repair in a subject comprising administering to the subject an amount of a composition effective to induce, enhance or otherwise increase expression and/or activity and/or level of TRX thereby enhancing the ability of a cell to participate in vascular repair. For example, the ability of a cell to participate in vascular repair in enhanced by virtue of the composition enhancing mobilization of a cell to a site where it can participate in vascular repair and/or enhancing an angiogenic process or neovascularization process in the subject.

In accordance with this example, it is preferred to administer such an agonist or partial agonist of TRX expression and/or activity and/or level with (i.e., in the same therapeutic regimen but not necessarily concomitantly or simultaneously with) an inverse agonist or antagonist of TXNIP expression and/or activity and/or level for enhanced therapeutic benefit to the subject.

Also in accordance with this example, the composition comprises an agonist or partial agonist of TRX expression and/or activity and/or level. Exemplary compounds include e.g., one or a plurality of peptides (e.g., aptamers, conformationally-constrained peptides) and/or proteins and/or small molecules (e.g., chemical compounds that are TRX agonists) and combinations thereof.

Also in accordance with this example, the composition may comprise a sufficient dosage of the agonist or partial agonist of TRX, optionally with any inverse agonist or antagonist of TXNIP, to promote vascular repair or enhance the ability of a cell to participate in vascular repair. Alternatively, multiple doses may be administered to achieve therapeutic or prophylactic benefit.

Alternatively, or in addition, the composition of this example may comprise a cell capable of participating in vascular repair, including a cell that is capable of differentiation into a vascular cell, including a monopotent stem cell or multipotent stem cell or pluripotent stem cell. Endothelial progenitor cells (EPCs) may also be employed. The cell is functional with respect to vascular repair e.g., by virtue of being functional in nature, or by virtue of having been genetically-modified or genetically-repaired so as to be capable of participating in one or more vascular repair processes e.g., because it can migrate and proliferate to form blood vessels such as by angiogenesis and/or neovascularization.

For example, the composition may comprise a cell that comprises and/or expresses a modulator of TRX expression and/or activity and/or level such as: (i) a cell that has been produced by transfection or transduction or transformation with nucleic acid encoding a peptide or polypeptide modulator of TRX expression and/or activity and/or level e.g., an aptamer that is an agonist or partial agonist of TRX activity or a conformationally-constrained peptide agonist or partial agonist; (ii) a cell that has been produced by transfection or transduction or transformation with nucleic acid that modulates TRX expression e.g., a positive regulator of TRX expression.

In accordance with this example, the present application further describes one or both of (i) treating a cell to render it capable of participating in vascular repair or enhancing the ability of the cell to participate in vascular repair; and (ii) introducing the cell to the subj ect.

Alternatively, or in addition, the present application further describes isolating a cell capable of participating in vascular repair from a subject.

As with cells e.g., stem cells or EPCs, described herein above or otherwise having modulated TXNIP expression and/or activity and/or level, cells having modified TRX activity and/or expression and/or level may be allogeneic, autologous, or xenogeneic, and similar considerations apply with respect to their isolation, pre-treatment and administration or re-introduction to a subject in need thereof.

The present invention relates to a composition for use in prevention or treatment of one or more vascular complication(s) in a subject suffering from diabetes and/or impaired glucose tolerance and/or hyperglycemia, wherein the composition comprises SiRNA that specifically targets a TXNIP-encoding nucleic acid and reduces expression of TXNIP thereby preventing or alleviating one or more vascular complication(s) in the subject.

In one example, the composition comprises a plurality of compounds e.g., one or more peptides (e.g., aptamers, conformationally-constrained peptides, dominant-negative mutants and combinations thereof) and/or proteins and/or nucleic acids (e.g., antisense molecule, ribozyme, RNAi, shRNA or siRNA and mixtures thereof) and/or antibodies (e.g., monoclonal antibodies, polyclonal antibodies, recombinant antibodies including humanized recombinant antibodies and combinations thereof) and/or small molecules (e.g., chemical compounds that are TXNIP antagonists).

Also in accordance with this example of the invention, the composition may comprise a sufficient dosage to reduce, delay or prevent one or more vascular complications in a subject and/or promote vascular repair and/or enhance vascular repair in a subject. Alternatively, multiple doses may be administered to achieve therapeutic or prophylactic benefit.

In addition, the composition of this example may comprise a cell, such as a stem cell and/or an endothelial progenitor cell (EPC) that is functional e.g., by virtue of being functional in nature or by virtue of having been genetically-modified or genetically-repaired so as to be capable of proliferating to form blood vessels and/or promoting vascular repair and/or enhancing vascular repair e.g., in the formation of a vascular network or in vascular repair, such as by angiogenesis and/or neovascularization.

Also described herein is a method of prevention or treatment of one or more vascular complication(s) in a subject at risk of developing diabetes and/or impaired glucose tolerance and/or hyperglycemia or suffering from diabetes , impaired glucose tolerance and/or hyperglycemia, said method comprising administering to the subject an amount of a composition effective to induce, enhance or otherwise increase expression and/or activity and/or level of TRX thereby preventing or alleviating one or more vascular complication(s) in the subject.

In accordance with this example, it is preferred to administer such an agonist or partial agonist of TRX expression and/or activity and/or level with (i.e., in the same therapeutic regimen but not necessarily concomitantly or simultaneously with) an inverse agonist or antagonist of TXNIP expression and/or activity and/or level for enhanced therapeutic benefit to the subject.

Also in accordance with this example, the composition comprises an agonist or partial agonist of TRX expression and/or activity and/or level. Exemplary compounds include e.g., one or a plurality of peptides (e.g., aptamers, conformationally-constrained peptides) and/or proteins and/or small molecules (e.g., chemical compounds that are TRX agonists) and combinations thereof. The compounds may include GLP-1 agonists or mimetics.

Also in accordance with this example, the composition may comprise a sufficient dosage of the agonist or partial agonist of TRX, optionally with any inverse agonist or antagonist of TXNIP, to thereby reduce, delay or prevent one or more vascular complications in a subject and/or promote vascular repair and/or enhance vascular repair in a subject. Alternatively, multiple doses may be administered to achieve therapeutic or prophylactic benefit.

Alternatively, or in addition, the composition of this example may comprise a cell, e.g., a stem cell or EPC that is functional e.g., by virtue of being functional in nature or by virtue of having been genetically-modified or genetically-repaired so as to be capable of proliferating to form blood vessels and/or promoting vascular repair and/or enhancing vascular repair e.g., in the formation of a vascular network or in vascular repair, such as by angiogenesis and/or neovascularization. For example, the composition may comprise a cell that comprises and/or expresses a modulator of TRX expression and/or activity and/or level such as: (i) a cell that has been produced by transfection or transduction or transformation with nucleic acid encoding a peptide or polypeptide modulator of TRX expression and/or activity and/or level e.g., an aptamer agonizing TRX activity, or a conformationally-constrained peptide agonist of TRX; (ii) a cell that has been produced by transfection or transduction or transformation with nucleic acid that enhances TRX expression e.g., encoding a positive regulator of TRX expression.

The cells e.g., stem cells or EPCs, may further have reduced or repressed TXNIP expression and/or activity and/or level e.g., achieved by any means described herein for maximum benefit.

In accordance with this example, the present application further describes one or both of (i) treating a cell to render it functional so as to be capable of proliferating to form blood vessels and/or promoting vascular repair and/or enhancing vascular repair e.g., in the formation of a vascular network, such as by angiogenesis and/or neovascularization and (ii) introducing the cell to the subject. Optionally, there may be a further step of isolating a cell capable of proliferating to form blood vessels and/or promoting vascular repair and/or enhancing vascular repair from a subject.

As with cells e.g., stem cells or EPCs, described herein above or otherwise having modulated TXNIP and/or TRX expression and/or activity and/or level, cells useful in performing this example may be allogeneic, autologous, or xenogeneic, and similar considerations apply with respect to their isolation, pre-treatment and administration or re-introduction to a subject in need thereof. For example, a therapeutic/prophylactic cellular composition may comprises a sufficient dosage of cells to reduce, delay or prevent one or more vascular complications in a subject and/or to induce and/or enhance vascular repair in a subject. Alternatively, multiple doses may be administered to achieve therapeutic or prophylactic benefit.

In the foregoing examples, the diabetes is Type 1 diabetes mellitus. In another example, the diabetes is Type 2 diabetes mellitus.

A subject to which the present invention can be applied is a human or other mammalian subject capable of developing diabetes and/or impaired glucose tolerance and/or hyperglycemia, including e.g., a domesticated animal such as a domestic pet or commercially-valuable animal. The prophylactic or therapeutic treatment of a dog, cat or horse is clearly encompassed by the present invention.

The present invention clearly contemplates repeated administration of a composition as described according to any embodiment hereof in the therapy or prophylaxis of vascular complications of diabetes and/or impaired glucose tolerance and/or hyperglycemia. For example, repeated ingestion and/or injection and/or inhalation of a composition of the present invention may be required to reduce or prevent TXNIP expression and/or activity and/or level in the circulatory system for a prolonged period of time. In addition, optionally repeated ingestion and/or injection and/or inhalation of a composition of the present invention may be required to enhance or induce TRX expression and/or activity and/or level in the circulatory system for a prolonged period of time. Repeated administration may be timed so as to ensure a sufficiently high concentration of the bioactive composition in plasma of the subject and/or at the site of action in the treatment regimen. For example, second and/or subsequent doses may be administered at a time when serum concentration provided by one or more previous doses fall(s) below a desired level at which it is active or provides sufficient benefit to the patient. Such booster doses are clearly contemplated in the prophylaxis and/or therapy of one or more vascular complications of diabetes and/or impaired glucose tolerance and/or hyperglycemia and/or to promote vascular repair and/or to enhance vascular repair according to the present invention.

In another example, a method of treatment or prophylaxis as described herein according to any embodiment additionally comprises providing or obtaining an amount of a composition comprising a cell e.g., a stem cell or EPC or compound as described herein above.

For example, described herein is a method of treatment or prophylaxis of a subject in need thereof, said method comprising:
(i) identifying a subject suffering from diabetes and/or impaired glucose tolerance and/or hyperglycemia and/or one or more vascular complications thereof or is at risk of developing one or more vascular complications associated with diabetes and/or impaired glucose tolerance and/or hyperglycemia and/or a subject in need of vascular repair or enhanced vascular repair;
(ii) obtaining an amount of a composition according to any embodiment hereof; and
(iii) administering said composition to said subject.

In another example, described herein is a method of treatment or prophylaxis of a subject in need thereof, said method comprising:
(i) identifying a subject suffering from diabetes and/or impaired glucose tolerance and/or hyperglycemia and/or one or more vascular complications thereof or is at risk of developing one or more vascular complications associated with diabetes and/or impaired glucose tolerance and/or hyperglycemia and/or a subject in need of vascular repair or enhanced vascular repair; and
(ii) recommending administration of a composition according to any embodiment hereof.

Also described herein is a method of treatment or prophylaxis comprising administering or recommending a composition according to any embodiment hereof to a subject previously identified as suffering from diabetes and/or impaired glucose tolerance and/or hyperglycemia and/or one or more vascular complications thereof or otherwise at risk of developing one or more vascular complications associated with diabetes and/or impaired glucose tolerance and/or hyperglycemia and/or a subject identified as being in need of vascular repair or enhanced vascular repair.

Also described herein is a method of treatment or prophylaxis comprising:
(i) identifying a subject suffering from diabetes and/or impaired glucose tolerance and/or hyperglycemia and/or one or more vascular complications thereof or otherwise at risk of developing one or more vascular complications associated with diabetes and/or impaired glucose tolerance and/or hyperglycemia and/or a subject in need of vascular repair or enhanced vascular repair;
(ii) obtaining a composition according to any embodiment hereof;
(iii) formulating the composition at (ii) with a suitable carrier and/or excipient, e.g., for oral administration, topical administration, inhalation, injection or infusion, wherein said composition is in an amount sufficient to alleviate or prevent one or more vascular complication(s) according to any embodiment hereof in a subject in need thereof and/or in an amount sufficient to inhibit, repress, delay or otherwise reduce expression and/or activity and/or level of TXNIP and/or to induce, enhance or otherwise increase expression and/or activity and/or level of TRX; and
(iv) administering said formulation to said subject.

Also described herein is a method of treatment or prophylaxis comprising:
(i) identifying a subject suffering from diabetes and/or impaired glucose tolerance and/or hyperglycemia and/or one or more vascular complications thereof or otherwise at risk of developing one or more vascular complications associated with diabetes and/or impaired glucose tolerance and/or hyperglycemia and/or a subject in need of vascular repair or enhanced vascular repair;
(ii) obtaining a composition according to any embodiment hereof;
(iii) formulating the composition at (ii) with a suitable carrier and/or excipient, e.g., for oral administration, topical administration, inhalation, injection or infusion, wherein said composition is in an amount sufficient to alleviate or prevent one or more vascular complication(s) according to any embodiment hereof in a subject in need thereof in an amount sufficient to inhibit, repress, delay or otherwise reduce expression and/or activity and/or level of TXNIP and/or to induce, enhance or otherwise increase expression and/or activity and/or level of TRX; and
(iv) recommending a formulation at (iii).

In a particularly preferred example, the method of treatment or prophylaxis involves repeated administration, e.g., by injection, of a formulation comprising a compound or other composition of matter that modulates TXNIP and/or TRX expression and/or activity and/or level, wherein each administration is timed so as to ensure a sufficiently high concentration of the bioactive compound or other composition of matter of the formulation in plasma of the subject in the treatment regimen.

This application also describes an antagonist or inverse agonist of TXNIP activity or expression for treatment of a vascular complication of diabetes mellitus or for treatment of a vascular disease associated with diabetes mellitus or other condition associated with impaired glucose tolerance and/or hyperglycemia, or for promoting vascular repair in a subject in need thereof.

Preferred TXNIP antagonists or inverse agonists are nucleic acid inhibitors of TXNIP expression e.g., siRNAs or shRNAs, incretin mimetics or non-peptidyl GLP-1 agonists or recombinant e.g., humanized antibodies that bind to TXNIP *in vivo.* The incretin mimetic peptide exenatide or exendin-4, and/or the GLP1 receptor agonist(s) Boc5 and/or SP4 may be employed to promote vascular repair in a subject. The present invention relates to the use of SiRNA, according to tne claims. In accordance with these examples, it is preferred to the claims. formulate TXNIP antagonists for promoting vascular repairs, and preferentially promoting vascular repair compared to other effects or side-effects of the compound(s), as determined e.g., by the ability of the formulation to enhance mobilization of cells that participate in vascular repair and/or enhance angiogenic processes or neovascularisation processes. The TXNIP antagonist may be administered in a formulation comprising a pharmaceutically acceptable excipient or carrier e.g., a liposome. In one example, a TXNIP antagonist is formulated for administration by the intramuscular route e.g., once or twice daily. Preferably, the TXNIP antagonist is formulated for administration to a site which is in need of vascular repair e.g., skeletal muscle of peripheral organs, including wounded tissues. In another example, a compound is formulated for administration by the subcutaneous route e.g., once or twice daily. In another example, a compound is formulated for oral administration e.g., once or twice daily.

The present application also describes a method for identifying a compound or other composition of matter for the treatment or prophylaxis of one or more vascular complications in subjects with diabetes and/or impaired glucose tolerance and/or hyperglycemia and/or for promoting vascular repair or enhancing vascular repair, said method comprising:
(i) identifying a composition capable of inhibiting, repressing, delaying or otherwise reducing expression and/or activity and/or level of TXNIP and/or capable of inducing, enhancing or otherwise increasing expression and/or activity and/or level of TRX in a cell;
(ii) providing the compound or other composition of matter to a cell having impaired ability to participate in vascular repair and/or having impaired angiogenic and/or neovascularization potential and/or activity wherein said cell is capable of expressing TXNIP and/or TRX e.g., in a glucose-regulated manner;
(iii) comparing ability of the cell to participate in vascular repair and/or comparing angiogenic and/or neovascularization potential and/or activity in the cells at (ii) to the level of angiogenic and/or neovascularization potential and/or in a cell to which the compound or other composition of matter has not been provided; and
(iv) selecting a compound or other composition of matter that enhances ability of the cell to participate in vascular repair and/or that enhances angiogenic and/or neovascularization potential and/or activity in a cell, thereby identifying a compound or other composition of matter for the treatment or prophylaxis of one or more vascular complications diabetes and/or impaired glucose tolerance and/or hyperglycemia and/or for promoting vascular repair or enhancing vascular repair.

In one example, the cell is an endothelial cell e.g., human umbilical vein endothelial cells (hereinafter "HUVEC") or human coronary artery endothelial cells (hereinafter "HCAEC"). In another example, the cell is an endothelial progenitor cell (EPC) e.g., a primary EPC or cultured EPC from blood, umbilical vein, bone marrow or other tissue known to contain EPCs or of vascular lineage. Exemplary EPCs include OECs.

In another example, ability to participate in vascular repair and/or to enhance vascular repair and/or angiogenic and/or neovascularization potential and/or activity of cells is determined by a parameter selected from cell migration, proliferation, tubulogenesis, differentiation and combinations thereof.

In another example, the method additionally comprises administering the compound or other composition of matter to an animal in need of vascular repair and/or having diabetes and/or impaired glucose tolerance and/or hyperglycemia or otherwise in need thereof, wherein said animal has impaired vascular repair process(es) and/or suffers from one or more vascular complications of diabetes and/or impaired glucose tolerance and/or hyperglycemia or is at risk of suffering from one or more vascular complications of diabetes and/or impaired glucose tolerance and/or hyperglycemia. For example, the animal may be an accepted animal model for diabetes and vascular complications thereof e.g., as described herein above or in Example 7 hereof. In accordance with this example, the animal is preferably tested e.g., by comparing angiogenic and/or neovascularization potential and/or activity in the animal to which the compound or other composition of matter has been administered to the level of angiogenic and/or neovascularization potential and/or in an otherwise isogenic animal to which the compound or other composition of matter has not been administered. Compound or other compositions of matter that modulate angiogenic and/or neovascularization potential and/or activity in the animal to which the compound or other composition of matter has been administered are selected.

In another example, this method additionally comprises:
(v) optionally, determining the structure of the compound or other composition of matter;
(vi) optionally, providing the name or structure of the compound or other composition of matter; and
(vii) providing the compound or other composition of matter.

The present application also describes a method for identifying a compound or other composition of matter for the treatment or prophylaxis of one or more vascular complications in subjects with diabetes and/or impaired glucose tolerance and/or hyperglycemia and/or for promoting vascular repair or enhancing vascular repair, said method comprising:
(i) identifying a compound or other composition of matter capable of inhibiting, repressing, delaying or otherwise reducing expression and/or activity and/or level of TXNIP and/or capable of inducing, enhancing or otherwise increasing expression and/or activity and/or level of TRX in a cell;
(ii) administering the compound or other composition of matter to an animal having impaired vascular repair capability and/or suffering from one or more vascular complications of diabetes and/or impaired glucose tolerance and/or hyperglycemia or is at risk of suffering from one or more vascular complications of diabetes and/or impaired glucose tolerance and/or hyperglycemia or is otherwise in need thereof;
(iii) comparing a parameter indicative of vascular network growth and/or development at (ii) to the level of the parameter in an animal to which the compound or other composition of matter has not been administered; and
(iv) selecting a compound or other composition of matter that enhances vascular network growth and/or development in an animal, thereby identifying a compound or other composition of matter for the promoting vascular repair and/or enhancing vascular repair and/or treatment and/or prophylaxis of one or more vascular complications in subjects with diabetes and/or impaired glucose tolerance and/or hyperglycemia.

In one example, a parameter indicative of vascular network growth and/or development is selected separately or collectively from TXNIP expression, TXNIP activity, TXNIP level, TRX:TXNIP protein complex formation, TRX:TXNIP protein complex level, TRX expression, TRX activity, TRX level, angiogenic potential, angiogenic function, neovascularization potential, neovascularization level, EPC morphology, EPC proliferative ability, a microvascular complication, a macrovascular complication and combinations thereof. Exemplary microvascular complications are selected from the group consisting of impaired angiogenesis, impaired neovascularization, neuropathy, retinopathy, nephropathy, impotence, impaired wound healing, slow wound healing, and combinations thereof. Exemplary macrovascular complications are selected from the group consisting of extracranial vascular disease, intracranial vascular disease, ischemia, stroke, coronary heart disease, peripheral vascular disease and combinations thereof. Combinations of any one or more microvascular complications and/or macrovascular complications are clearly encompassed.

In another example, angiogenic and/or neovascularization potential and/or activity of cells is determined by a parameter selected from EPC migration, proliferation, tubulogenesis, and combinations thereof.

In another example, a parameter indicative of vascular network growth and/or development is determined in an endothelial cell e.g., HUVEC, HCAEC. In another example, a parameter indicative of vascular network growth and/or development is determined in an endothelial progenitor cell (EPC) e.g., a primary EPC or cultured EPC from blood, umbilical vein, bone marrow or other tissue known to contain EPCs or of vascular lineage. Exemplary EPCs include OECs.

In another example, this method additionally comprises:
(v) optionally, determining the structure of the compound or other composition of matter;
(vi) optionally, providing the name or structure of the compound or other composition of matter; and
(vii) providing the compound or other composition of matter.

The present application also describes a method for isolating a compound or other composition of matter for the treatment or prophylaxis of one or more vascular complications in subjects with diabetes and/or impaired glucose tolerance and/or hyperglycemia and/or for promoting vascular repair or enhancing vascular repair, said method comprising:
(i) identifying a mixture of compounds or other compositions of matter capable of inhibiting, repressing, delaying or otherwise reducing expression and/or activity and/or level of TXNIP and/or capable of inducing, enhancing or otherwise increasing expression and/or activity and/or level of TRX in a cell or identifying a library comprising one or more compound or other compositions of matter capable of inhibiting, repressing, delaying or otherwise reducing expression and/or activity and/or level of TXNIP and/or capable of inducing, enhancing or otherwise increasing expression and/or activity and/or level of TRX in a cell;
(ii) providing said mixture or said one or more compounds or other compositions of matter identified at (i) to a cell having impaired vascular repair ability and/or having impaired angiogenic and/or neovascularization potential and/or activity wherein said cell is capable of expressing TXNIP and/or TRX e.g., in a glucose-regulated manner;
(iii) comparing angiogenic and/or neovascularization potential and/or activity in the cells at (ii) to the level of angiogenic and/or neovascularization potential and/or in a cell to which the mixture or one or more compound or other compositions of matter has not been provided;
(iv) identifying a mixture or a plurality of compounds or other compositions of matter that enhances vascular repair ability and/or angiogenic and/or neovascularization potential and/or activity in a cell; and
(v) separating a compound or other composition of matter from the mixture or plurality of compounds or other compositions of matter that enhances vascular repair ability and/or angiogenic and/or neovascularization potential and/or activity in a cell, thereby isolating a compound or other composition of matter for the treatment or prophylaxis of one or more vascular complications in hyperglycemic and/or diabetic subjects and/or for promoting or enhancing vascular repair.

In one example, the cell is an endothelial cell e.g., HUVEC, HCAEC. In another example, the cell is an endothelial progenitor cell (EPC) e.g., a primary EPC or cultured EPC from blood, umbilical vein, bone marrow or other tissue known to contain EPCs or of vascular lineage. Exemplary EPCs include OECs.

In another example, vascular repair ability and/or angiogenic and/or neovascularization potential and/or activity of EPCs are/is determined by a parameter selected from EPC migration, proliferation, tubulogenesis, and combinations thereof.

In another example, the method additionally comprises administering the compound or other composition of matter to an animal having impaired vascular ability and/or diabetes and/or impaired glucose tolerance and/or hyperglycemia, wherein said animal suffers from one or more vascular complications of diabetes and/or impaired glucose tolerance and/or hyperglycemia or is at risk of suffering from one or more vascular complications of diabetes and/or impaired glucose tolerance and/or hyperglycemia. For example, the animal may be an accepted animal model for diabetes and vascular complications thereof e.g., as described herein above or in Example 7 hereof. In accordance with this example, the animal is preferably tested e.g., by comparing angiogenic and/or neovascularization potential and/or activity in the animal to which the compound or other composition of matter has been administered to the level of angiogenic and/or neovascularization potential and/or in an otherwise isogenic animal to which the compound or other composition of matter has not been administered. Compound or other compositions of matter that modulate angiogenic and/or neovascularization potential and/or activity in the animal to which the compound or other composition of matter has been administered are selected.

In another example, the present application describes a method for isolating a compound or other composition of matter for the treatment or prophylaxis of one or more vascular complications in subjects with diabetes and/or impaired glucose tolerance and/or hyperglycemia and/or for promoting vascular repair or enhancing vascular repair, said method comprising:
(i) identifying a mixture of compound or other compositions of matter capable of inhibiting, repressing, delaying or otherwise reducing expression and/or activity and/or level of TXNIP and/or capable of inducing, enhancing or otherwise increasing expression and/or activity and/or level of TRX in a cell, or identifying a library comprising a plurality of compounds or other compositions of matter wherein at least one compound or other composition of matter is capable of inhibiting, repressing, delaying or otherwise reducing expression and/or activity and/or level of TXNIP and/or capable of inducing, enhancing or otherwise increasing expression and/or activity and/or level of TRX in a cell;
(ii) administering the mixture or plurality to an animal having impaired vascular repair ability and/or suffering from one or more vascular complications of diabetes and/or impaired glucose tolerance and/or hyperglycemia or is at risk of suffering from one or more vascular complications of diabetes and/or impaired glucose tolerance and/or hyperglycemia;
(iii) comparing a parameter indicative of vascular network growth and/or development at (ii) to the level of the parameter in an animal to which the mixture or plurality has not been administered;
(iv) identifying a mixture or a plurality of compounds or other compositions of matter that modulates a parameter indicative of vascular network growth and/or development in the animal to which the mixture or plurality is administered relative to an animal to which the mixture or plurality has not been administered; and
(v) separating a compound or other composition of matter from the mixture or plurality of compounds or other compositions of matter that enhances vascular network growth and/or development in an animal, thereby isolating a compound or other composition of matter for the treatment or prophylaxis of one or more vascular complications in subjects with diabetes and/or impaired glucose tolerance and/or hyperglycemia and/or for promoting vascular repair or enhancing vascular repair.

In one example, a parameter indicative of vascular network growth and/or development is selected separately or collectively from TXNIP expression, TXNIP activity, TXNIP level, TRX:TXNIP protein complex formation, TRX:TXNIP protein complex level, TRX expression, TRX activity, TRX level, angiogenic potential, angiogenic function, neovascularization potential, neovascularization level, EPC morphology, EPC proliferative ability, a microvascular complication, a macrovascular complication and combinations thereof. Exemplary microvascular complications are selected from the group consisting of impaired angiogenesis, impaired neovascularization, neuropathy, retinopathy, nephropathy, impotence, impaired wound healing, slow wound healing, and combinations thereof. Exemplary macrovascular complications are selected from the group consisting of extracranial vascular disease, intracranial vascular disease, ischemia, stroke, coronary heart disease, peripheral vascular disease and combinations thereof. Combinations of any one or more microvascular complications and/or macrovascular complications are clearly encompassed.

In another example, vascular repair ability and/or angiogenic and/or neovascularization potential and/or activity of EPCs are/is determined by a parameter selected from EPC migration, proliferation, tubulogenesis, and combinations thereof.

In another example, a parameter indicative of vascular network growth and/or development is determined in an endothelial cell e.g., HUVEC, HCAEC. In another example, a parameter indicative of vascular network growth and/or development is determined in an endothelial progenitor cell (EPC) e.g., a primary EPC or cultured EPC from blood, umbilical vein, bone marrow or other tissue known to contain EPCs or of vascular lineage. Exemplary EPCs include OECs.

In the foregoing examples of method for isolation of a compound or other composition of matter for the treatment or prophylaxis of one or more vascular complications in diabetic and/or hyperglycemic subjects and/or subjects having impaired glucose tolerance and/or having impaired vascular repair capability, the method(s) additionally comprise(s):
(vi) optionally, determining the structure of the compound or other composition of matter;
(vii) optionally, providing the name or structure of the compound or other composition of matter; and
(viii) providing the compound or other composition of matter.

Preferably, separation comprises the use of any chemical or biochemical purification process known in the art to fractionate the mixture of plurality of compounds or other compositions of matter coupled with assaying the fractions produced for activity with respect to angiogenic activity and/or neovascularization, and selecting fractions having one ore more of said activities.

More preferably, separation comprises iterated use of any chemical or biochemical purification process known in the art to partially or completely purify a compound or other composition of matter from a mixture of plurality of compounds or other compositions of matter and assaying the fractions produced in each iteration of the process for activity with respect to angiogenic activity and/or neovascularization, and selecting at each iteration one or more fractions having one or more of said activities. Preferably, the process is repeated for n iterations wherein n is sufficient number of iterations to reach a desired purity of the compound or other composition of matter e.g., 50% or 60% or 70% or 80% or 90% or 95% or 99%. More preferably, the process is repeated for zero to about ten iterations. As will be known to the skilled artisan, such iterations do not require iteration of precisely the same purification processes and more generally utilize different processes or purification conditions for each iteration.

In the case of a library of compound or other compositions of matter displayed separately wherein each compound or other composition of matter is substantially pure prior to performance of the method, such isolation results in the separation of the compound or other composition of matter from other compound or other compositions of matter in the library that do not have the requisite activity. In this case, the term "separating" extends to determining the activity of one library component relative to another library component and selecting a compound or other composition of matter having the desired activity.

Also described herein is the direct product of any method of identification or isolation of a compound or other composition of matter described herein.

It is to be understood that an identified or isolated compound or other composition of matter in substantially pure form i.e., free from contaminants that might cause adverse side effects or contraindications or antagonize the activity of the active compound or other composition of matter, can be formulated into a medicament suitable for treatment of vascular complication(s) in hyperglycemic and/or diabetic subjects. Accordingly, also described herein is the use of a compound or other composition of matter as described according to any embodiment hereof for therapy or prophylaxis of one or more vascular complications in the manufacture of a medicament for the treatment of a vascular complication associated with diabetes and/or impaired glucose tolerance and/or hyperglycemia. Preferably, the compound or other composition of matter is used in an amount sufficient to enhance vascular network growth and/or development.

The present application also describes a method of diagnosing a predisposition for one or more vascular complications in a subject, said method comprising determining a risk factor using a sample from a subject wherein the risk factor is selected from the group consisting of: (i) reduced TRX expression and/or activity and/or level; (ii) elevated TXNIP expression and/or activity and/or level; (iii) elevated TXNIP:TRX complex formation and/or level; (iv) elevated non-functional EPC count; (v) elevated count of impaired EPCs; (vi) aberrant EPC morphology; and (vii) a combination of any one or more of (i) to (vi), and wherein the risk factor is indicative of a predisposition for one or more vascular complications in a subject.

Methods for determining one or more risk factors are apparent from the description herein or known to the skilled artisan.

The subject is generally a subject with no apparent vascular disease or other vascular complications.

### 3. General

This specification contains nucleotide and amino acid sequence information prepared using PatentIn Version 3.3. Each nucleotide sequence is identified in the sequence listing by the numeric indicator <210> followed by the sequence identifier (e.g., <210>1, <210>2, <210>3, etc). The length and type of sequence (DNA, protein (PRT), etc), and source organism for each nucleotide sequence, are indicated by information provided in the numeric indicator fields <211>, <212> and <213>, respectively. Nucleotide sequences referred to in the specification are defined by the term "SEQ ID NO:", followed by the sequence identifier (e.g., SEQ ID NO: 1 refers to the sequence in the sequence listing designated as <400>1).

The designation of nucleotide residues referred to herein are those recommended by the IUPAC-IUB Biochemical Nomenclature Commission, wherein A represents Adenine, C represents Cytosine, G represents Guanine, T represents thymine, Y represents a pyrimidine residue, R represents a purine residue, M represents Adenine or Cytosine, K represents Guanine or Thymine, S represents Guanine or Cytosine, W represents Adenine or Thymine, H represents a nucleotide other than Guanine, B represents a nucleotide other than Adenine, V represents a nucleotide other than Thymine, D represents a nucleotide other than Cytosine and N represents any nucleotide residue.

Throughout this specification, unless specifically stated otherwise or the context requires otherwise, reference to a single step, composition of matter, group of steps or group of compositions of matter shall be taken to encompass one and a plurality (i.e. one or more) of those steps, compositions of matter, groups of steps or group of compositions of matter.

Each embodiment described herein is to be applied *mutatis mutandis* to each and every other embodiment unless specifically stated otherwise.

Each embodiment describing a composition or compound or its use shall be taken to apply *mutatis mutandis* to a formulation comprising the composition or compound or its use unless the context requires otherwise or specifically stated otherwise.

Those skilled in the art will appreciate that the invention described herein is susceptible to variations and modifications other than those specifically described. It is to be understood that the invention includes all such variations and modifications, to the extent that they fall within the claims.

The following definitions are also provided:

As used herein, the term "administer" shall be taken to mean that a composition that modulates TRX and/or TXNIP activity and/or expression and/or level is provided or recommended to a subject in need thereof e.g., in a single or repeated or multiple dosage by any administration route. In one example, the composition is administered by oral means e.g., as a tablet, capsule, liquid formulation. In another example, the composition is administered by inhalation or apsiration e.g., as a powder via the respiratory system of the subject including the nasal passage, buccal cavity, throat or eosophagus or lung. In another example, the composition is administered to the circulatory system of a subject by injection e.g., intramuscularly, subcutaneously, intravenously, intraperitoneally.

The term "alleviating" or "alleviate" as used throughout this specification shall not be taken to require abrogation of impaired vascular function or vascular complication in a subject that is more than a significant effect compared to the absence of treatment in accordance with the present invention.

The term "angiogenesis" shall be taken to mean a process by which new blood vessels are formed whether from extant blood vessels or not and/or the enlargement of pre-existing blood vessels. Determination of angiogenesis may be by measuring capillary density and/or cell proliferation and/or cell migration and/or tubulogenesis and/or the level of one or more known markers of angiogenesis e.g., sphingosine-1-phosphate or SPP (Lee et al., Cell 99, 301-312, 1999), sphingosine kinase, vascular endothelial growth factor (VEGF), fibroblast growth factor (FGF), insulin-like growth factor (IGF), an angiopoietin, platelet-derived endothelial growth factor (PD-EGF), transforming growth factor (TGF) e.g., TGF-β1, hypoxia-induced factor-alpha (HIF1-α), nitric oxide synthase, monocyte chemoattractant protein-1 (MCP-1), interleukin-8 (IL-8), an ephrin, neutrophil-activating protein-2 (NAP-2), epithelial neutrophil-activating peptide (ENA-78), a fragment of tyrosyl-tRNA synthetase.

The term "cell" in the broad context of a process of the invention as described according to any example hereof shall be take to includes without limitation any cell, and more specifically to at least include a cell selected from a stem cell, endothelial progenitor cell, cardiomyocyte, skeletal muscle cell, smooth muscle cell, endothelial cell, blood-derived cell, fibroblast or hepatocyte, or a mixture thereof.

The term "compound" means a discrete chemical entity e.g., peptide, protein, nucleic acid, antibody, small molecule, or a plurality of discrete chemical entities. The term "other composition of matter" includes e.g., EPCs and other cell types of therapeutic/prophylactic benefit.

The word "comprise", or variations such as "comprises" or "comprising", will be understood to imply the inclusion of a stated step or element or integer or group of steps or elements or integers but not the exclusion of any other step or element or integer or group of elements or integers.

The term "derived from" shall be taken to indicate that a specified integer may be obtained from a particular source albeit not necessarily directly from that source.

The term "diabetes" shall be taken to mean a diabetes characterized by one or more of impaired glucose tolerance, insulin resistance, and hyperglycemia e.g., including diabetes mellitus but not including diabetes insipidus. Examples of diabetes in this context include Type 1 diabetes and Type 2 diabetes.

The term "endothelial progenitor cell" or "EPC" shall be construed in its broadest context to mean a cell that, when functional or normal, is capable of proliferating to form blood vessels e.g., in the formation of a vascular network or in vascular repair, such as by angiogenesis and/or neovascularization. EPCs may be functional, non-functional, or have impaired function e.g., with respect to this proliferative ability and developmental capability, and non-functional EPCS or EPCs having impaired function may be identified readily e.g., by their aberrant morphology and impaired proliferative ability.

The term "enhance" or similar in the context of vascular repair shall be construed in its broadest context to include a change to any process that enhances the ability of a cell to contribute to vascular repair and/or induces, initiates, de-represses or otherwise enhances vascular repair e.g., by mobilizing cells such that they can participate in vascular repair and/or angiogenesis and/or neovascularization. The term "inhibit", "enhance", "de-repress", "initiate" or "induce", as used throughout this specification shall not be taken to require any particular quantitative change, merely a modified level and/or activity and/or expression, or modified timing thereof, that is significant compared to the absence of treatment in accordance with the present invention.

The term "neovascularization" shall be taken to mean a development of new blood vessels in tissues not possessing extant blood vessels.

The term "prevent" or derivations thereof shall be taken to indicate a delay or inhibition of initiation of one or more symptoms of vascular disease or other stated condition, and is not be taken to require an absolute i.e., 100% prevention of the development of a vascular complication in a subject having risk factors therefor. It is sufficient that there is a significant delay or inhibition of initiation in one or more symptoms using the method of the present invention compared to the absence of prophylaxis.

The term "promote" or similar in the context of vascular repair shall be construed in its broadest context to include the initiation of any process that initiates or activates a vascular process e.g., by mobilizing cells such that they can participate in vessel formation, vascular repair and/or angiogenesis and/or neovascularization. The terms "promote" and "initiate" and "activate" as used throughout this specification shall not be taken to require any particular quantitative change, merely a modified level and/or activity and/or expression, or modified timing thereof, that is significant compared to the absence of treatment in accordance with the present invention.

The term "small molecule" shall be taken to mean any organic or inorganic compound that is not a nucleic acid, protein, or peptide. The structure of small molecules varies considerably as do methods for their synthesis. The present invention contemplates the use of any small molecule(s) or small molecule library.

The term "stem cell" shall be construed in its broadest context to comprise a any progenitor cell type including monopotent, multipotent, pluripotent cells or induced pluripotent stem cells (iPSC) e.g., embryonic stem cells (ESC) or adult stem cells, such as multipotent adult progenitor cells (MAPC), haematopoeitic stem cells (HSC), mesenchymal stem cells (MSC), endothelial progenitor cells (EPC), skeletal muscle satellite cells, smooth muscle stem cells, cardiac stem cells, pluripotent stem cells, and stem cells from hepatic, renal, gut, pancreatic, neural, skin, ovarian/testicular tissue or teeth.

The term "subject in need" or similar shall be taken to mean a subject that has developed or suffers from or one or more symptoms of vascular complications of diabetes and/or impaired glucose tolerance and/or hyperglycemia, and/or is predisposed by virtue of having one or more risk factors to suffering from diabetes and/or impaired glucose tolerance and/or hyperglycemia. In one example, the subject has already suffered from diabetes and/or impaired glucose tolerance and/or hyperglycemia or one or more vascular complications thereof and/or has aberrant OEC morphology and/or OEC proliferation potential and/or activity, such as a subject suffering from Type 1 diabetes or Type 2 diabetes, in particular a juvenile subject suffering from diabetes e.g., Type 1 diabetes or Type 2 diabetes. In another example, the subject has not yet suffered apparent impairment of vascular function or any apparent vascular complication, however has one or more risk factors for a vascular complication of diabetes and/or impaired glucose tolerance and/or hyperglycemia. In another example, the subject is a subject in need of vascular repair or enhanced vascular repair.

The term "thioredoxin" or "TRX" shall be taken to mean a peptide, polypeptide or protein that is functionally equivalent and/or structurally-related to a human TRX polypeptide comprising the sequence set forth in SEQ ID NO: 2 or encoded by a nucleotide sequence set forth in SEQ ID NO: 1 or a homolog, analog or derivative thereof, including the TRX1 or TRX2 isoform.

The term "thioredoxin interacting protein" or "TXNIP" or "VDUP" shall be taken to mean a peptide, polypeptide or protein that is functionally equivalent and/or structurally-related to a human TXNIP polypeptide comprising the sequence set forth in SEQ ID NO: 4 or encoded by a nucleotide sequence set forth in SEQ ID NO: 3 or a homolog, analog or derivative thereof.

The term "treat" or derivations thereof shall be taken to mean a reduction in severity of a pre-existing symptoms of vascular disease or impaired vascular function, and is not be construed as requiring an absolute i.e., 100% abrogation of impaired vascular function or vascular complication. It is sufficient that there is a significant reduction in the adverse vascular effect(s) using the method of the present invention compared to the absence of therapy.

The term "vascular complication" shall be taken to mean any one or a combination of vascular complications associated with diabetes and/or impaired glucose tolerance and/or hyperglycemia referred to herein such as, for example, injury and/or damage to the endothelium, a microvascular or macrovascular complication and/or impaired angiogenesis and/or impaired neovascularization, including e.g., neuropathy, retinopathy (e.g., diabetic retinopathy), nephropathy (e.g., diabetic kidney disease), impotence, impaired or slow wound healing e.g., of ulcers, extracranial vascular disease, intracranial vascular disease, ischemia, stroke, coronary heart disease, peripheral vascular disease and combinations thereof.

### Brief description of the drawings:

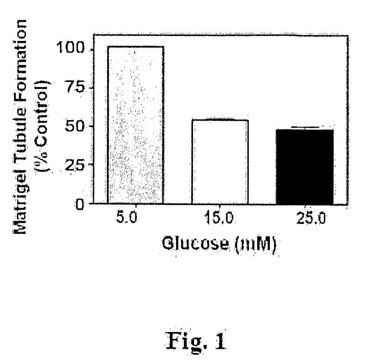

**Figure 1** is a graphical representation showing glucose concentration-dependent inhibition of vascular network formation as determined by Matrigel assay of HCAECs.

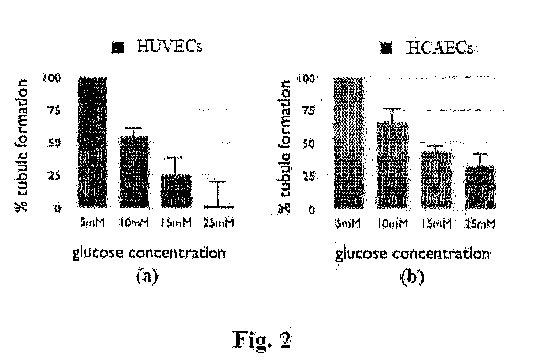

**Figure 2** is a graphical representation showing percentage of tubule formation following incubation of HUVECs (panel A) and HCAECs (panel B) in media containing 5, 10, 15 or 25 mM D-glucose for a period of 24 h in well plates coated with growth factor-reduced Matrigel. After 24 h photomicrographs were taken and tubule formation analysed using Image J software.

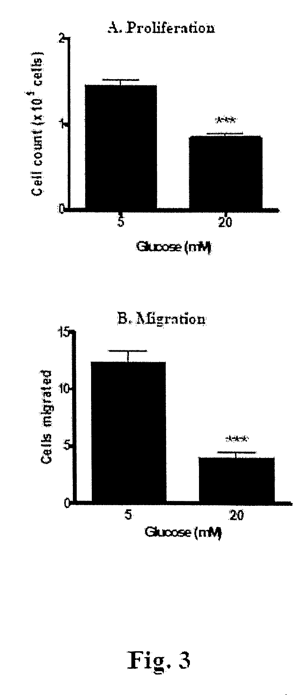

**Figure 3** is a graphical representation showing proliferation (panel A) and migration (panel b) of HUVECs cultured in media comprising 5 mM glucose ("normoglycemic" or non-hyperglycemic) or 20 mM glucose (hyperglycemic) for 24 h at which time they were replated for angiogenic assays for a further 24h, wherein the normoglycemic or hyperglycemic concentrations are continues throughout the angiogenic assays. Data shown in panel A and panel B are representative of at least three separate experiments and are expressed as mean ± SEM with ***p<0.05.

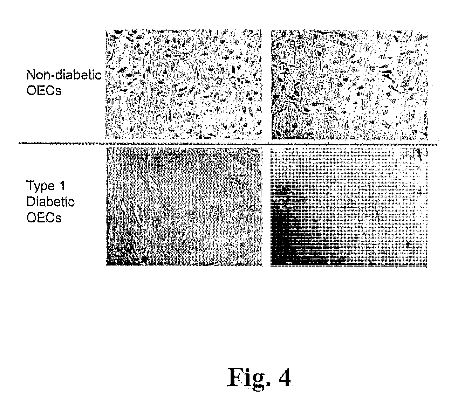

**Figure 4** is a photographic representation illustrating late Outgrowth Endothelial Cells (OECs) isolated from young (<35 years-old) male Type 1 diabetes mellitus (DM) patients (lower panel) and from sex- and age- matched non-diabetic healthy controls (top panel). EPCs were isolated from whole peripheral blood and cultured on collagen-coated plates for 14-21 days via standard techniques, at which stage OEC colonies emerged. Data show that late OECs from young Type 1 diabetes mellitus (DM) patients exhibit marked morphological differences compared to healthy control cells. Type 1 diabetic OECs do not form distinct colonies and do not maintain their growth velocity in culture compared to healthy control OECs.

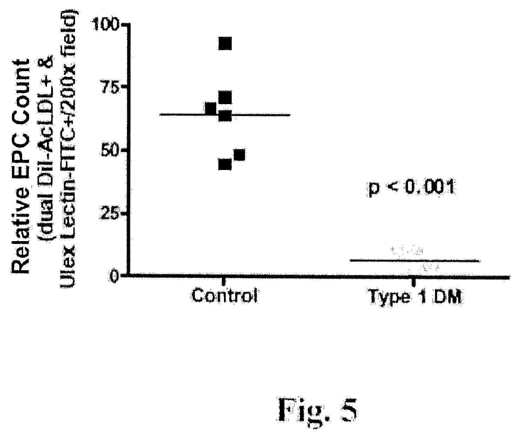

**Figure 5** is a graphical representation showing an analysis of Endothelial Progenitor Cell (EPC) count in peripheral whole blood of young Type 1 diabetes mellitus (DM) patients compared with sex- and age-matched controls. Young (<35 years-old) male type 1 DM patients with no other confounding factors were recruited along with sex- and age-matched controls. Data show that DM patients have reduced EPC count.

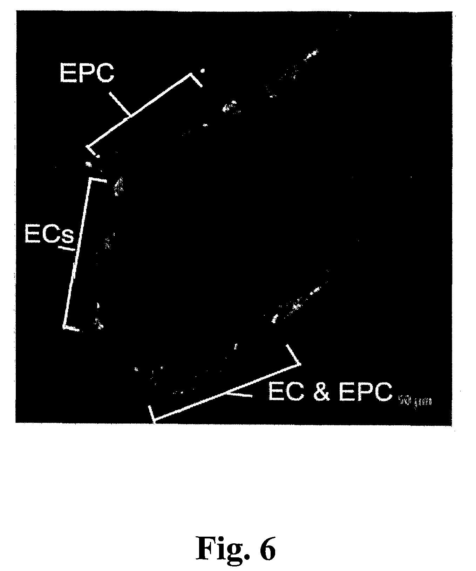

**Figure 6** is a photographic representation of an immunostained micrograph showing tubulogenesis *in vitro* of healthy human non-diabetic and diabetic endothelial progenitor cells (EPCs) and mature endothelial cells (ECs) on fibroblast monolayers. EPC tubulogenesis assay involves co-plating of 2x10⁴ DiI-labeled EPCs with 4x10⁴ HCAECs and primary human fibroblasts (MRC-5 cells, ATCC) in EGM-2 medium (Clonetics). After 72h, incorporation of EPCs into tubules was quantified by immunostaining and microscopy in 10 random fields. Positions of EPC and EC are indicated.

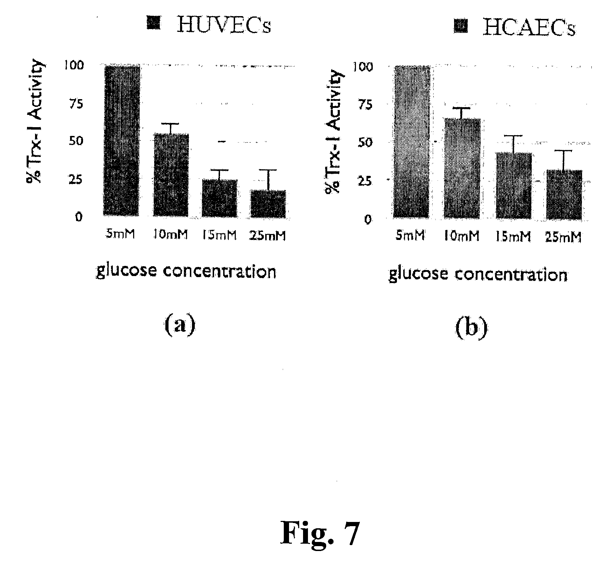

**Figure 7** is a graphical representation showing glucose concentration-dependent reduction in thioredoxin activity as percentage of control in HUVECs [panel (a)] and HCAECs [panel (b)] incubated in media containing 5mM, 15mM and 25mM D glucose for a period of 24 h.

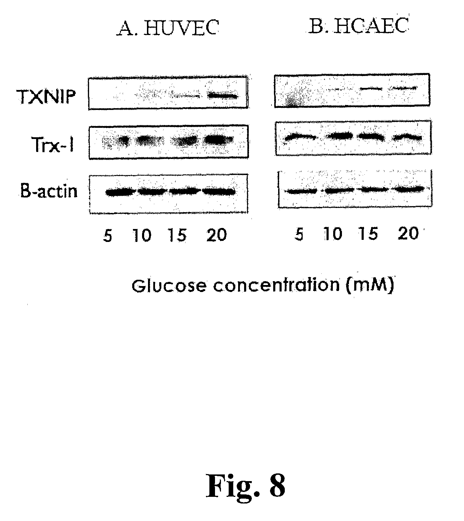

**Figure 8** provides photographic representations showing glucose concentration-dependant expression of TXNIP, TRX1 and beta-actin (β-actin) by HUVECs (panel A) and HCAECs (panel B), as determined by Western blot analysis. HUVECs and HCAECs were incubated in media containing 5mM, 10mM, 15mM or 20 mM D-glucose for a period of 24 h. After incubation cells were lysed and the protein isolated and quantified. Western blot analysis was performed on cell lysates using a rabbit-anti-VDUP1/TXNIP antibody specific for TXNIP.

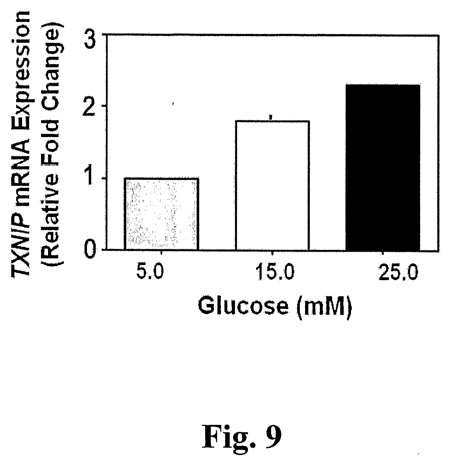

**Figure 9** is a graphical representation showing glucose concentration-dependent increase in TXNIP mRNA expression by HCAECs, demonstrating that hyperglycemia induces TXNIP mRNA expression.

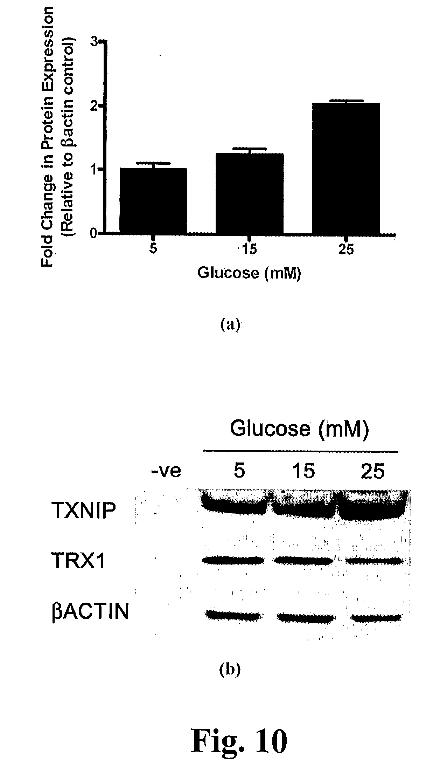

**Figure 10** provides a graphical representation (panel A) and a photographic representation (panel B) of a Western blot analysis of glucose concentration-dependent increase in TXNIP protein expression by HUVECs relative to beta-actin (β-actin). The data demonstrate that hyperglycemia induces TXNIP protein expression in HUVECs. HUVECs were cultured in 5mM, 15mM, or 25 mM glucose for a 24 h period. Protein was isolated from cells using the Mammalian Cell Lysis Kit (Sigma-Aldrich) and Western blot analysis performed using established techniques (Invitrogen). Membranes were probed for both human and murine proteins using antibodies against VDUP1/TXNIP (Invitrogen), TRX1 (Abcam), and actin (Sigma-Aldrich) as per the manufacturer's protocol. Results are expressed as mean ± SEM and are representative of at least three experiments performed on three individual occasions.

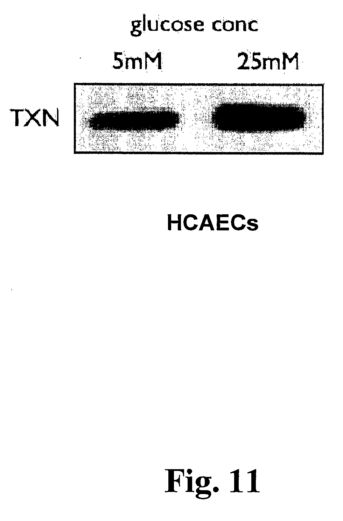

**Figure 11** is a photographic representation showing glucose-dependant increase in association between TRX1 and TXNIP proteins in HCAECs, as demonstrated by Western blot analysis of immunoprecipitates. HCAECs were cultured in media containing 5 mM D-glucose (control) or 25mM D-glucose (hyperglycemic conditions), the protein complexes immune precipitated using anti-TXNIP antibodies, resolved by gel electrophoresis, transferred to membranes and probed with anti-TRX antibodies. The data demonstrate that TXNIP binding to TRX-1 increases in hyperglycemic cells compared to cells cultured in 5mM D-glucose (normal glycemic conditions).

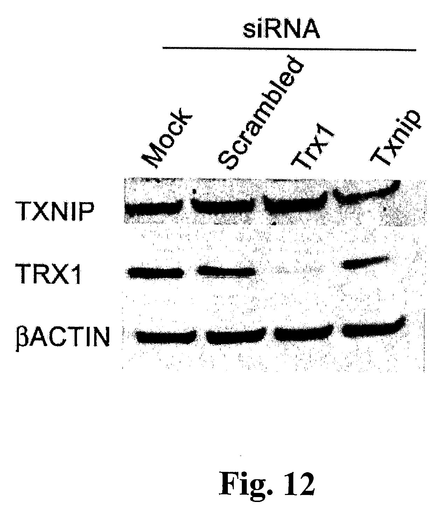

**Figure 12** is a diagrammatic representation showing Western blot analysis of HUVEC cells transfected with siRNAs to specifically silence TRX1 or TXNIP expression. Double-stranded (ds)-siRNA (SEQ ID NOs: 5 and 6) for specific silencing of human *TRX1* mRNA (e.g., SEQ ID NO: 1) or *TXNIP* mRNA (e.g., SEQ ID NO: 3) were transfected in HUVECs. A non-specific 'scrambled' ds-siRNA (SEQ ID NO: 7) was used as a negative control. After 24 h transfected protein was isolated from cells and Western blot analysis performed. Membranes were probed for human proteins using antibodies against TXNIP (Invitrogen), TRX1 (Abcam), and β-actin (Sigma-Aldrich) as per the manufacturer's protocol. This figure is representative of at least three separate experiments performed on three individual occasions.

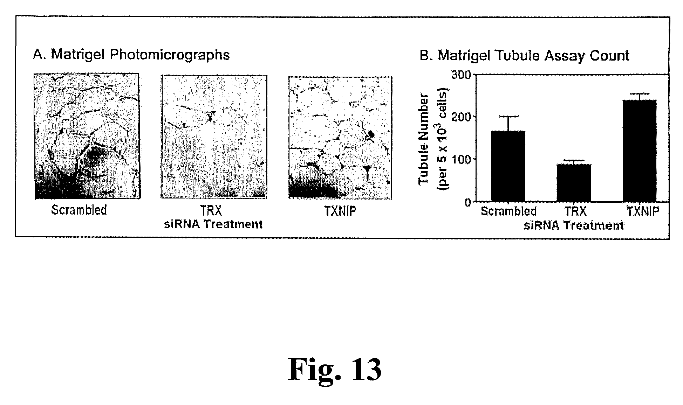

**Figure 13a** provides a photographic representation of the effects of TRX/TXNIP modulation on vascular network formation in HCAECs, assessed using a growth factor-reduced Matrigel assay following a gene knockdown of TRX1 or gene knockdown of TXNIP by siRNA and as demonstrated by Matrigel assay photomicrographs. Data show that gene knockdown of TRX1 reduces tubule formation, while gene-knockdown of TXNIP increases tubule formation.
**Figure 13b** provides a graphical representation of the effects of TRX/TXNIP modulation on vascular on vascular network formation in HCAECs as determined by tubule number in a growth factor-reduced Matrigel assay following siRNA-mediated knockdown of TRX1 or TXNIP expression.

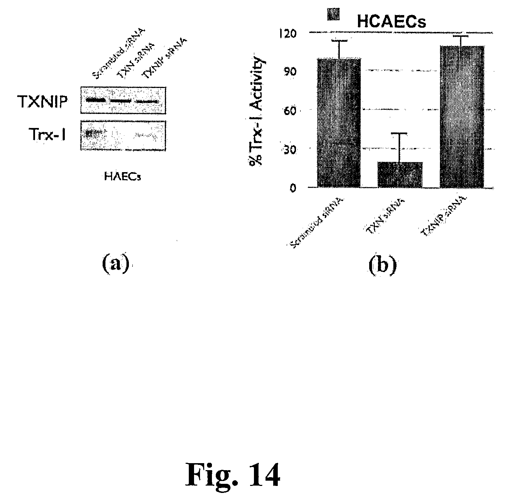

**Figure 14** provides representations showing the effect of siRNA-mediated gene silencing on TRX1 and TXNIP expression and activity. Panel (a) shows photographic representations of Western blots showing TXNIP protein and TRX-1 protein level. Western blot analysis of resolved cell lysates transferred to membranes was performed using a rabbit-anti-VDUP1/TXNIP antibody specific for TXNIP or a rabbit-anti-TRX1 antibody specific for the TRX1 as the primary antibody. Panel (b) provides a graphical representation showing the effect of the effect of siRNAs TRX1 activity for samples normalized according to their protein content, as determined by insulin disulphide reduction assay.

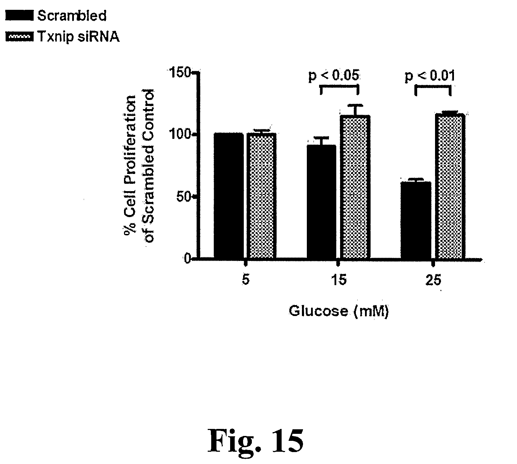

**Figure 15** provides a graphical representation showing alleviation of impaired proliferation of hyperglycemic HUVEC cells following siRNA-mediated silencing of TXNIP expression. HUVECs were cultured in 5mM, 15mM or 25 mM glucose for a 24 h period then transfected with double-stranded siRNA comprising SEQ ID NO: 5 for specific silencing of human *TRX1* mRNA e.g., SEQ ID NO: 1, or double-stranded siRNA comprising SEQ ID NO: 6 for specific silencing of human *TXNIP* mRNA e.g., SEQ ID NO: 3, using Lipofectamine 2000^{®} (Invitrogen) according to the manufacturer's protocol. A non-specific 'scrambled' ds-siRNA (SEQ ID NO: 7) was used as a negative control. At 18h post-transfection, EdU was added to the media at a final concentration of 10 µM and the cells cultured for a further 24 h. After this time period, cells were harvested, fixed and prepared for FACS analysis using the Click-iT™ EdU Flow Cytometry Assay Kit according to the manufacturer's protocol (Invitrogen). Data show average percentage cell proliferation (± SEM) relative to that observed for the cells exposed to the negative control "scrambled" siRNA, for at least three individual experiments performed on separate occasions.

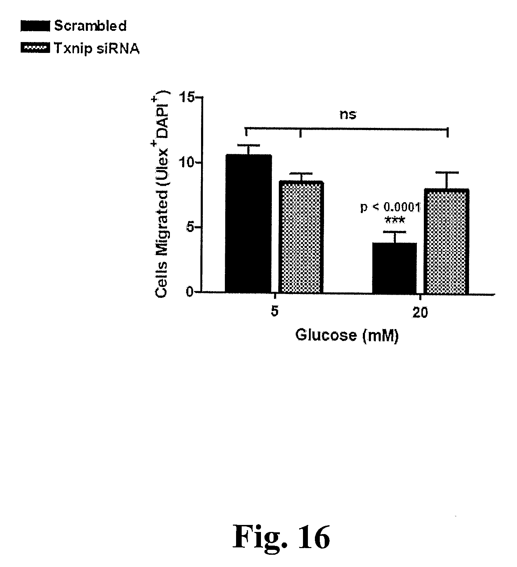

**Figure 16** provides a graphical representation showing alleviation of impaired migration of hyperglycemic HUVECs following siRNA-mediated gene silencing of TXNIP expression. HUVECs were cultured in 5mM or 20 mM glucose for a 24 h period then transfected with double-stranded siRNA comprising SEQ ID NO: 5 for specific silencing of human *TRX1* mRNA e.g., SEQ ID NO: 1, or double-stranded siRNA comprising SEQ ID NO: 6 for specific silencing of human *TXNIP* mRNA e.g., SEQ ID NO: 3, using Lipofectamine 2000^{®} (Invitrogen) according to the manufacturer's protocol. A non-specific 'scrambled' ds-siRNA (SEQ ID NO: 7) was used as a negative control. At 18h post-transfection, cells were harvested and placed in 8 µm transwells at a density of 1 x 10⁴ cells/well and incubated a further 18 h. After this time period, the transwells were removed and cells in the upper chamber discarded. Transwell membranes were stained with *Ulex*-Lectin-FITC and DAPI counter-stain. At least 15 fluorescent photomicrographs were taken per transwell and average cells migrated enumerated. Data are expressed as mean ± SEM and are representative of at least three separate experiments performed on individual occasions.

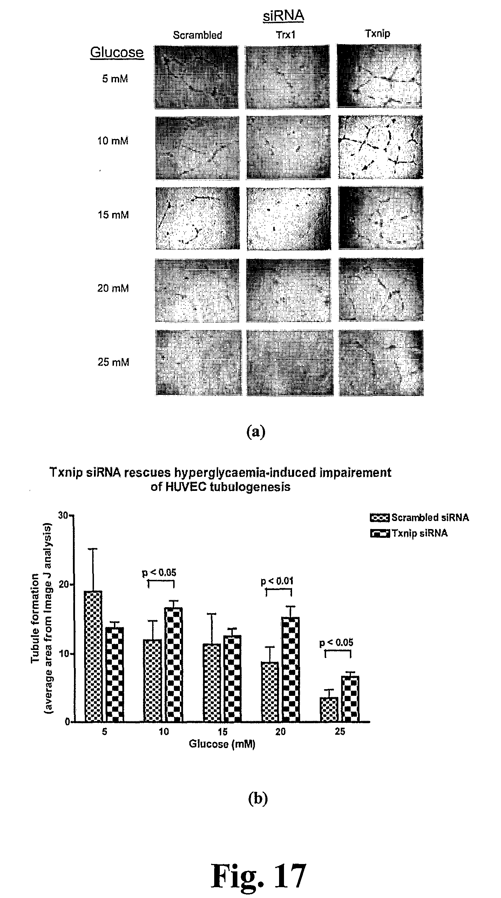

**Figure 17a** is a photographic representation showing alleviation of impaired tubulogenesis of hyperglycemic HUVEC tubulogenesis by siRNA-mediated gene silencing of TXNIP expression in a Matrigel assay. HUVECs were cultured in 5mM, 10mM, 15mM, 20mM or 25 mM glucose for a 24 h period then transfected with double-stranded siRNA comprising SEQ ID NO: 5 for specific silencing of human *TRX1* mRNA e.g., SEQ ID NO: 1, or double-stranded siRNA comprising SEQ ID NO: 6 for specific silencing of human *TXNIP* mRNA e.g., SEQ ID NO: 3, using Lipofectamine 2000^{®} (Invitrogen) according to the manufacturer's protocol. A non-specific 'scrambled' ds-siRNA comprising SEQ ID NO: 7 was used as a negative control. At 18h post-transfection, cells were harvested and cultured in wells coated with growth factor-reduced Matrigel for a further 18 h. After this time period, at least 15 photomicrographs were obtained. Data indicate that, at all concentrations of glucose tested, significantly higher tubulogenesis was observed for cells treated with double-stranded siRNA comprising SEQ ID NO: 6 and targeting TXNIP expression than for cells treated with either a control "scrambled siRNA or double stranded siRNA targeting TRX1 expression.
**Figure 17b** provides a graphical representation showing alleviation of impaired tubulogenesis of hyperglycemic HUVECs by siRNA-mediated gene silencing of TXNIP expression, wherein the siRNA comprised SEQ ID NO: 6. HUVECs were cultured in 5mM, 10mM, 15mM, 20mM or 25 mM glucose for a 24 h period then transfected with double-stranded siRNA comprising SEQ ID NO: 5 for specific silencing of human *TRX1* mRNA e.g., SEQ ID NO: 1, or double-stranded siRNA comprising SEQ ID NO: 6 for specific silencing of human *TXNIP* mRNA e.g., SEQ ID NO: 3, using Lipofectamine 2000^{®} (Invitrogen) according to the manufacturer's protocol. A non-specific 'scrambled' ds-siRNA comprising SEQ ID NO: 7 was used as a negative control. At 18h post-transfection, cells were harvested and cultured in wells coated with growth factor-reduced Matrigel for a further 18 h. After this time period, at least 15 photomicrographs were taken per well and average tubule area determined using Image J software. Data are expressed as mean tubule area ± SEM for at least three independent experiments performed on separate occasions.

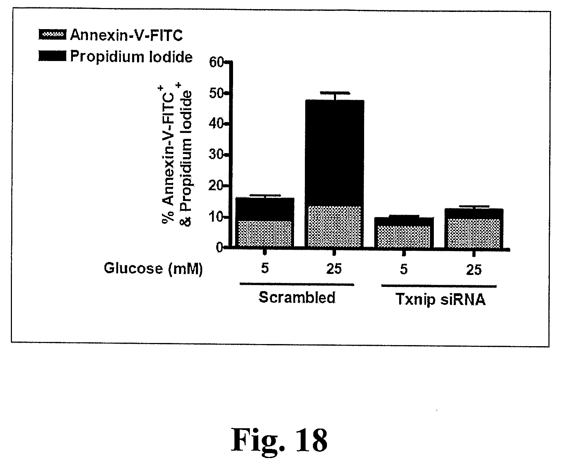

**Figure 18** is a graphical representation showing alleviation of the deleterious effect of hyperglycemia on viability of human umbilical vein endothelial cell (HUVEC) cells as demonstrated by annexin-V/propodium iodide assays, using double-stranded siRNA targeting expression of TXNIP and comprising the sequence set forth in SEQ ID NO: 6. HUVECs were cultured in 5mM or 25 mM glucose for a 24 h period then transfected with double-stranded siRNA comprising SEQ ID NO: 5 for specific silencing of human *TRX1* mRNA e.g., SEQ ID NO: 1, or double-stranded siRNA comprising SEQ ID NO: 6 for specific silencing of human *TXNIP* mRNA e.g., SEQ ID NO: 3, using Lipofectamine 2000^{®} (Invitrogen) according to the manufacturer's protocol. A non-specific 'scrambled' ds-siRNA comprising SEQ ID NO: 7 was used as a negative control. At 24h post-transfection, HUVECs were harvested, prepared and stained with Annexin-V-FITC/propidium iodide (Sigma-Aldrich) and analyzed by FACs according to the manufacturer's protocol. Data show mean staining ± SEM for a representative experiment, and indicate enhanced cell viability for cells treated with siRNA targeting TXNIP expression compared to cells treated with a negative control "scrambled" siRNA at both concentrations of glucose tested, however the effect is more dramatic for hyperglycemic cells exposed to the higher glucose concentration.

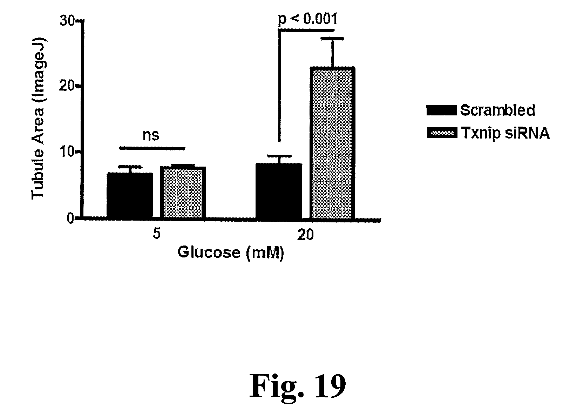

**Figure 19** is a graphical representation showing alleviation of hypoxia and/or hyperglycemia-induced suppression of HUVEC tubulogenesis as determined by tubule area, using siRNAs targeting TXNIP expression. HUVECs were cultured in 5mM or 20 mM glucose for a 24 h period then transfected with double-stranded siRNA comprising SEQ ID NO: 5 for specific silencing of human *TRX1* mRNA e.g., SEQ ID NO: 1, or double-stranded siRNA comprising SEQ ID NO: 6 for specific silencing of human *TXNIP* mRNA e.g., SEQ ID NO: 3, using Lipofectamine 2000^{®} (Invitrogen) according to the manufacturer's protocol. A non-specific 'scrambled' ds-siRNA comprising SEQ ID NO: 7 was used as a negative control. At 18h post-transfection, HUVECs were transferred to a hypoxic incubator (1% O₂/5% CO₂/N₂ balance; 37 °C) for a further 12 h. After inducing hypoxia, at least 15 photomicrographs were taken per well and tubule formation enumerated using Image J software. Data show that siRNAs targeting TXNIP expression are able to alleviate the suppression of tubulogenesis of hyperglycemic cells under hypoxic conditions. Data are shown as mean ± SEM.

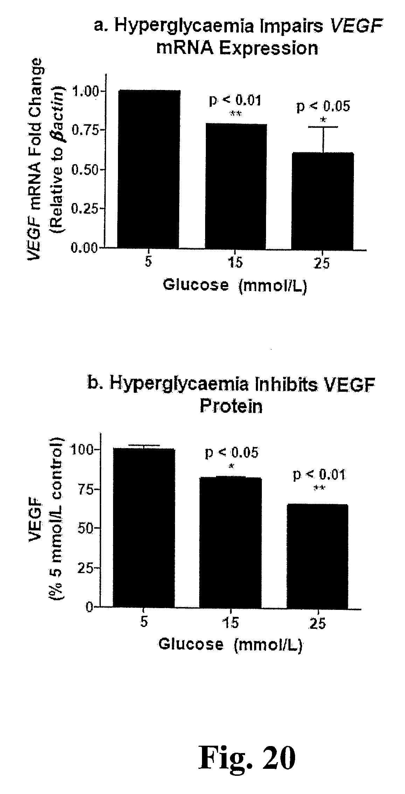

**Figure 20a** provides a graphical representation showing that hyperglycemia inhibits or reduces VEGF mRNA expression in HUVECs. HUVECs were grown in 5mM, 15mM or 25 mM D-glucose for 24 h. After 24 h, was RNA isolated and quantitative PCR performed using gene-specific oligonucleotide primers to amplify human *VEGFA165* mRNA and β-actin mRNA. Data show VEGF mRNA expression levels at each glucose concentration relative to expression at 5mM glucose, and normalized for β-actin mRNA expression (n=2 for each of three independent experiments).
**Figure 20b** provides a graphical representation showing that hyperglycemia reduces VEGF protein levels in HUVECs. HUVECs were grown in 5mM, 15mM or 25 mM D-glucose for 24 h. After 24 h, protein lysates were prepared and assayed by ELISA using antibodies specific for VEGF protein. Data show VEGF protein levels at each glucose concentration relative to expression at 5mM glucose, and normalized for total protein content of lysates (n=2 for each of three independent experiments).

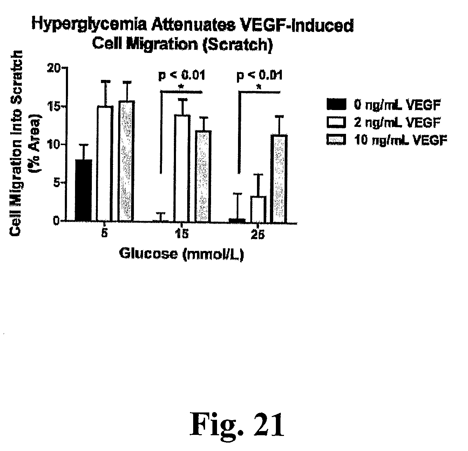

**Figure 21** provides a graphical representation showing that hyperglycemia attenuates VEGF-induced HUVEC migration. HUVECs were grown in media lacking VEGF or comprising 2ng/ml VEGF or 10ng/ml VEGF and 5mM glucose or 15mM glucose or 25 mM glucose for 24 h, and confluent HUVEC monolayers were scratched and the areas of the scratches quantified using Image J software. Cells were grown for a further 24 h in media comprising the same VEGF and glucose concentrations as before, further photomicrographs were obtained, and the migration of cells into the scratched areas quantitated using Image J software to analyze photomicrographs. Data indicate percentage of scratch areas occupied by cells following the second 24 hour incubation (n=3 in each of three independent experiments). The data confirm that VEGF induces HUVEC migration at all glucose concentrations tested and that hyperglycemia impairs migration of HUVECs, and demonstrate further that hyperglycemia attenuates the ability of VEGF to induce HUVEC migration. Higher VEGF concentrations are required to induce migration of hyperglycemic HUVECs than cells grown at low concentrations of glucose, demonstrating that VEGF may partially overcome impaired cell migration as a consequence of hyperglycemia.

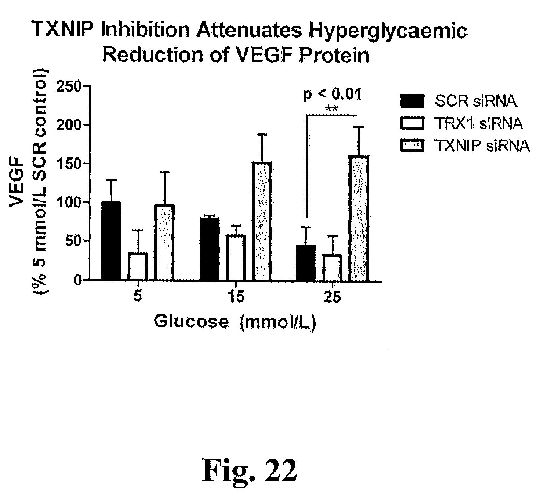

**Figure 22** is a graphical representation showing alleviation of hyperglycemia-induced reduction in VEGFA165 protein levels in HUVECs, using double-stranded siRNA to reduce TXNIP expression. HUVECs were cultured in 5mM, 15mM or 25 mM glucose for 24 h, then transfected with double-stranded siRNA comprising SEQ ID NO: 5 for specific silencing of human *TRX1* mRNA e.g., SEQ ID NO: 1, or double-stranded siRNA comprising SEQ ID NO: 6 for specific silencing of human *TXNIP* mRNA e.g., SEQ ID NO: 3, using Lipofectamine 2000^{®} (Invitrogen) according to the manufacturer's protocol. A non-specific 'scrambled' ds-siRNA (SCR) comprising SEQ ID NO: 7 was used as a negative control. At 24h post-transfection, cells were lysed and the lysates were subjected to an ELISA for detecting human VEGFA165 protein. Data indicate VEGF protein levels relative to protein level for cells grown in 5mM glucose and normalized for total protein contents of lysates (N=2 for each of three independent experiments). The data confirm that hyperglycemia reduces VEGF protein in the presence of the negative control SCR siRNA, and demonstrate that siRNA targeting expression of TXNIP significantly alleviates the adverse effect of hyperglycemia on VEGF protein expression (p<0.01).

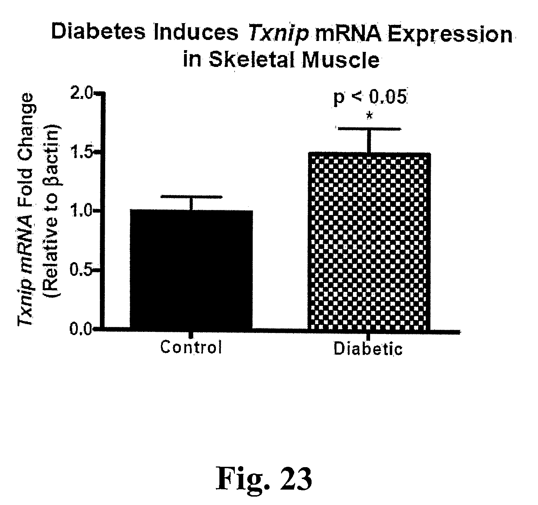

**Figure 23** provides a graphical representation showing that streptozotocin-induced diabetes mellitus enhances *TXNIP* mRNA expression in skeletal muscle *in vivo.* Diabetes mellitus was induced by administration of streptozotocin to male C57BL/6J mice and, two weeks following treatment, animals were sacrificed, their adductor muscles were removed, RNA was isolated, and real-time quantitative PCR was performed using oligonucleotide primers for amplifying both murine TXNIP-encoding mRNA transcripts (SEQ ID NOs: 415 and 416). Data show a significant increase (p<0.05) in TXNIP transcript levels relative to beta-actin transcript levels following induction of diabetes.

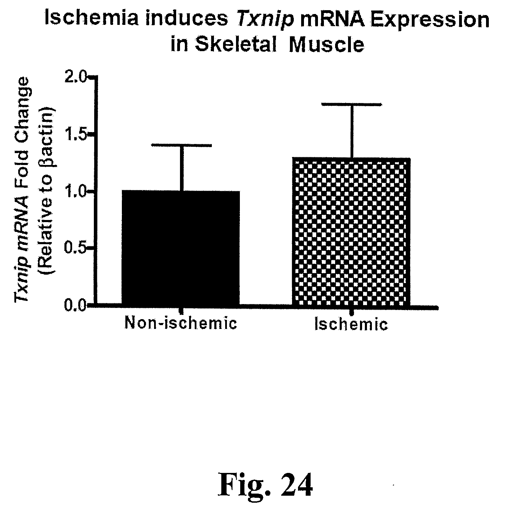

**Figure 24** is a graphical representation showing that ischemia enhances *TXNIP* mRNA expression in skeletal muscle *in vivo.* Ischemia was induced in a standard model of hindlimb ischemia, by performing unilateral femoral artery ligation in 7-week old male C57BL/6J mice and, two weeks following treatment, animals were sacrificed, their adductor muscles were removed, RNA was isolated, and real-time quantitative PCR was performed using oligonucleotide primers for amplifying both murine TXNIP-encoding mRNA transcripts (SEQ ID NOs: 415 and 416). Data show a significant increase (p<0.05) in TXNIP transcript levels due to ischemia, relative to beta-actin transcript levels, suggesting a biological role for TXNIP in vascular complications and/or vascular disease associated with ischemia.

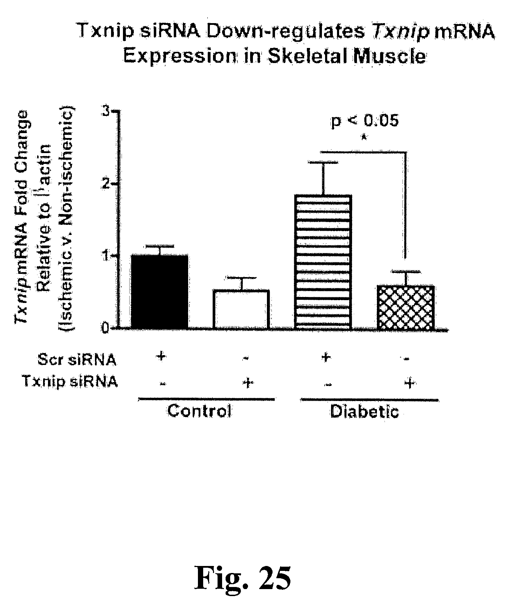

**Figure 25** is a graphical representation showing that siRNA-mediated gene silencing of TXNIP expression alleviates the enhancement of TXNIP expression by diabetes and ischemia *in vivo.* Diabetes mellitus was induced by administration of streptozotocin to male C57BL/6J mice and, two weeks following treatment, ischemia was induced by performing unilateral femoral artery ligation, plasmid encoding siRNA targeting murine TXNIP expression (SEQ ID NO: 417) or encoding a negative scrambled control siRNA (Scr) was administered to adductor muscle by intramuscular injection either on the same day (day 0) or 3 days or 7 days post-surgery, and animals were sacrificed at day 10 post-surgery, their adductor muscles were removed, RNA was isolated, and real-time quantitative PCR was performed using oligonucleotide primers for amplifying both murine TXNIP-encoding mRNA transcripts (SEQ ID NOs: 415 and 416). Data show a significant increase (p<0.05) in TXNIP transcript levels relative to β-actin transcript levels following induction of diabetes and ischemia, and that siRNA targeting is effective at reducing TXNIP expression *in vivo.* Control non-diabetic animals had received empty liposomes i.e., without siRNA.

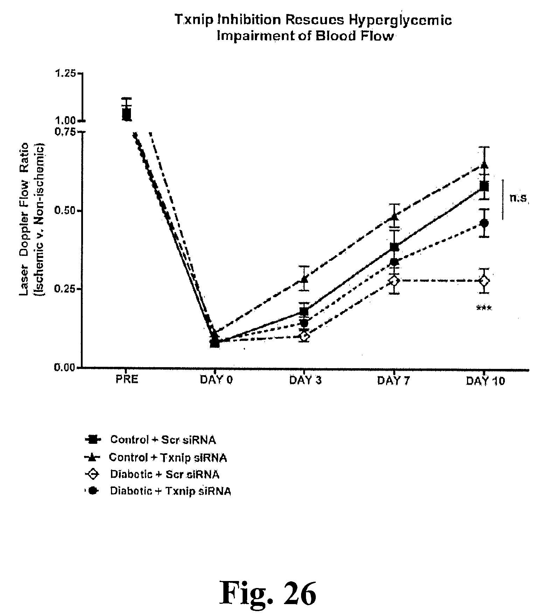

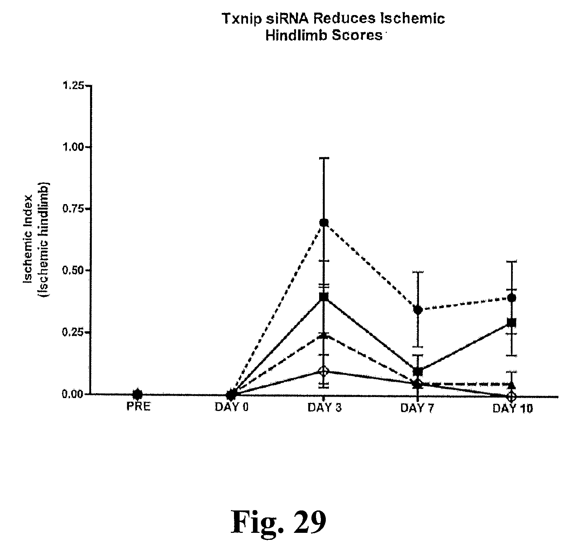

**Figure 26** provides a graphical representation showing that siRNA-mediated gene silencing of TXNIP expression alleviates a reduction in blood flow induced by ischemia in both diabetic and non-diabetic animals *in vivo.* Animals were rendered diabetic and ischemia induced in hindlimbs and siRNAs were administered as described in the legend to Figure 29, and Laser Doppler images of blood flow were recorded on anesthetized animals at 37°C. Data indicate ratios of blood flow for ischemic-to-non-ischemic hindlimbs of each animal. Control non-diabetic animals had received empty liposomes i.e., without siRNA. Data show that diabetes impairs blood flow and that ischemia impairs recovery in this animal model, however the impaired blood flow following an ischemic event in diabetic animals is alleviated by siRNA-mediated gene silencing of TXNIP expression. These data demonstrate a biological role for TXNIP in vascular complications and/or vascular disease associated with diabetes and ischemia, and indicate the beneficial effects of silencing or reducing TXNIP expression.

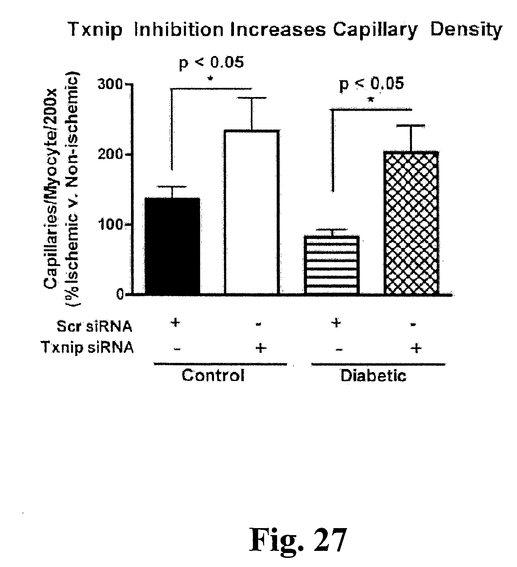

**Figure 27** provides a graphical representation showing that siRNA-mediated gene silencing of TXNIP expression alleviates a reduction in capillary density induced by ischemia in both diabetic and non-diabetic animals *in vivo.* Animals were rendered diabetic and ischemia induced in hindlimbs and siRNAs were administered as described in the legend to Figure 29, and animals were sacrificed at day 10 post-surgery and the distal portions of adductor muscles were removed and capillary densities determined relative to myocyte content by staining for von Willebrand factor (determinative of capillaries) and hematoxylin (determinative of myocytes). Data indicate ratios of capillary number to myocyte number for ischemic hindlimbs normalized relative to data for opposing non-ischemic hindlimbs of the same animals. Control non-diabetic animals had received empty liposomes i.e., without siRNA. Data show that diabetes and ischemia independently reduce capillary density in this animal model, however the reduced capillary density in diabetic animals and/or following an ischemic event is alleviated by siRNA-mediated gene silencing of TXNIP expression. These data also demonstrate a biological role for TXNIP in vascular complications and/or vascular disease associated with diabetes and ischemia, and indicate the beneficial effects of silencing or reducing TXNIP expression.

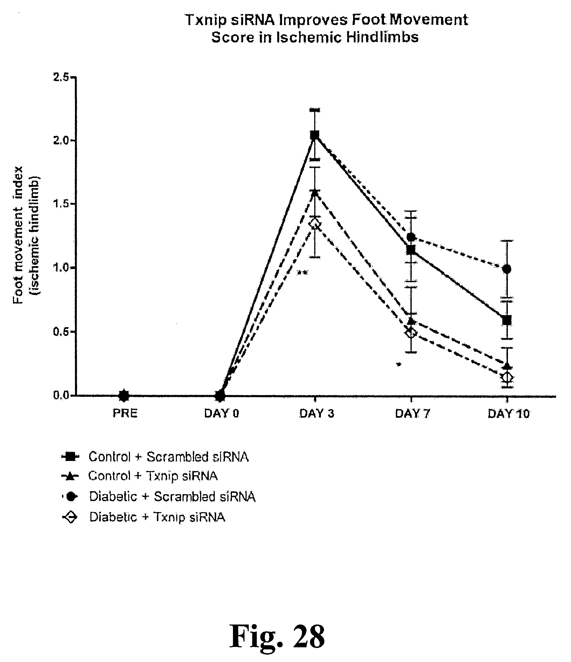

**Figure 28** provides a graphical representation showing that siRNA-mediated gene silencing of TXNIP expression alleviates a reduction in physical consequences of impaired blood flow induced by ischemia in both diabetic and non-diabetic animals in vivo. Animals were rendered diabetic and ischemia induced in hindlimbs and siRNAs were administered as described in the legend to Figure 29, and foot movements were determined for animals before surgery, on the day of surgery (day 0), and at day 3, day 7 and day 10 post-surgery. Data indicate foot movement indices for animals over time, wherein 0 = normal response, 1 = plantar but no toe flexion, 2 = no plantar or toe flexion, and score 3 = dragging of the limb. Control non-diabetic animals had received empty liposomes i.e., without siRNA. Data show that ischemia impairs foot movement in both diabetic and non-diabetic animals, and that recovery from ischemia as determined by improved foot movement in both diabetic and non-diabetic animals is enhanced by siRNA-mediated gene silencing of TXNIP expression. These data further demonstrate a biological role for TXNIP in vascular complications and/or vascular disease associated with diabetes and ischemia, and indicate the beneficial effects of silencing or reducing TXNIP expression.
**Figure 29** provides a graphical representation showing that siRNA-mediated gene silencing of TXNIP expression prevents or otherwise alleviates ischemic tissue damage in both diabetic and non-diabetic animals *in vivo.* Animals were rendered diabetic and ischemia induced in hindlimbs and siRNAs were administered as described in the legend to Figure 29, and ischemic tissue damage was determined for animals before surgery, on the day of surgery (day 0), and at day 3, day 7 and day 10 post-surgery. Data indicate tissue damage indices for animals over time, wherein 0 = no tissue damage, 1= skin discolouration, 2 = loss of 1 to 2 toes, 3 = loss of foot or more. Control non-diabetic animals had received empty liposomes i.e., without siRNA. Data show that ischemia causes mild tissue damage in both diabetic and non-diabetic animals, and that recovery from ischemia as determined by improved tissue coloration in both diabetic and non-diabetic animals is promoted by siRNA-mediated gene silencing of TXNIP expression. These data further demonstrate a biological role for TXNIP in vascular complications and/or vascular disease associated with diabetes and ischemia, and indicate the beneficial effects of silencing or reducing TXNIP expression.

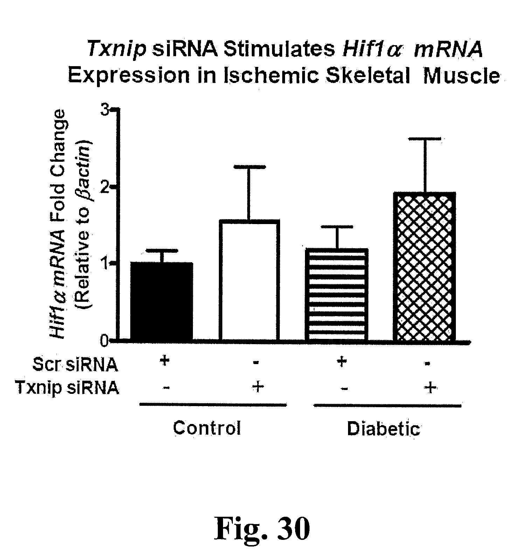

**Figure 30** provides a graphical representation showing that siRNA-mediated gene silencing of TXNIP expression enhances *Hif1-α* mRNA expression following an ischemic event in non-diabetic and diabetic animals. Diabetes mellitus was induced by administration of streptozotocin to male C57BL/6J mice and, two weeks following treatment, ischemia was induced by performing unilateral femoral artery ligation, plasmid encoding siRNA targeting murine TXNIP expression (SEQ ID NO: 417) or encoding a negative scrambled control siRNA (Scr) was administered to adductor muscle by intramuscular injection either on the same day (day 0) or 3 days or 7 days post-surgery, and animals were sacrificed at day 10 post-surgery, their adductor muscles were removed, RNA was isolated, and real-time quantitative PCR was performed using oligonucleotide primers for amplifying *Hif1-α* mRNA. Data indicate changes in *Hif1-α* mRNA relative to *β-actin* mRNA level. Data show that *Hif1-α* mRNA increases in both diabetic and non-diabetic animals exposed to an ischemic event and treated with siRNA targeting TXNIP expression, indicating enhanced angiogenesis following treatment. These data further indicate the beneficial effects of silencing or reducing TXNIP expression.

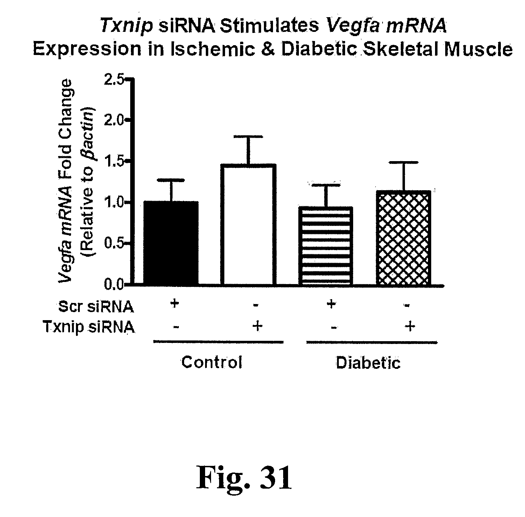

**Figure 31** provides a graphical representation showing that siRNA-mediated gene silencing of TXNIP expression enhances *VEGFa164* mRNA expression following an ischemic event in non-diabetic and diabetic animals. Diabetes mellitus was induced by administration of streptozotocin to male C57BL/6J mice and, two weeks following treatment, ischemia was induced by performing unilateral femoral artery ligation, plasmid encoding siRNA targeting murine TXNIP expression (SEQ ID NO: 417) or encoding a negative scrambled control siRNA (Scr) was administered to adductor muscle by intramuscular injection either on the same day (day 0) or 3 days or 7 days post-surgery, and animals were sacrificed at day 10 post-surgery, their adductor muscles were removed, RNA was isolated, and real-time quantitative PCR was performed using oligonucleotide primers for amplifying *VEGFa164* mRNA. Data indicate changes in *VEGFa164* mRNA relative to *β-actin* mRNA level. Data show that *VEGFa164* mRNA increases in both diabetic and non-diabetic animals exposed to an ischemic event and treated with siRNA targeting TXNIP expression, indicating enhanced angiogenesis following treatment. These data further indicate the beneficial effects of silencing or reducing TXNIP expression.

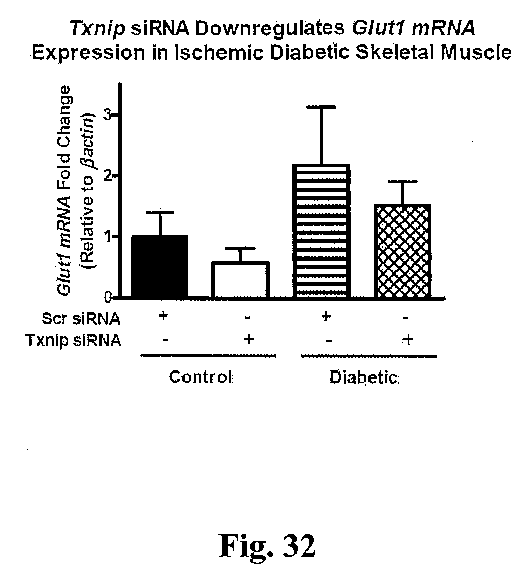

**Figure 32** provides a graphical representation showing that siRNA-mediated gene silencing of TXNIP expression leads to reduced *GLUT1* mRNA expression following an ischemic event in non-diabetic and diabetic animals. Diabetes mellitus was induced by administration of streptozotocin to male C57BL/6J mice and, two weeks following treatment, ischemia was induced by performing unilateral femoral artery ligation, plasmid encoding siRNA targeting murine TXNIP expression (SEQ ID NO: 417) or encoding a negative scrambled control siRNA (Scr) was administered to adductor muscle by intramuscular injection either on the same day (day 0) or 3 days or 7 days post-surgery, and animals were sacrificed at day 10 post-surgery, their adductor muscles were removed, RNA was isolated, and real-time quantitative PCR was performed using oligonucleotide primers for amplifying *GLUT1* mRNA. Data indicate changes in *GLUT1* mRNA relative to *β-actin* mRNA level. Data show that *GLUT1* mRNA increases in diabetic animals compared to non-diabetic animals, and that this enhanced expression is alleviated following treatment with siRNA targeting TXNIP expression. These data further indicate the beneficial effects of silencing or reducing TXNIP expression.

### Detailed description

### Thioredoxin (Trx) and thioredoxin interacting protein (TXNIP)

Exemplary TRX and TXNIP polypeptides comprise an amino acid sequence having at least about 80% amino acid sequence identity to an amino acid sequence set forth in SEQ ID NO: 2 or 4. Preferably, the degree of sequence identity is at least about 85%, more preferably, at least about 90% identical or 95% identical or 98% identical.

In another example, a TRX or TXNIP polypeptide is encoded by a nucleic acid comprising a nucleotide sequence that is at least about 80% identical to a nucleotide sequence set forth SEQ ID NO: 1 or 3. Preferably, the degree of sequence identity is at least about 85%, more preferably, at least about 90% identical or 95% identical or 98% identical.

In determining whether or not two amino acid sequences fall within the defined percentage identity limits supra, those skilled in the art will be aware that it is possible to conduct a side-by-side comparison of the amino acid sequences. In such comparisons or alignments, differences will arise in the positioning of non-identical residues depending upon the algorithm used to perform the alignment. In the present context, references to percentage identities and similarities between two or more amino acid sequences shall be taken to refer to the number of identical and similar residues respectively, between said sequences as determined using any standard algorithm known to those skilled in the art. In particular, amino acid identities and similarities are calculated using software of the Computer Genetics Group, Inc., University Research Park, Maddison, Wisconsin, United States of America, e.g., using the GAP program of Devereaux et al., Nucl. Acids Res. 12, 387-395, 1984, which utilizes the algorithm of Needleman and Wunsch, J. Mol. Biol. 48, 443-453, 1970. Alternatively, the CLUSTAL W algorithm of Thompson et al., Nucl. Acids Res. 22, 4673-4680, 1994, is used to obtain an alignment of multiple sequences, wherein it is necessary or desirable to maximize the number of identical/similar residues and to minimize the number and/or length of sequence gaps in the alignment.

Alternatively, a suite of commonly used and freely available sequence comparison algorithms is provided by the National Center for Biotechnology Information (NCBI) Basic Local Alignment Search Tool (BLAST) (Altschul et al. J. Mol. Biol. 215: 403-410, 1990), which is available from several sources, including the NCBI, Bethesda, Md. The BLAST software suite includes various sequence analysis programs including "blastn", that is used to align a known nucleotide sequence with other polynucleotide sequences from a variety of databases and "blastp" used to align a known amino acid sequence with one or more sequences from one or more databases. Also available is a tool called "BLAST 2 Sequences" that is used for direct pairwise comparison of two nucleotide sequences.

As used herein the term "NCBI" shall be taken to mean the database of the National Center for Biotechnology Information at the National Library of Medicine at the National Institutes of Health of the Government of the United States of America, Bethesda, MD, 20894.

In determining whether or not two nucleotide sequences fall within a particular percentage identity limitation recited herein, those skilled in the art will be aware that it is necessary to conduct a side-by-side comparison or multiple alignment of sequences. In such comparisons or alignments, differences may arise in the positioning of non-identical residues, depending upon the algorithm used to perform the alignment. In the present context, reference to a percentage identity between two or more nucleotide sequences shall be taken to refer to the number of identical residues between said sequences as determined using any standard algorithm known to those skilled in the art. For example, nucleotide sequences may be aligned and their identity calculated using the BESTFIT program or other appropriate program of the Computer Genetics Group, Inc., University Research Park, Madison, Wisconsin, United States of America (Devereaux et al, Nucl. Acids Res. 12, 387-395, 1984). As discussed supra, BLAST is also useful for aligning nucleotide sequences and determining percentage identity.

### Candidate compounds for screening and therapy

### Peptide compounds

An exemplary compound screened or assayed as described herein, or employed in therapy or prophylaxis, may be a peptidyl compound e.g., VEGF.

Such a peptidyl compound may be produced using any means known in the art. For example, a peptidyl compound is produced synthetically. Synthetic peptides are prepared using known techniques of solid phase, liquid phase, or peptide condensation, or any combination thereof, and can include natural and/or unnatural amino acids. Amino acids used for peptide synthesis may be standard Boc amino acid resin with the de-protecting, neutralization, coupling and wash protocols of the original solid phase procedure of Merrifield, J. Am. Chem. Soc., 85:2149-2154, 1963, or the base-labile Fmoc amino acids described by Carpino and Han, J. Org. Chem., 37:3403-3409, 1972. Both Fmoc and Boc Nÿ-amino protected amino acids can be obtained from various commercial sources, such as, for example, Fluka, Bachem, Advanced Chemtech, Sigma, Cambridge Research Biochemical, Bachem, or Peninsula Labs.

Alternatively, a synthetic peptide is produced using a technique known in the art and described, for example, in Stewart and Young (In: Solid Phase Synthesis, Second Edition, Pierce Chemical Co., Rockford, Ill. (1984) and/or Fields and Noble (Int. J. Pept. Protein Res., 35:161-214, 1990), or using an automated synthesizer. Accordingly, peptides may comprise D-amino acids, a combination of D- and L-amino acids, and various unnatural amino acids to convey special properties. Synthetic amino acids include ornithine for lysine, fluorophenylalanine for phenylalanine, and norleucine for leucine or isoleucine.

A peptidyl agent can also be produced using recombinant means. For example, an oligonucleotide or other nucleic acid is placed in operable connection with a promoter. Methods for producing such expression constructs, introducing an expression construct into a cell and expressing and/or purifying the expressed peptide, polypeptide or protein are known in the art and described, for example, in Ausubel et al (In: Current Protocols in Molecular Biology. Wiley Interscience, ISBN 047 150338, 1987) or Sambrook et al (In: Molecular Cloning: Molecular Cloning: A Laboratory Manual, Cold Spring Harbor Laboratories, New York, Third Edition 2001).

The term "promoter" is to be taken in its broadest context and includes the transcriptional regulatory sequences of a genomic gene, including the TATA box or initiator element, which is required for accurate transcription initiation, with or without additional regulatory elements (i.e., upstream activating sequences, transcription factor binding sites, enhancers and silencers) which alter gene expression in response to developmental and/or external stimuli, or in a tissue specific manner. In the present context, the term "promoter" is also used to describe a recombinant, synthetic or fusion molecule, or derivative which confers, activates or enhances the expression of a nucleic acid molecule to which it is operably linked, and which encodes the peptide or protein. Preferred promoters can contain additional copies of one or more specific regulatory elements to further enhance expression and/or alter the spatial expression and/or temporal expression of said nucleic acid molecule.

Placing a nucleic acid molecule under the regulatory control of, i.e., "in operable connection with", a promoter sequence means positioning said molecule such that expression is controlled by the promoter sequence, generally by positioning the promoter 5' (upstream) of the peptide-encoding sequence.

Suitable promoters and/or vectors for expressing a peptide will be apparent to the skilled artisan. For example, a typical promoters suitable for expression in a mammalian cell, mammalian tissue or intact mammal include, for example a promoter selected from the group consisting of, a retroviral LTR element, a SV40 early promoter, a SV40 late promoter, a cytomegalovirus (CMV) promoter, a CMV IE (cytomegalovirus immediate early) promoter, an EF1 promoter (from human elongation factor 1), an EM7 promoter or an UbC promoter (from human ubiquitin C).

A vector comprising such a suitable promoter is often used in an expression vector. A suitable expression vector for expression in a mammalian cell will be apparent to the skilled person and includes, for example, the pcDNA vector suite supplied by Invitrogen, the pCI vector suite (Promega), the pCMV vector suite (Clontech), the pM vector (Clontech), the pSI vector (Promega) or the VP16 vector (Clontech).

Typical promoters suitable for expression in viruses of bacterial cells and bacterial cells include, but are not limited to, the lacz promoter, the Ipp promoter, temperature-sensitive lambda-left and/or right promoters, T7 promoter, T3 promoter, SP6 promoter or semi-artificial promoters such as the IPTG-inducible tac promoter or lacUV5 promoter.

A number of other gene construct systems for expressing the nucleic acid fragment of the invention in bacterial cells are well-known in the art and are described for example, in Ausubel et al (In: Current Protocols in Molecular Biology. Wiley Interscience, ISBN 047 150338, 1987) and (Sambrook et al (In: Molecular Cloning: Molecular Cloning: A Laboratory Manual, Cold Spring Harbor Laboratories, New York, Third Edition 2001).

Numerous expression vectors for expression of recombinant polypeptides in bacterial cells and efficient ribosome binding sites have been described, such as for example, PKC30 (Shimatake and Rosenberg, Nature 292, 128, 1981); pKK173-3 (Amann and Brosius, Gene 40, 183, 1985), pET-3 (Studier and Moffat, J. Mol. Biol. 189, 113, 1986); the pCR vector suite (Invitrogen), pGEM-T Easy vectors (Promega), the pL expression vector suite (Invitrogen) the pBAD/TOPO (Invitrogen, Carlsbad, CA); the pFLEX series of expression vectors (Pfizer Inc., CT,USA); the pQE series of expression vectors (QIAGEN, CA, USA), or the pL series of expression vectors (Invitrogen), amongst others.

Methods for producing an expression construct will be apparent to the skilled artisan and/or described, for example, in Sambrook et al (In: Molecular cloning, A laboratory manual, second edition, Cold Spring Harbor Laboratory, Cold Spring Harbor, N.Y., 1989).

Following construction of a suitable expression construct, said construct is introduced into a cell using a method described herein, and the cell maintained for a time and under conditions sufficient for the expression of a peptide encoded by the nucleic acid. Optionally, the peptide is expressed as a fusion protein with a tag, e.g., a hexa-his tag or a myc tag to facilitate purification of the peptide, e.g., by affinity purification.

Alternatively, the peptide, polypeptide or protein is expressed using a cell free system, such as, for example, the TNT system available from Promega. Such an in vitro translation system is useful for screening a peptide library by, for example, ribosome display, covalent display or mRNA display.

A preferred source for a peptide useful for altering a phenotype of a genetically modified animal of the invention is, for example, a dominant negative mutant derived from the full-length sequence of a TXNIP polypeptide.

In another embodiment, a peptide library is screened to identify or isolate a compound that modulates angiogenic potential and/or activity and/or modulates neovascularization potential and/or activity in isolated cells, or alternatively or in addition, to identify or isolate a compound that enhances vascular network formation and/or development in an appropriate animal model. By "peptide library" is meant a plurality of peptides that may be related in sequence and/or structure or unrelated (e.g., random) in their structure and/or sequence. Suitable methods for production of such a library will be apparent to the skilled artisan and/or described herein.

For example, a random peptide library is produced by synthesizing random oligonucleotides of sufficient length to encode a peptide of desired length, e.g., 7 or 9 or 15 amino acids. Methods for the production of an oligonucleotide are known in the art. For example, an oligonucleotide is produced using standard solid-phase phosphoramidite chemistry. Essentially, this method uses protected nucleoside phosphoramidites to produce a short oligonucleotide (i.e., up to about 80 nucleotides). Typically, an initial 5'-protected nucleoside is attached to a polymer resin by its 3'-hydroxy group. The 5'hydroxyl group is then de-protected and the subsequent nucleoside-3'-phophoramidite in the sequence is then coupled to the de-protected group. The internucleotide bond is then formed by oxidising the linked nucleosides to form a phosphotriester. By repeating the steps of de-protection, coupling and oxidation an oligonucleotide of desired length and sequence is obtained. Suitable methods of oligonucleotide synthesis are described, for example, in Caruthers, M. H., et al., "Methods in Enzymology," Vol. 154, pp. 287-314 (1988).

Each of the oligonucleotides is then inserted into an expression construct (in operable connection with a promoter) and introduced into a cell or animal. Suitable methods for producing a random peptide library are described, for example, in Oldenburg et al., Proc. Natl. Acad. Sci. USA 89:5393-5397, 1992; Valadon et al., J. Mol. Biol., 261:11-22, 1996; Westerink Proc. Natl. Acad. Sci USA., 92:4021-4025, 1995; or Felici, J. Mol. Biol., 222:301-310, 1991.

Optionally, the nucleic acid is positioned so as to produce a fusion protein, wherein the random peptide is conformationally constrained within a scaffold structure, e.g., a thioredoxin (Trx) loop (Blum et al. Proc. Natl. Acad. Sci. USA, 97, 2241-2246, 2000) or a catalytically inactive staphylococcal nuclease (Norman et al, Science, 285, 591-595, 1999), to enhance their stability. Such conformational constraint within a structure has been shown, in some cases, to enhance the affinity of an interaction between a random peptides and its target.

To facilitate screening of a large number of peptides (i.e., to avoid producing a number of animals capable of expressing a peptide to be screened) a peptide may optionally be fused or conjugated to a protein transduction domain to facilitate its uptake into a cell of a transgenic mouse of the invention. A suitable protein transduction domain will be apparent to the skilled person and includes, for example, HIV-1 TAT or polyarginine. Protein transduction domains are reviewed, for example, in Deitz and Bahr, Mol. Cell Neurosci. Oct;27: 85-131, 2004.

In another example, described herein is the use of a mimetic of incretin e.g., exenatide or exendin-4, in the preparation of a medicament for the treatment of a vascular complication of diabetes mellitus or in the treatment of a vascular disease associated with diabetes mellitus or other condition associated with impaired glucose tolerance and/or hyperglycemia, or to other wise promote vascular repair in a subject. In a particular example, a mimetic of incretin e.g., exenatide or exendin-4 is employed to promote vascular repair in a subject, wherein the peptide is administered to a subject in need thereof for a time and under conditions sufficient to promote vascular repair e.g., by enhancing mobilization of cells that participate in vascular repair and/or enhancing angiogenic processes or neovascularisation processes. For example, exenatide is a 39 amino acid peptide agonist of the insulinotropic gut hormone glucagon-like peptide 1 (GLP-1). GLP-1 may also be employed. In one example, exenatide is formulated for administration once or twice daily by the intramuscular route. In another example, exenatide is formulated for administration once or twice daily by the subcutaneous route.

### Antibodies

The present application additionally describes screening and use of antibody-based compounds and fragments of antibodies capable of entering a cell. Preferred antibody-based compounds and fragments are capable of modulating endothelial cell function and/or angiogenic potential and/or activity and/or capable of modulating neovascularization potential and/or activity in isolated cells, or alternatively or in addition, capable of enhancing vascular network formation and/or development in an appropriate animal model.

By "antibody based compound" is meant an antibody or a fragment thereof, whether produce using standard or recombinant techniques. Accordingly, an antibody based compound includes, for example, an intact monoclonal or polyclonal antibody, an immunoglobulin (IgA, IgD, IgG, IgM, IgE) fraction, a humanized antibody, or a recombinant single chain antibody, as well as a fragment of an antibody, such as, for example Fab, F(ab)2, and Fv fragments.

A suitable antibody for altering a phenotype of the genetically modified animal of the invention is, for example, directed against TXNIP or a B-cell epitope thereof.

Such an antibody, preferably a monoclonal antibody, is produced by immunizing an animal (e.g., a mouse) with the relevant immunogen. Optionally, the immunogen is injected in the presence of an adjuvant, such as, for example Freund's complete or incomplete adjuvant, lysolecithin and/or dinitrophenol to enhance the immune response to the immunogen. The immunogen may also be linked to a carrier protein, such as, for example, BSA. Spleen cells are then obtained from the immunized animal. The spleen cells are then immortalized by, for example, fusion with a myeloma cell fusion partner, preferably one that is syngenic with the immunized animal. A variety of fusion techniques may be employed, for example, the spleen cells and myeloma cells may be combined with a nonionic detergent or electrofused and then grown in a selective medium that supports the growth of hybrid cells, but not myeloma cells. A preferred selection technique uses HAT (hypoxanthine, aminopterin, thymidine) selection. After a sufficient time, usually about 1 to 2 weeks, colonies of hybrids are observed. Single colonies are selected and growth media in which the cells have been grown is tested for the presence of binding activity against the polypeptide (immunogen). Hybridomas having high reactivity and specificity are preferred.

Monoclonal antibodies are isolated from the supernatants of growing hybridoma colonies using methods such as, for example, affinity purification using the immunogen used to immunize the animal to isolate an antibody capable of binding thereto. In addition, various techniques may be employed to enhance the yield, such as injection of the hybridoma cell line into the peritoneal cavity of a suitable vertebrate host, such as a mouse. Monoclonal antibodies are then harvested from the ascites fluid or the blood of such an animal subject. Contaminants are removed from the antibodies by conventional techniques, such as chromatography, gel filtration, precipitation, and/or extraction.

Alternatively, an antibody library and preferably a library of antibodies that are expressed within a cell is used for the screening method. Such an antibody, also known as an intrabody, is essentially as recombinant ScFv antibody fragment. Essentially, an ScFv antibody fragment is a recombinant single chain molecule containing the variable region of a light chain of an antibody and the variable region of a heavy chain of an antibody, linked by a suitable, flexible polypeptide linker.

A library of ScFv fragments is produced, for example, by amplifying a variable region of a large and/or small chain from nucleic acid amplified from spleen cells that are derived from a subject (e.g., a mouse) that optionally, has been previously immunized with an immunogen of interest. These regions are cloned into a vector encoding a suitable framework including a linker region to facilitate expression of an intrabody that is stable when expressed in a cell (for example, see Worn et al., J. Biol. Chem., 275: 2795-803, 2003). An intrabody may be directed to a particular cellular location or organelle, for example by constructing a vector that comprises a polynucleotide sequence encoding the variable regions of an intrabody that may be operatively fused to a polynucleotide sequence that encodes a particular target antigen within the cell (see, e.g., Graus-Porta et al., Mol. Cell Biol. 15:1182-91, 1995; Lener et al., Eur. J. Biochem. 267:1196-205 2000).

In another example, the present application describes the use of a therapeutic antibody e.g., a humanized recombinant antibody that binds to human TXNIP protein in the preparation of a medicament for the treatment of a vascular complication of diabetes mellitus or in the treatment of a vascular disease associated with diabetes mellitus or other condition associated with impaired glucose tolerance and/or hyperglycemia, or to other wise promote vascular repair in a subject. In a particular example, antibodies that bonds to human TXNIP protein are employed to promote vascular repair in a subject, wherein the antibodies are administered to a subject in need thereof for a time and under conditions sufficient to promote vascular repair e.g., by enhancing mobilization of cells that participate in vascular repair and/or enhancing angiogenic processes or neovascularisation processes. In one example, the antibody is formulated for administration by the intramuscular route e.g., once or twice daily. In another example, the antibody is formulated for administration by the subcutaneous route e.g., once or twice daily.

### Nucleic acids

The present application additionally contemplates screening and use of a nucleic acid compound e.g., RNA, DNA, etc. Preferred nucleic acids for therapy and/or prophylaxis are capable of being expressed in or entering a cell, to thereby modulate endothelial cell function or proliferative potential and/or angiogenic potential and/or activity and/or neovascularization potential and/or activity in isolated cells, or alternatively or in addition, capable of enhancing vascular network formation and/or development in an appropriate animal model.

Preferably, a library of nucleic acids that are capable of being expressed in or entering a cell are screened to identify or isolate a compound capable of modulating angiogenic potential and/or activity and/or capable of modulating neovascularization potential and/or activity in isolated cells, or alternatively or in addition, capable of enhancing vascular network formation and/or development in an appropriate animal model.

Preferred nucleic acid-based compounds inhibit TXNIP expression and/or activity and/or level e.g., by virtue of having or comprising a sequence complementary to or comprising a sequence encoding a TXNIP protein or fragment thereof. When introduced into a cell using suitable methods, such a nucleic acid inhibits the expression of the target gene encoded by the sense strand.

An anti-sense compound shall be taken to mean an oligonucleotide comprising DNA or RNA or a derivative thereof (e.g., PNA or LNA) that is complementary to at least a portion of a specific nucleic acid target e.g., TXNIP mRNA. Preferably, an antisense molecule comprises at least about 15 or 20 or 30 or 40 nucleotides complementary to the nucleotide sequence of a target nucleic acid. The use of antisense methods is known in the art (Marcus-Sakura, Anal. Biochem. 172: 289, 1988).

A ribozyme is an antisense nucleic acid molecule that is capable of specifically binding to and cleaving a target nucleic acid e.g., TXNIP mRNA. A ribozyme that binds to a target nucleic acid and cleaves this sequence reduces or inhibits the translation of said nucleic acid. Five different classes of ribozymes have been described based on their nucleotide sequence and/or three dimensional structure, namely, Tetrahymena group I intron, Rnase P, hammerhead ribozymes, hairpin ribozymes and hepatitis delta virus ribozymes. Generally, a ribozyme comprises a region of nucleotides (e.g., about 12 to 15 nucleotides) that are complementary to a target sequence.

An RNAi (or siRNA or small interfering RNA) is a double stranded RNA molecule that is identical to a specific gene product e.g., TXNIP mRNA. The dsRNA when expressed or introduced into a cell induces expression of a pathway that results in specific cleavage of a nucleic acid highly homologous to the dsRNA.

RNAi molecules are described, for example, by Fire et al., Nature 391: 806-811, 1998, and reviewed by Sharp, Genes and Development, 13: 139-141, 1999). As will be known to those skilled in the art, short hairpin RNA ("shRNA") is similar to siRNA. However, the shRNA molecule comprises a single strand of nucleic acid with two complementary regions (highly homologous to the sequence of a region of an IRES or the complement thereof) separated by an intervening hairpin loop such that, following introduction to a cell, it is processed by cleavage of the hairpin loop into siRNA.

The siRNA or shRNA molecule will typically comprise a nucleotide sequence that is identical to about 19-21 contiguous nucleotides of the target mRNA e.g., TXNIP mRNA. Preferably, the target sequence commences with the dinucleotide AA, comprises a GC-content of about 30-70% (preferably, 30-60%, more preferably 40-60% and more preferably about 45%-55%), and is specific to the nucleic acid of interest.

The siRNA or shRNA is preferably capable of down-regulating expression of human TXNIP in a cell. The cell-based and animal models exemplified herein provide support for reducing human and murine TXNIP expression.

For animal models wherein human TXNIP is expressed ectopically in murine animal hosts, it may be appropriate for the siRNA or shRNA molecules to reduce expression of both endogenous murine TXNIP, as well as ectopically expressed human TXNIP. Alternatively, animal models of human TXNIP knockdown may have murine genetic backgrounds that are TXNIP^{-/-}. In any event, confirmation of a specific activity of any antagonist against human TXNIP is determined by assessing the activity of an inhibitor in a cell derived from a TXNIP^{-/-} mouse that has been engineered to express human TXNIP.

Preferred siRNA or shRNA targeting TXNIP expression are presented in Table 1 hereof, and comprise nucleotide sequences set forth in SEQ ID No: 6 or any one of SEQ ID NOs: 8-403 or complementary sequences thereto. For the purposes of nomenclature, the sequences set forth in SEQ ID Nos: 6 and 8-403 comprise: (i) target sequences in the human TXNIP mRNA target sequence adjacent and downstream of a dinucleotide AA in said mRNA target; (ii) sequences of sense strand siRNAs comprising the mRNA target sequences at (i) each without the 5'-dinucleotide AA and each comprising a 3'-dinucleotide UU; and (iii) sequences of antisense strand siRNAs complementary to the human TXNIP mRNA target sequences. The sequence set forth in SEQ ID NO: 6 targeting residues 533-551 of TXNIP mRNA (SEQ ID NO: 3) is particularly preferred for construction of siRNA, and for the construction of shRNA targeting the same sequence, the following protocol is employed.

For producing any shRNA targeting any region of TXNIP mRNA such as a target sequence shown in Table 1, and/or for producing shRNA from the exemplified sense strand and antisense strand siRNAs set forth in SEQ ID NOs: 6 or Table 1, the sense and antisense strands for each target sequence are positioned such that they flank an intervening loop e.g., comprising a sequence selected from the group consisting of:
(i) CCC (SEQ ID NO: 404);
(ii) TTCG (SEQ ID NO: 405);
(iii) CCACC (SEQ ID NO: 406);
(iv) CTCGAG (SEQ ID NO: 407);
(v) AAGCTT (SEQ ID NO: 408);
(vi) CCACACC (SEQ ID NO: 409);
(vii) TTCAAGAGA (SEQ ID NO: 410);
(viii) AAGTTCTCT (SEQ ID NO: 411);
(ix) TTTGTGTAG (SEQ ID NO: 412);
(x) CTTCCTGTCA (SEQ ID NO: 413); and
(xi) CTTGATATCCGAG (SEQ ID NO: 414).

Of these loop sequences, the sequence set forth in SEQ ID NO: 414 is particularly preferred for modulating human TXNIP expression.

Preferred siRNA molecules that are selectively active against human TXNIP expression compared to murine TXNIP expression are derived from the sequence of the 5'-non-coding and/or 3'-non-coding region of the human TXNIP gene. Such specific siRNAs include, e.g., SEQ ID Nos: 57-59, 117-121, 175-177 and 235-239.

Antisense RNA, ribozyme, siRNA or shRNA can be introduced directly to a cell or cell-free extract capable of expressing TXNIP as naked DNA. Alternatively, DNA encoding a nucleic acid inhibitory molecule can be introduced into a cell in operable connection with a suitable promoter and transcription terminator sequence to facilitate expression of the inhibitory nucleic acid. Preferred promoters for expression in mammalian cells that express TXNIP include the CMV promoter, ubiquitin promoter, U6 small nuclear RNA promoter (Lee et al., Nature Biotech. 20, 500-505, 2002; Miyagishi et a;., Nature Biotech 20, 497-500, 2002; Paul et al., Nature Biotech. 20, 505-508, 2002; and Yu et al., Proc. Natl Acad. Sci USA 99, 6047-6052, 2002), H1-RNA promoter (Brummelkamp et al., Science 296, 550-553, 2002), or other RNA polymerase III promoter. The pol III terminator is also useful for such applications. Other promoters and terminators are not to be excluded.

In one example, the DNA encoding the inhibitory nucleic acid is operably connected to promoter and terminator regulatory sequences by cloning into a suitable vector that comprises the necessary promoter and transcriptional terminator sequences, and the recombinant vector is then introduced to the cell, tissue or organ by transient transfection of plasmid DNA, by establishing permanent cell lines or in infection with retroviral expression vectors (Barton et al., Proc. Natl Acad. Sci USA 99, 14943-14945, 2002; Devroe et al., BMC Biotech. 2, p15, 2002).

In high throughput primary assays e.g., to test inhibitors for their efficacy in reducing TXNIP expression, an *in vitro* cell-free system or cell-based system in which TXNIP activity or expression is monitored in the absence and presence of the inhibitory nucleic acid may be employed. Several vectors are known for this purpose, including, for example, the pSilencer series of vectors (pSilencer 2.0, pSilencer 2.1, pSilencer 3.0, pSilencer 3.1, pSilencer 1.0-U6) provided by Ambion.

Retroviral vectors are suitable for transiently transfecting nucleic acid inhibitors into isolated cells e.g., by calcium phosphate precipitation (Ketteler et al., Gene Ther. 9, 477-487, 2002) in high throughput screens for inhibitor activity, or for the production of transducing supernatants (Ketteler et al., Gene Ther. 9, 477-487, 2002) for lower-throughput screening or validation of primary screen results.

Exemplary retroviral vectors include pBABE (Morgenstern et al., Nuc. Acids Res. 18, 3587-3596, 1990) and JZenNeo. The pBabe retroviral vector constructs transmit inserted genes at high titres and express them from the Mo MuLV Long Terminal Repeat (LTR). The pBabe vectors comprise one of four different dominantly-acting selectable markers, allowing the growth of infected mammalian cells in the presence of G418, hygromycin B, bleomycin/phleomycin or puromycin. The high titre ecotropic helper free packaging cell line, omega E, reduces the risk of generation of wild type Mo MuLV via homologous recombination events. Together, the pBabe vectors and omega E cell line provide high frequency gene transfer, and/or concomitant expression of TXNIP with one or more other genes (e.g., APS and/or insulin receptor β-subunit) in a single cell, with minimal risk of helper virus contamination.

For lower throughput primary screening of inhibitory nucleic acids, or for validation of inhibitory activity, adenoviral vectors pAdTrack and pAdTrack-CMV (He et al., Proc. Natl Acad. Sci USA 95, 2509-2514, 1998; pAdTrack-HP (Zhao et al., Gene 316, 137-141, 2003), an Ad5CMV-based vector e.g., Ad5CMV-GFP (Suoka et al., Am. J. Respir. Cell Mol. Biol. 23, 297-303, 2000), and pSilencer adeno 1.0-CMV (Ambion) are particularly suited. These vector provide delivery and expression in specific organs or tissues, in particular muscle tissue of a mouse model. The pAdTrack and pAdTrack-CMV vectors are particularly preferred for applications which require standardization for transfection or transduction efficiency e.g., injection of adenovirus into hindlimb muscles of transgenic mouse models. The pAdTrack vector is used for production of GFP-trackable viruses containing transgenes under the control of a chosen promoter. It contains the gene encoding enhanced GFP, a polylinker for insertion of exogenous transgenes surrounded by adenoviral sequences ("arms") that allow homologous recombination with pAdEasy-1. The left arm contains Ad5 nucleotides 34,931-35,935, which mediate homologous recombination with pAdEasy vectors in *E. coli,* plus inverted terminal repeat (ITR) and packaging signal sequences (nucleotides 1-480 of Ad5) required for viral production in mammalian cells. The right arm contains Ad5 nucleotides 3,534-5,790, which mediate homologous recombination with pAdEasy vectors. Artificially created *Pad* sites surround both arms. The AdTrack plasmid also contains a kanamycin resistance gene from pZero 2.1 (Invitrogen) and the origin of replication from pBR322 (Life Technologies). The relatively low copy number of plasmids generated with this origin is essential for the stability of large constructs in E. *coli.* The pAdTrack-CMV vector is identical to pAdTrack except for the addition of a cytomegalovirus (CMV) promoter and polyadenylation site (both from pEGFP-C1, Clontech). A polylinker is present between the CMV promoter and polyadenylation site.

As will be known to the skilled artisan, such adenoviral vectors are also suitable for transfection of cell lines.

A nucleic acid aptamer (adaptable oligomer) is a nucleic acid molecule that is capable of forming a secondary and/or tertiary structure that provides the ability to bind to a molecular target e.g., TXNIP mRNA. Suitable methods for producing and/or screening an aptamer library is described, for example, in Ellington and Szostak, Nature 346:818-22,1990.

Generally, an aptamer is identified from a library of nucleic acids that comprise at least a random region are screened. For example, the library is screened to identify an aptamer that is capable of modulating angiogenic potential and/or activity and/or capable of modulating neovascularization potential and/or activity in isolated cells, or alternatively or in addition, capable of enhancing vascular network formation and/or development in an appropriate animal model.

An aptamer usually comprises at least one random region of nucleic acid, often flanked by a known sequence that may provide the annealing site for hybridization of a PCR primer for later amplification of the aptamer and/or may provide for conformational constraint, e.g., by forming a hairpin. The random sequence portion of the oligonucleotide can be of any length (however, is generally 30 to 50 nucleotides in length) and can comprise ribonucleotides and/or deoxyribonucleotides and can include modified or non-natural nucleotides or nucleotide analogs. Random oligonucleotides can be synthesized from phosphodiester-linked nucleotides using solid phase oligonucleotide synthesis techniques well known in the art and/or described herein. Typical syntheses carried out on automated DNA synthesis equipment yield 1015-1017 molecules.

**Table 1. Exemplary target sequences for reducing human TXNIP expression by siRNA and/or shRNA knock-down, and sequences for production of siRNA and/or shRNA therefor**

| **Sequence Description** | **SEQ ID NO:** |
|---|---|
| Target sequence 1: AAACUAACCCCUCUUUUUCUC | 8 |
| Sense strand siRNA: CUAACCCCUCUUUUUCUCUU | 9 |
| Antisense strand siRNA:GAGAAAAAGAGGGGUUAGUUU | 10 |
| | |
| Target sequence 2: AACCCCUCUUUUUCUCCAAAG | 11 |
| Sense strand siRNA: CCCCUCUUUUUCUCCAAAGUU | 12 |
| Antisense strand siRNA: CUUUGGAGAAAAAGAGGGGUU | 13 |
| | |
| Target sequence 3: AAAGGAGUGCUUGUGGAGAUC | 14 |
| Sense strand siRNA: AGGAGUGCUUGUGGAGAUCUU | 15 |
| Antisense strand siRNA: GAUCUCCACAAGCACUCCUUU | 16 |
| | |
| Target sequence 4: AAUUGGGGGAAAGAAGGCUUU | 17 |
| Sense strand siRNA: UUGGGGGAAAGAAGGCUUUUU | 18 |
| Antisense strand siRNA: AAAGCCUUCUUUCCCCCAAUU | 19 |
| | |
| Target sequence 5: AAAGAAGGCUUUUUCUCUGAA | 20 |
| Sense strand siRNA: AGAAGGCUUUUUCUCUGAAUU | 21 |
| Antisense strand siRNA: UUCAGAGAAAAAGCCUUCUUU | 22 |
| | |
| Target sequence 6: AAGGCUUUUUCUCUGAAUUCG | 23 |
| Sense strand siRNA: GGCUUUUUCUCUGAAUUCGUU | 24 |
| Antisense strand siRNA: CGAAUUCAGAGAAAAAGCCUU | 25 |
| | |
| Target sequence 7: AAUUCGCUUAGUGUAACCAGC | 26 |
| Sense strand siRNA: UUCGCUUAGUGUAACCAGCUU | 27 |
| Antisense strand siRNA: GCUGGUUACACUAAGCGAAUU | 28 |
| | |
| Target sequence 8: AACCAGCGGCGUAUAUUUUUU | 29 |
| Sense strand siRNA: CCAGCGGCGUAUAUUUUUUUU | 30 |
| Antisense strand siRNA: AAAAAAUAUACGCCGCUGGUU | 31 |
| | |
| Target sequence 9: AACCUAGUAGUUAAUAUUCAU | 32 |
| Sense strand siRNA: CCUAGUAGUUAAUAUUCAUUU | 33 |
| Antisense strand siRNA: AUGAAUAUUAACUACUAGGUU | 34 |
| | |
| Target sequence 10: AAUAUUCAUUUGUUUAAAUCU | 35 |
| Sense strand siRNA: UAUUCAUUUGUUUAAAUCUUU | 36 |
| Antisense strand siRNA: AGAUUUAAACAAAUGAAUAUU | 37 |
| | |
| Target sequence 11: AAAUCUUAUUUUAUUUUUAAG | 38 |
| Sense strand siRNA: AUCUUAUUUUAUUUUUAAGUU | 39 |
| Antisense strand siRNA: CUUAAAAAUAAAAUAAGAUUU | 40 |
| | |
| Target sequence 12: AAGCUCAAACUGCUUAAGAAU | 41 |
| Sense strand siRNA: GCUCAAACUGCUUAAGAAUUU | 42 |
| Antisense strand siRNA: AUUCUUAAGCAGUUUGAGCUU | 43 |
| | |
| Target sequence 13: AAACUGCUUAAGAAUACCUUA | 44 |
| Sense strand siRNA: ACUGCUUAAGAAUACCUUAUU | 45 |
| Antisense strand siRNA: UAAGGUAUUCUUAAGCAGUUU | 46 |
| | |
| Target sequence 14: AAGAAUACCUUAAUUCCUUAA | 47 |
| Sense strand siRNA: GAAUACCUUAAUUCCUUAAUU | 48 |
| Antisense strand siRNA: UUAAGGAAUUAAGGUAUUCUU | 49 |
| | |
| Target sequence 15: AAUACCUUAAUUCCUUAAAGU | 50 |
| Sense strand siRNA: UACCUUAAUUCCUUAAAGUUU | 51 |
| Antisense strand siRNA: ACUUUAAGGAAUUAAGGUAUU | 52 |
| | |
| Target sequence 16: AAUUCCUUAAAGUGAAAUAAU | 53 |
| Sense strand siRNA: UUCCUUAAAGUGAAAUAAUUU | 54 |
| Antisense strand siRNA: AUUAUUUCACUUUAAGGAAUU | 55 |
| | |
| Target sequence 17: AAAGUGAAAUAAUUUUUUGCA | 56 |
| Sense strand siRNA: AGUGAAAUAAUUUUUUGCAUU | 57 |
| Antisense strand siRNA: UGCAAAAAAUUAUUUCACUUU | 58 |
| | |
| Target sequence 18: AAAUAAUUUUUUGCAAAGGGG | 59 |
| Sense strand siRNA: AUAAUUUUUUGCAAAGGGGUU | 60 |
| Antisense strand siRNA: CCCCUUUGCAAAAAAUUAUUU | 61 |
| | |
| Target sequence 19: AAUUUUUUGCAAAGGGGUUUC | 62 |
| Sense strand siRNA: UUUUUUGCAAAGGGGUUUCUU | 63 |
| Antisense strand siRNA: GAAACCCCUUUGCAAAAAAUU | 64 |
| | |
| Target sequence 20: AAAGGGGUUUCCUCGAUUUGG | 65 |
| Sense strand siRNA: AGGGGUUUCCUCGAUUUGGUU | 66 |
| Antisense strand siRNA: CCAAAUCGAGGAAACCCCUUU | 67 |
| | |
| Target sequence 21: AACCAACUCAGUUCCAUCAUG | 68 |
| Sense strand siRNA: CCAACUCAGUUCCAUCAUGUU | 69 |
| Antisense strand siRNA: CAUGAUGGAACUGAGUUGGUU | 70 |
| | |
| Target sequence 22: AACUCAGUUCCAUCAUGGUGA | 71 |
| Sense strand siRNA: CUCAGUUCCAUCAUGGUGAUU | 72 |
| Antisense strand siRNA: UCACCAUGAUGGAACUGAGUU | 73 |
| | |
| Target sequence 23: AAGAAGAUCAAGUCUUUUGAG | 74 |
| Sense strand siRNA: GAAGAUCAAGUCUUUUGAGUU | 75 |
| Antisense strand siRNA: CUCAAAAGACUUGAUCUUCUU | 76 |
| | |
| Target sequence 24: AAGAUCAAGUCUUUUGAGGUG | 77 |
| Sense strand siRNA: GAUCAAGUCUUUUGAGGUGUU | 78 |
| Antisense strand siRNA: CACCUCAAAAGACUUGAUCUU | 79 |
| | |
| Target sequence 25: AAGUCUUUUGAGGUGGUCUUU | 80 |
| Sense strand siRNA: GUCUUUUGAGGUGGUCUUUUU | 81 |
| Antisense strand siRNA: AAAGACCACCUCAAAAGACUU | 82 |
| | |
| Target sequence 26: AAGGUGUACGGCAGUGGCGAG | 83 |
| Sense strand siRNA: GGUGUACGGCAGUGGCGAGUU | 84 |
| Antisense strand siRNA: CUCGCCACUGCCGUACACCUU | 85 |
| | |
| Target sequence 27: AAGGUGGCUGGCCGGGUGAUA | 86 |
| Sense strand siRNA: GGUGGCUGGCCGGGUGAUAUU | 87 |
| Antisense strand siRNA: UAUCACCCGGCCAGCCACCUU | 88 |
| | |
| Target sequence 28: AAGUUACUCGUGUCAAAGCCG | 89 |
| Sense strand siRNA: GUUACUCGUGUCAAAGCCGUU | 90 |
| Antisense strand siRNA: CGGCUUUGACACGAGUAACUU | 91 |
| | |
| Target sequence 29: AAAGCCGUUAGGAUCCUGGCU | 92 |
| Sense strand siRNA: AGCCGUUAGGAUCCUGGCUUU | 93 |
| Antisense strand siRNA: AGCCAGGAUCCUAACGGCUUU | 94 |
| | |
| Target sequence 30: AAAGUGCUUUGGAUGCAGGGA | 95 |
| Sense strand siRNA: AGUGCUUUGGAUGCAGGGAUU | 96 |
| Antisense strand siRNA: UCCCUGCAUCCAAAGCACUUU | 97 |
| | |
| Target sequence 31: AAACAGACUUCGGAGUACCUG | 98 |
| Sense strand siRNA: ACAGACUUCGGAGUACCUGUU | 99 |
| Antisense strand siRNA: CAGGUACUCCGAAGUCUGUUU | 100 |
| | |
| Target sequence 32: AAGACACGCUUCUUCUGGAAG | 101 |
| Sense strand siRNA: GACACGCUUCUUCUGGAAGUU | 102 |
| Antisense strand siRNA: CUUCCAGAAGAAGCGUGUCUU | 103 |
| | |
| Target sequence 33: AAGACCAGCCAACAGGUGAGA | 104 |
| Sense strand siRNA: GACCAGCCAACAGGUGAGAUU | 105 |
| Antisense strand siRNA: UCUCACCUGUUGGCUGGUCUU | 106 |
| | |
| Target sequence 34: AACAGGUGAGAAUGAGAUGGU | 107 |
| Sense strand siRNA: CAGGUGAGAAUGAGAUGGUUU | 108 |
| Antisense strand siRNA: ACCAUCUCAUUCUCACCUGUU | 109 |
| | |
| Target sequence 35: AAUGAGAUGGUGAUCAUGAGA | 110 |
| Sense strand siRNA: UGAGAUGGUGAUCAUGAGAUU | 111 |
| Antisense strand siRNA: UCUCAUGAUCACCAUCUCAUU | 112 |
| | |
| Target sequence 36: AAACAAAUAUGAGUACAAGUU | 113 |
| Sense strand siRNA: ACAAAUAUGAGUACAAGUUUU | 114 |
| Antisense strand siRNA: AACUUGUACUCAUAUUUGUUU | 115 |
| | |
| Target sequence 37: AAAUAUGAGUACAAGUUCGGC | 116 |
| Sense strand siRNA: AUAUGAGUACAAGUUCGGCUU | 117 |
| Antisense strand siRNA: GCCGAACUUGUACUCAUAUUU | 118 |
| | |
| Target sequence 38: AAGUUCGGCUUUGAGCUUCCU | 119 |
| Sense strand siRNA: GUUCGGCUUUGAGCUUCCUUU | 120 |
| Antisense strand siRNA: AGGAAGCUCAAAGCCGAACUU | 121 |
| | |
| Target sequence 39: AAUAUGGGUGUGUAGACUACU | 122 |
| Sense strand siRNA: UAUGGGUGUGUAGACUACUUU | 123 |
| Antisense strand siRNA: AGUAGUCUACACACCCAUAUU | 124 |
| | |
| Target sequence 40: AAGGCUUUUCUUGACCGCCCG | 125 |
| Sense strand siRNA: GGCUUUUCUUGACCGCCCGUU | 156 |
| Antisense strand siRNA: CGGGCGGUCAAGAAAAGCCUU | 127 |
| | |
| Target sequence 41: AAACUUUGAAGUAGUGGAUCU | 128 |
| Sense strand siRNA: ACUUUGAAGUAGUGGAUCUUU | 129 |
| Antisense strand siRNA: AGAUCCACUACUUCAAAGUUU | 130 |
| | |
| Target sequence 42: AAGUAGUGGAUCUGGUGGAUG | 131 |
| Sense strand siRNA: GUAGUGGAUCUGGUGGAUGUU | 132 |
| Antisense strand siRNA: CAUCCACCAGAUCCACUACUU | 133 |
| | |
| Target sequence 43: AAUACCCCUGAUUUAAUGGCA | 134 |
| Sense strand siRNA: UACCCCUGAUUUAAUGGCAUU | 135 |
| Antisense strand siRNA: UGCCAUUAAAUCAGGGGUAUU | 136 |
| | |
| Target sequence 44: AAUGGCACCUGUGUCUGCUAA | 137 |
| Sense strand siRNA: UGGCACCUGUGUCUGCUAAUU | 138 |
| Antisense strand siRNA: UUAGCAGACACAGGUGCCAUU | 139 |
| | |
| Target sequence 45: AAGAAAGUUUCCUGCAUGUUC | 140 |
| Sense strand siRNA: GAAAGUUUCCUGCAUGUUCUU | 141 |
| Antisense strand siRNA: GAACAUGCAGGAAACUUUCUU | 142 |
| | |
| Target sequence 46: AAAGUUUCCUGCAUGUUCAUU | 143 |
| Sense strand siRNA: AGUUUCCUGCAUGUUCAUUUU | 144 |
| Antisense strand siRNA: AAUGAACAUGCAGGAAACUUU | 145 |
| | |
| Target sequence 47: AAGGAUUCUGUGAAGGUGAUG | 146 |
| Sense strand siRNA: GGAUUCUGUGAAGGUGAUGUU | 147 |
| Antisense strand siRNA: CAUCACCUUCACAGAAUCCUU | 148 |
| | |
| Target sequence 48: AAGGUGAUGAGAUUUCCAUCC | 149 |
| Sense strand siRNA: GGUGAUGAGAUUUCCAUCCUU | 150 |
| Antisense strand siRNA: GGAUGGAAAUCUCAUCACCUU | 151 |
| | |
| Target sequence 49: AAUACAUGUUCCCGAAUUGUG | 152 |
| Sense strand siRNA: UACAUGUUCCCGAAUUGUGUU | 153 |
| Antisense strand siRNA: CACAAUUCGGGAACAUGUAUU | 154 |
| | |
| Target sequence 50: AAUUGUGGUCCCCAAAGCUGC | 155 |
| Sense strand siRNA: UUGUGGUCCCCAAAGCUGCUU | 156 |
| Antisense strand siRNA: GCAGCUUUGGGGACCACAAUU | 157 |
| | |
| Target sequence 51: AAAGCUGCCAUUGUGGCCCGC | 158 |
| Sense strand siRNA: AGCUGCCAUUGUGGCCCGCUU | 159 |
| Antisense strand siRNA: GCGGGCCACAAUGGCAGCUUU | 160 |
| | |
| Target sequence 52: AAUGGCCAGACCAAGGUGCUG | 161 |
| Sense strand siRNA: UGGCCAGACCAAGGUGCUGUU | 162 |
| Antisense strand siRNA: CAGCACCUUGGUCUGGCCAUU | 163 |
| | |
| Target sequence 53: AAGGUGCUGACUCAGAAGUUG | 164 |
| Sense strand siRNA: GGUGCUGACUCAGAAGUUGUU | 165 |
| Antisense strand siRNA: CAACUUCUGAGUCAGCACCUU | 166 |
| | |
| Target sequence 54: AAGUUGUCAUCAGUCAGAGGC | 167 |
| Sense strand siRNA: GUUGUCAUCAGUCAGAGGCUU | 168 |
| Antisense strand siRNA: GCCUCUGACUGAUGACAACUU | 169 |
| | |
| Target sequence 55: AAUCAUAUUAUCUCAGGGACA | 170 |
| Sense strand siRNA: UCAUAUUAUCUCAGGGACAUU | 171 |
| Antisense strand siRNA: UGUCCCUGAGAUAAUAUGAUU | 172 |
| | |
| Target sequence 56: AAGAGCCUUCGGGUUCAGAAG | 173 |
| Sense strand siRNA: GAGCCUUCGGGUUCAGAAGUU | 174 |
| Antisense strand siRNA: CUUCUGAACCCGAAGGCUCUU | 175 |
| | |
| Target sequence 57: AAGAUCAGGCCUUCUAUCCUG | 176 |
| Sense strand siRNA: GAUCAGGCCUUCUAUCCUGUU | 177 |
| Antisense strand siRNA: CAGGAUAGAAGGCCUGAUCUU | 178 |
| | |
| Target sequence 58: AACAUCCUUCGAGUUGAAUAU | 179 |
| Sense strand siRNA: CAUCCUUCGAGUUGAAUAUUU | 180 |
| Antisense strand siRNA: AUAUUCAACUCGAAGGAUGUU | 181 |
| | |
| Target sequence 59: AAUAUUCCUUACUGAUCUAUG | 182 |
| Sense strand siRNA: UAUUCCUUACUGAUCUAUGUU | 183 |
| Antisense strand siRNA: CAUAGAUCAGUAAGGAAUAUU | 184 |
| | |
| Target sequence 60: AAGAAGGUCAUCCUUGACCUG | 185 |
| Sense strand siRNA: GAAGGUCAUCCUUGACCUGUU | 186 |
| Antisense strand siRNA: CAGGUCAAGGAUGACCUUCUU | 187 |
| | |
| Target sequence 61: AAGGUCAUCCUUGACCUGCCC | 188 |
| Sense strand siRNA: GGUCAUCCUUGACCUGCCCUU | 189 |
| Antisense strand siRNA: GGGCAGGUCAAGGAUGACCUU | 190 |
| | |
| Target sequence 62: AAUUGGCAGCAGAUCAGGUCU | 191 |
| Sense strand siRNA: UUGGCAGCAGAUCAGGUCUUU | 192 |
| Antisense strand siRNA: AGACCUGAUCUGCUGCCAAUU | 193 |
| | |
| Target sequence 63: AAGCAGCAGAACAUCCAGCAU | 194 |
| Sense strand siRNA: GCAGCAGAACAUCCAGCAUUU | 195 |
| Antisense strand siRNA: AUGCUGGAUGUUCUGCUGCUU | 196 |
| | |
| Target sequence 64: AACAUCCAGCAUGGCCAGCCG | 197 |
| Sense strand siRNA: CAUCCAGCAUGGCCAGCCGUU | 198 |
| Antisense strand siRNA: CGGCUGGCCAUGCUGGAUGUU | 199 |
| | |
| Target sequence 65: AACCAGCUCUGAGAUGAGUUG | 200 |
| Sense strand siRNA: CCAGCUCUGAGAUGAGUUGUU | 201 |
| Antisense strand siRNA: CAACUCAUCUCAGAGCUGGUU | 202 |
| | |
| Target sequence 66: AACAUCCCUGAUACCCCAGAA | 203 |
| Sense strand siRNA: CAUCCCUGAUACCCCAGAAUU | 204 |
| Antisense strand siRNA: UUCUGGGGUAUCAGGGAUGUU | 205 |
| | |
| Target sequence 67: AAGCUCCUCCCUGCUAUAUGG | 206 |
| Sense strand siRNA: GCUCCUCCCUGCUAUAUGGUU | 207 |
| Antisense strand siRNA: CCAUAUAGCAGGGAGGAGCUU | 208 |
| | |
| Target sequence 68: AAGAUCACCGAUUGGAGAGCC | 209 |
| Sense strand siRNA: GAUCACCGAUUGGAGAGCCUU | 210 |
| Antisense strand siRNA: GGCUCUCCAAUCGGUGAUCUU | 211 |
| | |
| Target sequence 69: AACCACUCCUCUGCUAGAUGA | 212 |
| Sense strand siRNA: CCACUCCUCUGCUAGAUGAUU | 213 |
| Antisense strand siRNA: UCAUCUAGCAGAGGAGUGGUU | 214 |
| | |
| Target sequence 70: AAGACAGCCCUAUCUUUAUGU | 215 |
| Sense strand siRNA: GACAGCCCUAUCUUUAUGUUU | 216 |
| Antisense strand siRNA: ACAUAAAGAUAGGGCUGUCUU | 217 |
| | |
| Target sequence 71: AAGUUCAUGCCACCACCGACU | 218 |
| Sense strand siRNA: GUUCAUGCCACCACCGACUUU | 219 |
| Antisense strand siRNA: AGUCGGUGGUGGCAUGAACUU | 220 |
| | |
| Target sequence 72: AACAACAAUGUGCAGUGAGCA | 221 |
| Sense strand siRNA: CAACAAUGUGCAGUGAGCAUU | 222 |
| Antisense strand siRNA: UGCUCACUGCACAUUGUUGUU | 223 |
| | |
| Target sequence 73: AACAAUGUGCAGUGAGCAUGU | 224 |
| Sense strand siRNA: CAAUGUGCAGUGAGCAUGUUU | 225 |
| Antisense strand siRNA: ACAUGCUCACUGCACAUUGUU | 226 |
| | |
| Target sequence 74: AAUGUGCAGUGAGCAUGUGGA | 227 |
| Sense strand siRNA: UGUGCAGUGAGCAUGUGGAUU | 228 |
| Antisense strand siRNA: UCCACAUGCUCACUGCACAUU | 229 |
| | |
| Target sequence 75: AAGAAGCAGCUUUACCUACUU | 230 |
| Sense strand siRNA: GAAGCAGCUUUACCUACUUUU | 231 |
| Antisense strand siRNA: AAGUAGGUAAAGCUGCUUCUU | 232 |
| | |
| Target sequence 76: AAGCAGCUUUACCUACUUGUU | 233 |
| Sense strand siRNA: GCAGCUUUACCUACUUGUUUU | 234 |
| Antisense strand siRNA: AACAAGUAGGUAAAGCUGCUU | 235 |
| | |
| Target sequence 77: AACAGUCUCUGCAAUGGAGUG | 236 |
| Sense strand siRNA: CAGUCUCUGCAAUGGAGUGUU | 237 |
| Antisense strand siRNA: CACUCCAUUGCAGAGACUGUU | 238 |
| | |
| Target sequence 78: AAUGGAGUGUGGGUCCACCUU | 239 |
| Sense strand siRNA: UGGAGUGUGGGUCCACCUUUU | 240 |
| Antisense strand siRNA: AAGGUGGACCCACACUCCAUU | 241 |
| | |
| Target sequence 79: AAUGUAGGAGGUGGUCAGCAG | 242 |
| Sense strand siRNA: UGUAGGAGGUGGUCAGCAGUU | 243 |
| Antisense strand siRNA: CUGCUGACCACCUCCUACAUU | 244 |
| | |
| Target sequence 80: AAUCUCCUGGGCCUUAAAGGA | 245 |
| Sense strand siRNA: UCUCCUGGGCCUUAAAGGAUU | 246 |
| Antisense strand siRNA: UCCUUUAAGGCCCAGGAGAUU | 247 |
| | |
| Target sequence 81: AAAGGAUGCGGACUCAUCCUC | 248 |
| Sense strand siRNA: AGGAUGCGGACUCAUCCUCUU | 249 |
| Antisense strand siRNA: GAGGAUGAGUCCGCAUCCUUU | 250 |
| | |
| Target sequence 82: AAACUCAGGCCCAUCCAUUUU | 251 |
| Sense strand siRNA: ACUCAGGCCCAUCCAUUUUUU | 252 |
| Antisense strand siRNA: AAAAUGGAUGGGCCUGAGUUU | 253 |
| | |
| Target sequence 83: AAUUGAGGCCUUUUCGAUAGU | 254 |
| Sense strand siRNA: UUGAGGCCUUUUCGAUAGUUU | 255 |
| Antisense strand siRNA: ACUAUCGAAAAGGCCUCAAUU | 256 |
| | |
| Target sequence 84: AAAUGGCCUCCUGGCGUAAGC | 257 |
| Sense strand siRNA: AUGGCCUCCUGGCGUAAGCUU | 258 |
| Antisense strand siRNA: GCUUACGCCAGGAGGCCAUUU | 259 |
| | |
| Target sequence 85: AAGCUUUUCAAGGUUUUUUGG | 260 |
| Sense strand siRNA: GCUUUUCAAGGUUUUUUGGUU | 261 |
| Antisense strand siRNA: CCAAAAAACCUUGAAAAGCUU | 262 |
| | |
| Target sequence 86: AAGGUUUUUUGGAGGCUUUUU | 263 |
| Sense strand siRNA: GGUUUUUUGGAGGCUUUUUUU | 264 |
| Antisense strand siRNA: AAAAAGCCUCCAAAAAACCUU | 265 |
| | |
| Target sequence 87: AAAUUGUGAUAGGAACUUUGG | 266 |
| Sense strand siRNA: AUUGUGAUAGGAACUUUGGUU | 267 |
| Antisense strand siRNA: CCAAAGUUCCUAUCACAAUUU | 268 |
| | |
| Target sequence 88: AACUUUGGACCUUGAACUUAU | 269 |
| Sense strand siRNA: CUUUGGACCUUGAACUUAUUU | 270 |
| Antisense strand siRNA: AUAAGUUCAAGGUCCAAAGUU | 271 |
| | |
| Target sequence 89: AACUUAUGUAUCAUGUGGAGA | 272 |
| Sense strand siRNA: CUUAUGUAUCAUGUGGAGAUU | 273 |
| Antisense strand siRNA: UCUCCACAUGAUACAUAAGUU | 274 |
| | |
| Target sequence 90: AAGAGCCAAUUUAACAAACUA | 275 |
| Sense strand siRNA: GAGCCAAUUUAACAAACUAUU | 276 |
| Antisense strand siRNA: UAGUUUGUUAAAUUGGCUCUU | 277 |
| | |
| Target sequence 91: AAUUUAACAAACUAGGAAGAU | 278 |
| Sense strand siRNA: UUUAACAAACUAGGAAGAUUU | 279 |
| Antisense strand siRNA: AUCUUCCUAGUUUGUUAAAUU | 280 |
| | |
| Target sequence 92: AAUCAGUGUUUCCCCUUUGUG | 281 |
| Sense strand siRNA: UCAGUGUUUCCCCUUUGUGUU | 282 |
| Antisense strand siRNA: CACAAAGGGGAAACACUGAUU | 283 |
| | |
| Target sequence 93: AAACCUUCUAGAGCUGAUUUG | 284 |
| Sense strand siRNA: ACCUUCUAGAGCUGAUUUGUU | 285 |
| Antisense strand siRNA: CAAAUCAGCUCUAGAAGGUUU | 286 |
| | |
| Target sequence 94: AAUGGAGAGAGCUUUCCCUGU | 287 |
| Sense strand siRNA: UGGAGAGAGCUUUCCCUGUUU | 288 |
| Antisense strand siRNA: ACAGGGAAAGCUCUCUCCAUU | 289 |
| | |
| Target sequence 95: AAGGAAGCUAGCUGCUCUACG | 290 |
| Sense strand siRNA: GGAAGCUAGCUGCUCUACGUU | 291 |
| Antisense strand siRNA: CGUAGAGCAGCUAGCUUCCUU | 292 |
| | |
| Target sequence 96: AAGCUAGCUGCUCUACGGUCA | 293 |
| Sense strand siRNA: GCUAGCUGCUCUACGGUCAUU | 294 |
| Antisense strand siRNA: UGACCGUAGAGCAGCUAGCUU | 295 |
| | |
| Target sequence 97: AAGAGUAUACUUUAACCUGGC | 296 |
| Sense strand siRNA: GAGUAUACUUUAACCUGGCUU | 297 |
| Antisense strand siRNA: GCCAGGUUAAAGUAUACUCUU | 298 |
| | |
| Target sequence 98: AACCUGGCUUUUAAAGCAGUA | 299 |
| Sense strand siRNA: CCUGGCUUUUAAAGCAGUAUU | 300 |
| Antisense strand siRNA: UACUGCUUUAAAAGCCAGGUU | 301 |
| | |
| Target sequence 99: AAAGCAGUAGUAACUGCCCCA | 302 |
| Sense strand siRNA: AGCAGUAGUAACUGCCCCAUU | 303 |
| Antisense strand siRNA: UGGGGCAGUUACUACUGCUUU | 304 |
| | |
| Target sequence 100: AACUGCCCCACCAAAGGUCUU | 305 |
| Sense strand siRNA: CUGCCCCACCAAAGGUCUUUU | 306 |
| Antisense strand siRNA: AAGACCUUUGGUGGGGCAGUU | 307 |
| | |
| Target sequence 101: AAGCCAUUUUUGGAGCCUAUU | 308 |
| Sense strand siRNA: GCCAUUUUUGGAGCCUAUUUU | 309 |
| Antisense strand siRNA: AAUAGGCUCCAAAAAUGGCUU | 310 |
| | |
| Target sequence 102: AAAUAUUUUCAUAUGGGAGGA | 311 |
| Sense strand siRNA: AUAUUUUCAUAUGGGAGGAUU | 312 |
| Antisense strand siRNA: UCCUCCCAUAUGAAAAUAUUU | 313 |
| | |
| Target sequence 103: AAGUCCUUGGAAUUGAUUCUA | 314 |
| Sense strand siRNA: GUCCUUGGAAUUGAUUCUAUU | 315 |
| Antisense strand siRNA: UAGAAUCAAUUCCAAGGACUU | 316 |
| | |
| Target sequence 104: AAUUGAUUCUAAGGUGAUGUU | 317 |
| Sense strand siRNA: UUGAUUCUAAGGUGAUGUUUU | 318 |
| Antisense strand siRNA: AACAUCACCUUAGAAUCAAUU | 319 |
| | |
| Target sequence 105: AAGGUGAUGUUCUUAGCACUU | 320 |
| Sense strand siRNA: GGUGAUGUUCUUAGCACUUUU | 321 |
| Antisense strand siRNA: AAGUGCUAAGAACAUCACCUU | 322 |
| | |
| Target sequence 106: AAUUCCUGUCAAAUUUUUUGU | 323 |
| Sense strand siRNA: UUCCUGUCAAAUUUUUUGUUU | 324 |
| Antisense strand siRNA: ACAAAAAAUUUGACAGGAAUU | 325 |
| | |
| Target sequence 107: AAAUUUUUUGUUCUCCCCUUC | 326 |
| Sense strand siRNA: AUUUUUUGUUCUCCCCUUCUU | 327 |
| Antisense strand siRNA: GAAGGGGAGAACAAAAAAUUU | 328 |
| | |
| Target sequence 108: AAAUGUAAGCUGAAACUGGUC | 329 |
| Sense strand siRNA: AUGUAAGCUGAAACUGGUCUU | 330 |
| Antisense strand siRNA: GACCAGUUUCAGCUUACAUUU | 331 |
| | |
| Target sequence 109: AAGCUGAAACUGGUCUACUGU | 332 |
| Sense strand siRNA: GCUGAAACUGGUCUACUGUUU | 333 |
| Antisense strand siRNA: ACAGUAGACCAGUUUCAGCUU | 334 |
| | |
| Target sequence 110: AAACUGGUCUACUGUGUCUCU | 335 |
| Sense strand siRNA: ACUGGUCUACUGUGUCUCUUU | 336 |
| Antisense strand siRNA: AGAGACACAGUAGACCAGUUU | 337 |
| | |
| Target sequence 111: AAUUUUACUACUUUUGAGAUU | 338 |
| Sense strand siRNA: UUUUACUACUUUUGAGAUUUU | 339 |
| Antisense strand siRNA: AAUCUCAAAAGUAGUAAAAUU | 340 |
| | |
| Target sequence 112: AAUGUACAGAAUUAUAUAAUU | 341 |
| Sense strand siRNA: UGUACAGAAUUAUAUAAUUUU | 342 |
| Antisense strand siRNA: AAUUAUAUAAUUCUGUACAUU | 343 |
| | |
| Target sequence 113: AAUUAUAUAAUUCUAACGCUU | 344 |
| Sense strand siRNA: UUAUAUAAUUCUAACGCUUUU | 345 |
| Antisense strand siRNA: AAGCGUUAGAAUUAUAUAAUU | 346 |
| | |
| Target sequence 114: AAUUCUAACGCUUAAAUCAUG | 347 |
| Sense strand siRNA: UUCUAACGCUUAAAUCAUGUU | 348 |
| Antisense strand siRNA: CAUGAUUUAAGCGUUAGAAUU | 349 |
| | |
| Target sequence 115: AACGCUUAAAUCAUGUGAAAG | 350 |
| Sense strand siRNA: CGCUUAAAUCAUGUGAAAGUU | 351 |
| Antisense strand siRNA: CUUUCACAUGAUUUAAGCGUU | 352 |
| | |
| Target sequence 116: AAAUCAUGUGAAAGGGUUGCU | 353 |
| Sense strand siRNA: AUCAUGUGAAAGGGUUGCUUU | 354 |
| Antisense strand siRNA: AGCAACCCUUUCACAUGAUUU | 355 |
| | |
| Target sequence 117: AAAGGGUUGCUGCUGUCAGCC | 356 |
| Sense strand siRNA: AGGGUUGCUGCUGUCAGCCUU | 357 |
| Antisense strand siRNA: GGCUGACAGCAGCAACCCUUU | 358 |
| | |
| Target sequence 118: AAACCCAAGGAGGAACUCUUG | 359 |
| Sense strand siRNA: ACCCAAGGAGGAACUCUUGUU | 360 |
| Antisense strand siRNA: CAAGAGUUCCUCCUUGGGUUU | 361 |
| | |
| Target sequence 119: AAGGAGGAACUCUUGAUCAAG | 362 |
| Sense strand siRNA: GGAGGAACUCUUGAUCAAGUU | 363 |
| Antisense strand siRNA: CUUGAUCAAGAGUUCCUCCUU | 364 |
| | |
| Target sequence 120: AACUCUUGAUCAAGAUGCCGA | 365 |
| Sense strand siRNA: CUCUUGAUCAAGAUGCCGAUU | 366 |
| Antisense strand siRNA: UCGGCAUCUUGAUCAAGAGUU | 367 |
| | |
| Target sequence 121: AAGAUGCCGAACCCUGUGUUC | 368 |
| Sense strand siRNA: GAUGCCGAACCCUGUGUUCUU | 369 |
| Antisense strand siRNA: GAACACAGGGUUCGGCAUCUU | 370 |
| | |
| Target sequence 122: AACCCUGUGUUCAGAACCUCC | 371 |
| Sense strand siRNA: CCCUGUGUUCAGAACCUCCUU | 372 |
| Antisense strand siRNA: GGAGGUUCUGAACACAGGGUU | 373 |
| | |
| Target sequence 123: AACCUCCAAAUACUGCCAUGA | 374 |
| Sense strand siRNA: CCUCCAAAUACUGCCAUGAUU | 375 |
| Antisense strand siRNA: UCAUGGCAGUAUUUGGAGGUU | 376 |
| | |
| Target sequence 124: AAAUACUGCCAUGAGAAACUA | 377 |
| Sense strand siRNA: AUACUGCCAUGAGAAACUAUU | 378 |
| Antisense strand siRNA: UAGUUUCUCAUGGCAGUAUUU | 379 |
| | |
| Target sequence 125: AAACUAGAGGGCAGGUCUUCA | 380 |
| Sense strand siRNA: ACUAGAGGGCAGGUCUUCAUU | 381 |
| Antisense strand siRNA: UGAAGACCUGCCCUCUAGUUU | 382 |
| | |
| Target sequence 126: AAGCCCUUUGAACCCCCUUCC | 383 |
| Sense strand siRNA: GCCCUUUGAACCCCCUUCCUU | 384 |
| Antisense strand siRNA: GGAAGGGGGUUCAAAGGGCUU | 385 |
| | |
| Target sequence 127: AACCCCCUUCCUGCCCUGUGU | 386 |
| Sense strand siRNA: CCCCCUUCCUGCCCUGUGUUU | 387 |
| Antisense strand siRNA: ACACAGGGCAGGAAGGGGGUU | 388 |
| | |
| Target sequence 128: AAAGUGUGGGCUGAAGAUGGU | 389 |
| Sense strand siRNA: AGUGUGGGCUGAAGAUGGUUU | 390 |
| Antisense strand siRNA: ACCAUCUUCAGCCCACACUUU | 391 |
| | |
| Target sequence 129: AAGAUGGUUGGUUUCAUGUUU | 392 |
| Sense strand siRNA: GAUGGUUGGUUUCAUGUUUUU | 393 |
| Antisense strand siRNA: AAACAUGAAACCAACCAUCUU | 394 |
| | |
| Target sequence 130: AAAGACUAUAUUUUGUACUUA | 395 |
| Sense strand siRNA: AGACUAUAUUUUGUACUUAUU | 396 |
| Antisense strand siRNA: UAAGUACAAAAUAUAGUCUUU | 397 |
| | |
| Target sequence 131: AACCAGAUAUAUUUUUACCCC | 398 |
| Sense strand siRNA: CCAGAUAUAUUUUUACCCCUU | 399 |
| Antisense strand siRNA: GGGGUAAAAAUAUAUCUGGUU | 400 |
| | |
| Target sequence 132: AAUAAAGUUUUUUUAAUGGAA | 401 |
| Sense strand siRNA: UAAAGUUUUUUUAAUGGAAUU | 402 |
| Antisense strand siRNA: UUCCAUUAAAAAAACUUUAUU | 403 |
| | |

Due to the large number of aptamers that may be screened, a screening assay described herein may be performed using arrays of aptamers, and those arrays capable of modifying a phenotype of the knockout of the invention rescreened to determine the specific aptamer of interest.

### Small molecules

The present application additionally contemplates small molecules that are capable of entering a cell to thereby alleviate the vascular complications of diabetes or hyperglycemia e.g., to modulate angiogenic potential and/or activity and/or neovascularization potential and/or activity, or alternatively or in addition, capable of enhancing vascular network formation and/or development e.g., in an appropriate animal model.

Exemplary small molecule regulators of TXNIP include organic or inorganic compounds that are not a nucleic acids, proteins, or peptides. The present application contemplates the use of any small molecule(s) or small molecule library.

An example of a suitable small molecule library is described, for example, in USSN 6,168,192. The method described is for producing a multidimensional chemical library that comprises converting a set of at least two different allylic carbonyl monomers to form monomer derivatives by converting the allyl group to another group and covalently linking at least two of the produced monomers to form oligomers.

In an alternative method, McMillan et al., (Proc Natl Acad Sci USA. 97: 1506-1511, 2000) describes the production of an encoded chemical library (ECLiPS method) based on a pyrimidine-imidazole core prepared on polyethylene glycol-grafted polystyrene support. Compounds are attached to resin by a photolabile O-nitrobenzyl amide linker. The first synthetic step introduced primary amines. After a pool and split step, the amines are acylated with fluorenylmethoxycarbonyl (Fmoc)-protected amino acids. A second pool and split step is performed, followed by Fmoc deprotection and subsequent hetero-arylation of the resulting free amines by electrophilic substitution with a set of nine substituted pyrimidines. The library produced comprised 8,649 compounds.

Additional small molecule libraries include, for example, libraries comprising statine esters (USSN 6,255,120), moeomycin analogs (USSN 6,207,820), fused 2, 4-pyrimidinediones (USSN 6,025,371), dihydrobenzopyran based molecules (USSN 6,017,768), 1,4-benzodiazepin-2,5-dione based compounds (USSN 5,962,337), benzofuran derivatives (USSN 5,919,955), indole derivatives (USSN 5,856,496), products of polyketides (USSN 5,712,146), morpholino compounds (USSN 5,698,685) or sulphonamide compounds (USSN 5,618,825).

Compounds can be screened using high throughput screening techniques, such as, for example, sequential high throughput screening (SHTS). SHTS is an iterative process of screening a sample of compounds for activity, analyzing the results, and selecting a new set of compounds for screening, based on compounds identified in one or more previous screens. Selection of compounds is driven by finding structure activity relationships (SARs) within the screened compounds and using those relationships to drive further selection.

Recursive partitioning (RP) is a statistical methodology that can be used in conjunction with high-throughput screening techniques, such as, SHTS, by identifying relationships between specific chemical structural features of the molecules and biological activity. The premise of this method is that the biological activity of a compound is a consequence of its molecular structure. Accordingly, it is useful to identify those aspects of molecular structure that are relevant to a particular biological activity. By gaining a better understanding of the mechanism by which the compound acts, additional compounds for screening can more accurately be selected. Suitable RP methods are described, for example in Hawkins, D. M. and Kass, G. V., (In: Automatic Interaction Detection. In Topics in Applied Multivariate Analysis; Hawkins, D. H., Ed.; 1982, Cambridge University Press, pp. 269-302).

Quantitative structure activity relationship (QSAR) is also useful for determining a feature or features of a compound required for or useful for a desired biological activity. QSAR models are determined using sets of compounds whose molecular structure and biological activity are known, a training set. QSAR approaches are either linear or nonlinear. The linear approach assumes that the activity varies linearly with the level of whatever features affect it, and that there are no interactions among the different features.

Nonlinear QSAR approaches account for the fact that activity can result from threshold effects; a feature must be present for at least some threshold level for activity to occur. Furthermore, as interactions between features are observed in many QSAR settings, the utility of one feature depends upon the presence of another. For example, activity may require the simultaneous presence of two features.

In one example, the small molecule is selected from one or a plurality of compounds that regulate TXNIP e.g., one or more of resveratrol, diazoxide, verapamil, exenatide, nitric oxide, triethylene glycol dimethacrylate, Boc5 and SP4.

In another example, the present application describes the use of one or a plurality of compounds that regulate TXNIP e.g., one or more of resveratrol, diazoxide, verapamil, exenatide, nitric oxide, triethylene glycol dimethacrylate, Boc5 and SP4 in the preparation of a medicament for the treatment of a vascular complication of diabetes mellitus or in the treatment of a vascular disease associated with diabetes mellitus or other condition associated with impaired glucose tolerance and/or hyperglycemia, or to other wise promote vascular repair in a subject.

In a particular example, one or a plurality of compounds that regulate TXNIP e.g., one or more of resveratrol, diazoxide, verapamil, exenatide, nitric oxide, triethylene glycol dimethacrylate, Boc5 and SP4 is employed to promote vascular repair in a subject, wherein the compound(s) is/are administered to a subject in need thereof for a time and under conditions sufficient to promote vascular repair e.g., by enhancing mobilization of cells that participate in vascular repair and/or enhancing angiogenic processes or neovascularisation processes. In one example, a compound is formulated for administration by the intramuscular route e.g., once or twice daily.

In another example, one or a plurality of compounds that regulate TXNIP e.g., one or more of resveratrol, diazoxide, verapamil, exenatide, nitric oxide, triethylene glycol dimethacrylate, Boc5 and SP4 is formulated for administration by the subcutaneous route e.g., once or twice daily.

In another example, one or a plurality of compounds that regulate TXNIP e.g., one or more of resveratrol, diazoxide, verapamil, exenatide, nitric oxide, triethylene glycol dimethacrylate, Boc5 and SP4 is formulated for oral administration e.g., once or twice daily.

In another example, the present application describes the use of a non-peptidic GLP-1 receptor agonist e.g., compound Boc5 or SP4 (Chen et al., Proc Natl Acad Sci USA 104, 943-948, 2007) in the preparation of a medicament for the treatment of a vascular complication of diabetes mellitus or in the treatment of a vascular disease associated with diabetes mellitus or other condition associated with impaired glucose tolerance and/or hyperglycemia, or to other wise promote vascular repair in a subject.

In a particular example, a non-peptidic GLP-1 receptor agonist e.g., Boc5 and/or SP4 is employed to promote vascular repair in a subject, wherein the compound(s) is/are administered to a subject in need thereof for a time and under conditions sufficient to promote vascular repair e.g., by enhancing mobilization of cells that participate in vascular repair and/or enhancing angiogenic processes or neovascularisation processes.

In one example, a compound is formulated for administration by the intramuscular route e.g., once or twice daily.

In another example, a compound is formulated for administration by the subcutaneous route e.g., once or twice daily. In another example, a compound is formulated for oral administration e.g., once or twice daily.

### Cell-based modulators

The present application also describes a composition comprising a cell, e.g., a stem cell or EPC comprising and/or expressing a modulator of TRX and/or modulator of TXNIP expression and/or activity and/or level or otherwise capable of enhancing vascular repair or preventing or reducing vascular complication(s) or degeneration..

In one example, the cell is an endothelial cell e.g., OEC, or an EPC. Such a cell may be isolated from an autologous subject or allogeneic of xenogeneic subject to the subject being treated. Autologous cells have the advantage of being less prone to rejection compared to other allogenic (or xenogeneic) cells. Also, the use of autologous cells avoids any issue of "doping" (e.g., with "foreign" DNA).

EPCs may be isolated from a healthy subject i.e., they are normal EPCs having proliferative ability with respect to vascular formation. It will be appreciated that some of the cells may be saved for use at a later date, and typically such cells are frozen under conditions that retains their viability. It will be appreciated that the cells may be obtained and enriched (expanded if necessary) before vascular complications arise or are apparent in a subject, and kept for immediate administration when necessary.

Alternatively, an EPC is isolated from a subject in need of treatment, and transformed, transfected or transduced with a nucleic acid capable of expressing a peptide or polypeptide modulator of TRX and/or TXNIP. Methods for isolating and/or administering EPCs will be apparent to the skilled artisan and/or described herein.

For example, a natural source of EPCs includes bone marrow (e.g., with and without previous bleeding), peripheral blood (e.g., with and without enhancement from marrow), and peripheral blood mononuclear cells. Other sources are not to be excluded.

EPCs may be isolated from such a source by any suitable method, typically involving cell fractionation and concentration. Suitable methods include Ficoll-Paque methodology or concentration of EPCs using antibodies directed to EPC markers which are immobilized, for example in an affinity chromatography column or to a substratum in a "panning" scheme.

In one example, a cell is isolated from a subject, and is transfected with an expression vector or expression construct comprising a nucleic acid encoding an inhibitor of TXNIP expression operably linked to a promoter active in said cell. In one example, the cell is additionally transfected with a nucleic acid encoding an agonist of TRX expression. Methods for transfecting cells will be apparent to the skilled artisan and/or described herein and/or described in International Patent Application No. PCT/AU2006/000550. The resulting recombinant cell is then administered to a subject using a method described herein.

As will be apparent to the skilled artisan based on the foregoing description, the present application also describes a method additionally comprising isolating or obtaining an EPC. Such a method may additionally comprise producing an EPC comprising or expressing a modulator of TRX or TXNIP as described herein above, e.g., by performing a process comprising transforming or transfecting an EPC with nucleic acid.

### Production of compounds

In one embodiment, the present application describes a process for providing a therapeutic compound capable of modulating angiogenic potential and/or activity and/or capable of modulating neovascularization potential and/or activity and/or enhancing vascular network formation and/or development, said process comprising:
(i) identifying a compound using a method described herein;
(ii) optionally, determining the structure of the compound; and
(iii) providing the compound or the name or structure of the compound.

Naturally, for compounds that are known albeit not previously tested for their function using a screen provided by the present invention, determination of the structure of the compound is implicit in step (i) supra. This is because the skilled artisan will be aware of the name and/or structure of the compound at the time of performing the screen.

As used herein, the term "providing the compound" shall be taken to include any chemical or recombinant synthetic means for producing said compound or alternatively, the provision of an agent that has been previously synthesized by any person or means. Suitable methods for producing a compound are described herein or known in the art.

In a preferred example, the compound or modulator or the name or structure of the compound or modulator is provided with an indication as to its use e.g., as determined by a screen described herein.

In another embodiment, the application describes a process for providing a therapeutic compound, for example, for the treatment of one or more vascular complications of diabetes and/or impaired glucose tolerance and/or hyperglycemia, said process comprising:
(i) identifying a compound using a method described herein;
(ii) optionally, determining the structure of the compound; compound; and
(iii) providing, the compound.

Optionally, the candidate agent is provided with an indication as to its use, for example, as determined using a method described herein.

The present application additionally describes a process for manufacturing a medicament for the treatment or prophylaxis of one or more vascular complications of diabetes and/or impaired glucose tolerance and/or hyperglycemia, said process comprising:
(i) identifying a compound using a method described herein
(ii) optionally, isolating the compound;
(iii) optionally, providing the name or structure of the compound;
(iv) optionally, providing the compound; and
(v) using the compound in the manufacture of a medicament for one or more vascular complications of diabetes and/or impaired glucose tolerance and/or hyperglycemia.

### Formulations

A compound for the prophylaxis and/or therapy of one more vascular complications of diabetes and/or impaired glucose tolerance and/or hyperglycemia is formulated into a pharmaceutical formulation.

Formulation of a pharmaceutical compound will vary according to the route of administration selected (e.g., solution, emulsion, capsule). An appropriate composition comprising the identified compound to be administered can be prepared in a physiologically acceptable vehicle or carrier or excipient.

The term "carrier or excipient" as used herein, refers to a carrier or excipient that is conventionally used in the art to facilitate the storage, administration, and/or the biological activity of an active compound. A carrier may also reduce any undesirable side effects of the active compound. A suitable carrier is, for example, stable, e.g., incapable of reacting with other ingredients in the formulation. In one example, the carrier does not produce significant local or systemic adverse effect in recipients at the dosages and concentrations employed for treatment. Such carriers and excipients are generally known in the art. Suitable carriers for this invention include those conventionally used, e.g., water, saline, aqueous dextrose, dimethyl sulfoxide (DMSO), and glycols are preferred liquid carriers, particularly (when isotonic) for solutions. Suitable pharmaceutical carriers and excipients include starch, cellulose, glucose, lactose, sucrose, gelatin, malt, rice, flour, chalk, silica gel, magnesium stearate, sodium stearate, glycerol monostearate, sodium chloride, glycerol, propylene glycol, water, ethanol, and the like.

For solutions or emulsions, suitable carriers include, for example, aqueous or alcoholic/aqueous solutions, emulsions or suspensions, including saline and buffered media. Parenteral vehicles can include e.g., sodium chloride solution, Ringer's dextrose, dextrose and sodium chloride, lactated Ringer's or fixed oils.

Injectables can include various additives, preservatives, or fluid, nutrient or electrolyte replenishers and the like (See, generally, Remington's Pharmaceutical Sciences, 17th Edition, Mack Publishing Co., Pa., 1985).

For inhalation, the compound can be solubilized and loaded into a suitable dispenser for administration (e.g., an atomizer, nebulizer or pressurized aerosol dispenser).

Furthermore, where the compound is a protein or peptide or antibody or fragment thereof, the compound can be administered via *in vivo* expression of the recombinant protein. *In vivo* expression can be accomplished via somatic cell expression according to suitable methods (see, e.g. U.S. Pat. No. 5,399,346). In this embodiment, nucleic acid encoding the protein can be incorporated into a retroviral, adenoviral or other suitable vector (preferably, a replication deficient infectious vector) for delivery, or can be introduced into a transfected or transformed host cell capable of expressing the protein for delivery. In the latter embodiment, the cells can be implanted (alone or in a barrier device), injected or otherwise introduced in an amount effective to express the protein in a therapeutically effective amount.

One or more solubilizing agents may be included in the formulation to promote dissolution in aqueous media. Suitable solubilizing agents include e.g., wetting agents such as polysorbates, glycerin, a poloxamer, non-ionic surfactant, ionic surfactant, food acid, food base e.g., sodium bicarbonate, or an alcohol. Buffer salts may also be included for pH control.

Stabilizers are used to inhibit or retard drug decomposition reactions in storage or *in vivo* which include, by way of example, oxidative reactions, hydrolysis and proteolysis. A number of stabilizers may be used e.g., protease inhibitors, polysaccharides such as cellulose and cellulose derivatives, and simple alcohols, such as glycerol; bacteriostatic agents such as phenol, m-cresol and methylparaben; isotonic agents, such as sodium chloride, glycerol, and glucose; lecithins, such as example natural lecithins (e.g. egg yolk lecithin or soya bean lecithin) and synthetic or semisynthetic lecithins (e.g. dimyristoylphosphatidylcholine, dipalmitoylphosphatidylcholine or distearoyl-phosphatidylcholine; phosphatidic acids; phosphatidylethanolamines; phosphatidylserines such as distearoyl-phosphatidylserine, dipalmitoylphosphatidylserine and diarachidoylphospahtidylserine; phosphatidylglycerols; phosphatidylinositols; cardiolipins; sphingomyelins. In one example, the stabilizer may be a combination of glycerol, bacteriostatic agents and isotonic agents.

### Modes of administration

The present invention contemplates any mode of administration of a medicament or formulation as described herein. Combinations of different administration routes are also encompassed.

Inhalable compositions are generally administered in an aqueous solution e.g., as a nasal or pulmonary spray. Preferred systems for dispensing liquids as a nasal spray are disclosed in U.S. Pat. No. 4,511,069. Such formulations may be conveniently prepared by dissolving compositions according to the present invention in water to produce an aqueous solution, and rendering the solution sterile. The formulations may be presented in multi-dose containers, for example in the sealed dispensing system disclosed in U.S. Pat. No. 4,511,069. Other suitable nasal spray delivery systems have been described in Transdermal Systemic Medication, Y. W. Chien Ed., Elsevier Publishers, New York, 1985; and in U.S. Pat. No. 4,778,810. Additional aerosol delivery forms may include, e.g., compressed air-, jet-, ultrasonic-, and piezoelectric nebulizers, which deliver the biologically active agent dissolved or suspended in a pharmaceutical solvent, e.g., water, ethanol, or a mixture thereof.

Mucosal formulations are administered as dry powder formulations e.g., comprising the biologically active agent in a dry, usually lyophilized, form of an appropriate particle size, or within an appropriate particle size range, for intranasal delivery. Minimum particle size appropriate for deposition within the nasal or pulmonary passages is often about 0.5 micron mass median equivalent aerodynamic diameter (MMEAD), commonly about 1 micron MMEAD, and more typically about 2 micron MMEAD. Maximum particle size appropriate for deposition within the nasal passages is often about 10 micron MMEAD, commonly about 8 micron MMEAD, and more typically about 4 micron MMEAD. Intranasally respirable powders within these size ranges can be produced by a variety of conventional techniques, such as jet milling, spray drying, solvent precipitation, supercritical fluid condensation, and the like. These dry powders of appropriate MMEAD can be administered to a patient via a conventional dry powder inhaler (DPI) which rely on the patient's breath, upon pulmonary or nasal inhalation, to disperse the power into an aerosolized amount. Alternatively, the dry powder may be administered via air assisted devices that use an external power source to disperse the powder into an aerosolized amount, e.g., a piston pump.

Standard methods are used to administer injectable formulations of the present invention.

The present invention is described further in the following non-limiting examples:

### EXAMPLE 1 - Materials and Methods

### 1.1 Endothelial cell culture and glucose treatments:

Human umbilical vein endothelial cells (HUVEC) were cultured and expanded for use in investigations at no later than passage 4. Cells were grown under standard culture conditions of 37 °C in a 95% air/5% CO₂ atmosphere in M199 media (Cell Applications) supplemented with 10% foetal bovine serum (FBS; Trace Bioscience) and with a D-glucose concentration of 5.0 mM. For hyperglycemic studies D-glucose (Sigma-Aldrich) was added to the above media and filter-sterilised to give glucose concentrations of 10, 15, 20 and 25 mM for a period of 24 h. Twelve hours prior to experiments, human endothelial cells were cultured in glucose-free media. To examine the effects of hypoxia, cells were grown for 6 to 18 h in a hypoxia incubator (1% O₂/5%CO₂/N₂ balance; Kendro International) at 37 °C. Human recombinant vascular endothelial growth factor (VEGF; Sigma-Aldrich) was added to media at a final concentration of 10 ng/mL where appropriate.

Human coronary artery endothelial cells (HCAECs) were cultured and expanded for use in investigations at no later than passage 4. Cells were grown under standard culture conditions of 37°C in a 95% air/5% CO₂ atmosphere in HCAEC growth media (Cell Applications) supplemented with 10% foetal bovine serum (FBS; Trace Bioscience) and with a D-glucose concentration of 5.0 mM. For hyperglycemic studies D-glucose (Sigma-Aldrich) was added to the above media and filter-sterilised to give glucose concentrations of 10, 15, 20 and 25 mM. Twelve hours prior to experiments, human endothelial cells were cultured in glucose-free media. To examine the effects of hypoxia, cells were grown for 6 to 18 h in a hypoxia incubator (1%O₂/5%CO₂/N₂ balance; Kendro International) at 37°C.

The effects of glucose concentrations on the thioredoxin and TXNIP mRNA and protein expression and thioredoxin activity were studies by RT-PCR, western immunoblotting, and thioredoxin activity assays as described below.

### 1.2 Post transcription Gene-silencing of TRX and TXNIP expression through siRNA:

### 1.2.1 Exemplary mRNA target sequences and siRNAs

Double-stranded 20-mer siRNA for selective silencing of human TRX1 (SEQ ID NO: 1), human TXNIP (SEQ ID NO: 3) or murine TXNIP (SEQ ID NOs: 415 and 416) were produced according to standard procedures as described herein. For targeting expression of TRX1, siRNA was designed to target positions 72-90 of SEQ ID NO: 1 (Genbank accession: NM_003329) by producing sense siRNA comprising the sequence set forth in SEQ ID NO: 5 i.e., GCAGAUCGAGAGCAAGACU, and the corresponding antisense siRNA according to standard procedures as described herein.

For targeting expression of human TXNIP, double-stranded siRNA was designed to target positions 533-551 of SEQ ID NO: 3 by producing sense siRNA comprising the sequence set forth in SEQ ID NO: 6 i.e., ACAGACUUCGGAGUACCUG, and the corresponding antisense siRNA according to standard procedures as described herein, however the same protocol is readily followed without undue experimentation employing the sense siRNA and antisense siRNA sequences presented in Table 1 hereof. For delivery *in vivo,* DNA comprising the sequence of an exemplified siRNA construct is produced and sub-cloned into a suitable mammalian expression vector using standard procedures.

For targeting expression of murine TXNIP, double-stranded siRNA was designed to target positions 778-796 of SEQ ID NO: 415 and positions 774-792 of SEQ ID NO: 416 by producing a DNA construct capable of encoding double-stranded siRNA/shRNA, wherein the DNA comprised the sequence set forth in SEQ ID NO: 417 i.e., comprising sequence encoding the sense siRNA and antisense siRNA with intervening loop sequence of SEQ ID NO: 414, according to standard procedures as described herein. The sequence set forth in SEQ ID NO: 417 also comprises suitable restriction sites for sub-cloning into a suitable mammalian expression vector *via Bam*HI and *Hin*dIII restriction sites. The exemplified construct comprising SEQ ID NO: 417 was designated pRNA-U6.1/neo.

As a negative control for siRNA knock-down, a non-specific "scrambled" (randomly arranged) double-stranded siRNA was prepared comprising the sense siRNA sequence set forth in SEQ ID NO: 7 i.e., GUUGGCCAUUCUACUUCGCTT and corresponding antisense siRNA sequence.

### 1.2.2 Transfection of cells

Double-stranded siRNAs were transfected into cells according to the manufacturer's protocol (Lipofectamine2000 reagent, Invitrogen). After 24 h post transfection cells were lysed and the protein isolated using Mammalian Cell Lysis Kit (Sigma-Aldrich) and quantified. To determine transfection efficiency, BlockIT^{®} fluorescein isothiocyanate (FITC)-labelled scrambled siRNA was utilised (Invitrogen). Photos were taken on an Olympus IX71 fluorescent microscope and analysed using DP Controller software. After 24 h transfected cells were used for experiments.

### 1.2.3 Delivery in vivo

Expression plasmid DNA(s) encoding double-stranded siRNA(s) or shRNA(s) are rendered endotoxin-free using commercially available reagents. Purified plasmid DNA is then suspended in a commercially available cationic-liposomal *in vivo* delivery reagent at 1 µg/µL. For delivery to the hindlimb of an animal model of ischemia e.g., a murine hindlimb model of ischemia performed in streptozotocin-induced diabetic C57B/6J mice, the liposomes are generally injected into the adductor muscle e.g., at 4 sites (12.5 µL per site) on one or more occasions following unilateral femoral ligation e.g., on day 0 and day 3 and day 7 following unilateral femoral ligation. Animals are sacrificed at each time interval and at day 10 post-femoral ligation. Prior to intramuscular injection on days 0, 3 and 7, and prior to sacrifice on day 10, animals are anesthetized and blood flow to the hindlimb assessed by laser Doppler imaging. At sacrifice, muscle tissue is harvested for histological and molecular assessment of neovascularization. See also a more detailed description below e.g., Sections 1.9 and 1.10.

### 1.3 RNA Isolation from cells and amplification

Total RNA was isolated from isolated cells, e.g., HUVECs and HCAECs, using TRI Reagent (Sigma-Aldrich), and real-time quantitative polymerase chain reaction (qPCR) performed. In brief, cDNA was synthesised using iScript cDNA Synthesis Reagent (Biorad) then real-time qPCR performed using gene specific oligonucleotides previously described for human *TRX* and *TXNIP* and iScript Sybr Green I Mix (Biorad) on an i5 ThermoCycler (Biorad). The reaction conditions were 95°C for 3 min, followed by 40 cycles of 95°C for 30 sec, 60°C for 30 sec and 72°C for 30 sec. All samples were in triplicate and reactions with no template and/or enzyme were used as controls. Both *B-actin* and the *18S* subunit were used as internal controls, with RNA quantity expressed relative to the corresponding *B-actin* control. Fold induction over control was determined by normalising treated samples to the control.

### 1.4 Protein Isolation and Western Blot Analysis

Protein was isolated from cells using the Mammalian Cell Lysis Kit (Sigma-Aldrich) and Western blot analysis performed using established techniques (Invitrogen). Membranes were probed for both human proteins using antibodies against VDUP1/TXNIP and HIF1a (Invitrogen), TRX1, and VEGFR2/KDR (Abcam), and *B-*actin (Sigma-Aldrich) as per the manufacturer's protocol. A rabbit anti-VDUP1/TXNIP antibody specific for TXNIP (Zymed Laboratories) and a rabbit anti-TRX1 antibody specific for TRX1 (Sapphire Bioscience) were employed. Detection was carried out with horse radish peroxidase (HRP)-goat anti-rabbit IgG conjugate (Zymed).

### 1.5 In Vitro Angiogenic Assays

### 1.5.1 Cell Proliferation assay.

Cell proliferation was determined by EdU incorporation using the Click-iT™ EdU Flow Cytometry Assay Kit according to the manufacturer's protocol (Invitrogen).

### 1.5.2 Cell Migration assay

Cell migration assays were performed in modified Boyden chambers in a 24 well plate 4 format (Costar) with a polyvinyl 8. µm membrane. In brief, 1 x 10 HUVECs were seeded in the upper well of the chamber in a 100 µL volume of serum free M199 media, 60Q µL of M199 media + 10% FBS was added to the lower chamber. After 18h, cells were removed from the upper chamber and the transwell washed twice in PBS. Next the transwell was incubated for 30 min in 600 µL of PBS containing 10 µg/mL Ulex-Lectin FITC conjugate (Sigma-Aldrich) at room temperature. The transwell was rinsed twice in PBS, the membrane removed and mounted in DAPI-containing aqueous VectaMount (Vector Laboratories). DAPI and FITC dual-positive cells were then visualized by fluorescent microscopy (Olympus), photomicrographs obtained (DP Controller) and the number of HUVEC cells that migrated to the lower transwell side of the membrane per field calculated.

### 1.5.3 Matrigel Tubulogenesis Assays

EC formation of vascular-like structures was assessed on growth factor-reduced Matrigel (Becton Dickinson) in a method described by Weis et al., Art. Thromb. Vasc. Biol. 22, 1817-1823 (2002). Approximately 30 µL of Growth Factor Reduced Matrigel (BD) was added to each well of a 96-well plate and allowed to coat for 1 h at 37°C under standard tissue culture incubator conditions. Next, 2 x 10³ cells were added to each well and tubule formation observed over a period of 18 h. Photomicrographs were taken on the Olympus microscope and the tubule area and formation assessed using ImageJ Software (NIH).

### 1.6 Thioredoxin Activity Assay

Thioredoxin activity was assayed as described in a previously published colorimetric protocol (Ng et al., Arterioscler Thromb Vasc Biol. 27, 106-112, 2007), where the level of TRX activity is assessed at 405 nm *via* the oxidation of the colorimetric substrate DTNB (5,5'-dithiobis(2-nitrobenzoic acid); Sigma-Aldrich). Thioredoxin activity was normalised against total protein concentration.

### 1.7 Annexin-V Staining for Apoptosis

To assess apoptosis and necrosis, cells were stained with Annexin V-FITC and propidium iodide according to the manufacturer's protocol (Sigma-Aldrich) in chamber slides (Nunc). As a positive control for apoptosis, staurosporine (100, µg/mL in DMSO) incubations were performed. Cells were counterstained with DAPI. Fluorescent photomicrographs were taken on the Olympus IX71 and the images merged and analysed using DP Controller software. Apoptosis and necrosis were expressed as a percentage of the total cell population.

### 1.8 Statistical Analysis:

Measurement of all angiogenic assays (cell proliferation, cell migration and tubulogenesis) revealed a Gaussian distribution. Hence, comparisons between groups were analysed by a two-sided t-test or ANOVA for experiments with more than two groups. For t-tests, Bonferroni post-tests were also computed. Statistical analyses were performed using GraphPad Prism 4 software.

### 1.9 Induction of Diabetes Mellitus and Unilateral Hindlimb Ischemia in Mice

All animal experiments were conducted in accordance with guidelines and approval from the Central Sydney Area Health Service Animal Welfare Committee. Diabetes mellitus was induced in 5-week-old male C57BL/6J mice by administration of streptozotocin according to standard procedures. Briefly, streptozotocin prepared fresh, in citrate buffer. Mice were fasted for 6 h and 165 µg streptozotocin per gram of body weight, or citrate buffer control, was administered by intraperitoneal injection. Glycemic status was assessed 7 days after administration of streptozotocin, by determining tail vein blood glucose level using an Accu-Chek Perfroma Blood Glucometer (Roche Diagnostics). Mice were deemed hyperglycemic and used for further experiments if blood glucose was consistently elevated at or above 15 mmol/L.

At 7 weeks following induction of hyperglycemia, animals were anesthetized by methoxyflurane inhalation, the superficial and deep femoral arteries were ligated using 7-0 silk sutures, and the superficial femoral artery was excised to the re-entry of collateral branches arising from the lateral circumflex artery, to induce ischemia. As a negative control, the same procedure was performed on the contralateral leg of each animal without ligation of femoral arteries i.e., the "sham" control.

### 1.10 Intramuscular Gene Delivery

Constructs encoding siRNAs, including pRNA-U6.1/neo and a scrambled control siRNA, were delivered separately to the adductor muscle (day 0) of each leg of test animals before wound approximation. Briefly, endotoxin-free plasmid DNA was suspended in 50µL TransIT IN VIVO® Gene Delivery Reagent to a final concentration of 1 µg/µL, according to the manufacturer's instructions. Using a 29-gauge needle, 4 x 12.5 µL aliquots were injected at different sites along the adductor muscle. On day 3 and day 7 post-surgery, the injections were repeated.

### 1.11 Laser Doppler imaging of blood flow

Laser Doppler analysis of blood flow was assessed on anesthetized mice prior to induction of unilateral hindlimb ischemia (i.e., pre-surgery) and post-surgery on day 0, and again at days 3 and 7 post-surgery and prior to booster injections with siRNA constructs, and again on day 10 prior to sacrifice.

Laser Doppler imaging was performed using the moorLDI2-IR near-infrared Laser Doppler Perfusion Imager (Moor Instruments Ltd, UK). Briefly, mice were placed on a heating pad at 37°C to minimize variations in temperature. After 2 recordings of laser Doppler colour images, the average perfusions for ischemic and non-ischemic limbs were calculated based on coloured histogram pixels within equal-area polygonal regions of interest. To minimize variations from ambient light/temperature, calculated perfusion was expressed as the ratio of ischemic to non-ischemic hindlimb perfusion.

### 1.12 Ischernic Indices

Foot movement for the hindlimb ischemic model was assessed on days 3, 7 and 10 post-surgery. Foot movement scores were based on the following criteria in response to tail traction: score 0 = normal response, 1 = plantar but no toe flexion, 2 = no plantar or toe flexion, or score 3 = dragging of the limb. Tissue damage was also scored similar to on days 3, 7 and 10 using the following criteria: 0 = no tissue damage, 1= skin discolouration, 2 = loss of 1 to 2 toes, 3 = loss of foot or more.

### 1.13 Determination of Capillary Density

At sacrifice, a distal portion of the adductor muscle was removed and paraffin-sectioned for immunohistochemical analysis of von Willebrand factor ("vWF") staining for capillary density. In brief, slides were de-paraffinized, blocked and endogenous peroxidases removed. Slides were then incubated in 1:500 (v/v) dilution of anti-vWF antibody (Dako North America, Inc., Carpinteria, CA 93013, USA) for 1 h at room temperature. Slides were then treated to remove background according to standard procedures, counterstained using H&E, and cover-slipped using DPX mountant. Photographic representations were produced (15 images per section, 3 sections per adductor muscle) using an Olympus microscope. Positively-stained capillaries and myocytes were enumerated by a blinded observer, and the ratio of capillaries to myocytes per 200x field calculated. Data were expressed as a percentage of ischemic to non-ischemic hindlimb.

### 1.14 Isolation and analysis of skeletal muscle mRNA

RNA was isolated from skeletal muscle tissue using TRI Reagent (Sigma-Aldrich). Total RNA was purified using the RNeasy MinElute Cleanup Kit (Qiagen; Valencia, CA), qualitatively assessed by Experion (Bio-Rad; Hercules, CA), and quantified spectrophotometrically. Using commercially available kits from Bio-Rad, RNA was reverse-transcribed into cDNA and real-time qPCR performed by SYBR green assay on an iCycler 5.0 (BioRad) and gene specific-primers, essentially as described *supra*.

### 1.15 Isolation and analysis of skeletal muscle protein

Protein was isolated from mechanically homogenized skeletal muscle tissue using the Mammalian Cell Lysis Kit (Sigma-Aldrich), and quantified using Pierce BCA Protein Assay (Thermo-Fisher; Rockford, IL).

For Western blotting, protein lysates were separated on NuPAGE 4-12% (w/v) gels, and transferred to PVDF membranes using iBlot apparatus (Invitrogen). Membranes were blocked in 3% (w/v) BSA, and incubated with either rabbit anti-VDUP1/TXNIP antibody (Invitrogen), or rabbit anti-TRX1 antibody (Abcam; Cambridge, MA). Binding of primary antibody was detected using goat anti-rabbit HRP secondary antibody (Invitrogen). The level of β-actin in protein lysates was determined for normalization of signals, based on the accepted housekeeping role of the protein. Blots were imaged by chemiluminescence and densitometric analysis performed using Quantity One Software (BioRad).

Human VEGFA165 and murine VEGFA164 protein levels were assessed using a commercially available Human VEGF ELISA Kit (R&D Systems).

### EXAMPLE 2 - Hyperglycemia inhibits neovascularization and angiogenesis

The inventors have assessed the effects of glucose concentration on HCAECs and HUVECs vascular network formation in Matrigel assay, as shown in Figure 1 and Figure 2B. The results illustrate that glucose induces a concentration-dependent inhibition of HCAEC vascular network formation in Matrigel by 48±1.6% attenuation vs. control (at 15mM glucose, P<0.001; Figure 1). Similarly, the inventors also investigated the effects of glucose concentration on key angiogenic processes (proliferation, cell migration and tubulogenesis) on the impairment of endothelial cell function in HUVECs as illustrated in Table 2 below, Figure 2A, Figure 3A and Figure 3B. These results demonstrate that significant impairment of endothelial cell function with increased glucose concentration of both HUVECs and HCAECs cells.

**Table 2. Hyperglycemia impairs key angiogenic processes in HUVECs**

| | **Impairment of endothelial cell function (% 5mM control)** | | |
|---|---|---|---|
| **Glucose (mM)** | **10** | **15** | **20** |
| **Proliferation** | 19.2 ± 7.5 | 24.7 ± 5.2 | 39.7 ± 7.8 |
| **Migration** | 9.3 ± 4.5 | 41.0 ± 4.5 | 51.2 ± 2.5 |
| **Tubule formation** | 36.8 ± 2.7 | 40.1 ± 4.4 | 54.2 ± 2.3 |

Tubulogenesis is also an important process of neovascularization of endothelial cells as well as for neovascularization. Therefore the results presented here implicate a role for hyperglycemia in impairment or inhibition of key processes of neovascularization and angiogenesis. As illustrated in Figure 3A and Figure 3B, hyperglycemia significantly impairs: (A) HUVEC proliferation by ∼40% as determined by Tryptan blue staining and cell counts, (B) HUVEC migration by ∼68% in a modified Boyden chamber, (C) HUVEC tubulogenesis by ∼54% in a growth factor-reduced Matrigel assay.

Endothelial progenitor cells (EPCs) are a heterogeneous population which maintain/repair the endothelium and participate in angiogenesis. While there is debate about the nature of various EPC sub-populations, we recently characterized late endothelial outgrowth endothelial cells and demonstrated that OECs directly participate in angiogenesis. We hypothesise that OECs from healthy Type 1 diabetes mellitus patients have impaired angiogenic function contributing to the pathogenesis of diabetic vascular complications.

To test for this, young males (age <35 years) with Type 1 diabetes mellitus were recruited and had clinical, biochemical and haematological data collected. Age and sex-matched controls were also recruited. Peripheral blood mononuclear cells (PBMCs) were isolated and cultured on fibronectin plates. Sub-populations of EPCs were isolated on days 4 (early EPCs) and 14 to 21 (OECs) and lineage confirmed by FACs and AcLDL uptake with positive Ulex-Lectin-FITC staining.

The results herein illustrate that young type 1 diabetics on insulin replacement therapy, but otherwise healthy, show marked differences in their OEC sub-population compared to non-diabetic controls. Isolated diabetic OECs do not proliferate under standard EPC culture conditions, a factor which prevented an assessment of their migration and tubule formation. Moreover, diabetic OECs display profound morphological differences as demonstrated in the Figure 4.

The results herein demonstrating that Young Type 1 diabetes mellitus patients display profound morphological differences in their OEC sub-population, provide important implications for the pathogenesis of the vascular complications of diabetes mellitus.

The inventors also determined Endothelial Progenitor Cell (EPC) counts of peripheral whole blood of young Type 1 diabetes mellitus (DM) patients, and compared those counts with sex-matched and age-matched controls (Figure 5). EPCs were isolated from whole peripheral blood arid cultured on fibronectin-coated plates for 7 days via standard techniques. On day 7 cells were washed in PBS then incubated in serum-free media containing Dil-Acetylated Low Density Lipoprotein for 4 h at 37 °C. Next, cells were washed then incubated for 30 min in Ulex Europaeus-Lectin FITC conjugate. At least 30 fluorescent photomicrographs were taken per well using a 200x objective. Dual positive cells were enumerated. Figure 5 illustrates that EPC numbers are markedly reduced in peripheral whole blood of young Type 1 diabetes mellitus patients.

Data presented in Figure 20a and Figure 20b show that VEGF mRNA and protein, respectively, are reduced in hyperglycemic HUVECs, supporting the conclusion that angiogenic pathways are inhibited under hyperglycemic conditions.

Data provided in Figure 21 further support this conclusion, by showing that hyperglycemia attenuates VEGF-induced HUVEC migration. HUVECs were grown media lacking VEGF or comprising 2ng/ml VEGF or 10ng/ml VEGF and 5mM glucose or 15mM glucose or 25 mM glucose for 24 h, and confluent HUVEC monolayers were scratched, the areas of the scratches quantified using Image J software, cells were grown for a further 24 h in media comprising the same VEGF and glucose concentrations as before, further photomicrographs were obtained, and the migration of cells into the scratched areas quantitated using Image J software to analyze photomicrographs. The data confirm that VEGF induces HUVEC migration at all glucose concentrations tested and that hyperglycemia impairs migration of HUVECs, and demonstrate further that hyperglycemia attenuates the ability of VEGF to induce HUVEC migration. Higher VEGF concentrations are required to induce migration of hyperglycemic HUVECs than cells grown at low concentrations of glucose, demonstrating that VEGF may partially overcome impaired cell migration as a consequence of hyperglycemia.

### EXAMPLE 3 - Hyperglycemia inhibits thioredoxin activity

The inventors have investigated the effects of hyperglycemic culture conditions on thioredoxin activity in vitro, as determined by insulin disulphide reduction assay. A noticeable and significant glucose mediated reduction in TRX activity was also observed in HCAECs as shown in Figure 7.

These results illustrate that hyperglycemia not only inhibits neovascularization and angiogenesis but it also results in concomitant decrease in thioredoxin activity.

### EXAMPLE 4 - Hyperglycemia induces TXNIP expression.

Western blot analysis of protein expression in HUVEC and HCAEC cells incubated in media containing 5mM to 25 mM D glucose for a period of 24 h indicates glucose concentration dependant increase in expression of TXNIP protein. In contrast, thioredoxin protein expression has remained unchanged in different glucose conditions (Figure 8). The approximately 4 fold increase in TXNIP protein expression observed at 15 mM to 20 mM glucose (P<0.0001) (Figure 8) in HCAEC cells is associated with matching concentration -dependent up-regulation of TXNIP mRNA of approximately 2 fold at 15 mM glucose, as demonstrated in Figure 9.

The hyperglycemic induction of TXNIP mRNA and protein expression was also clearly demonstrated in HUVECs, as illustrated in details in Figures 9 and 10. Approximately 2.5-fold increase in TXNIP mRNA expression was observed at 25 mM glucose (Figures 9 and 10) with an associated 2-fold increase in TXNIP protein expression at 25mM glucose (Figure 10). The data illustrates that hyperglycemia induces TXNIP mRNA and protein expression in human endothelial cells.

### EXAMPLE 5 - Hyperglycemia increases TXNIP binding to thioredoxin.

Figure 11 shows the Western blot results of a immunoprecipitation assay of TXNIP from HCAECs cultured in media containing either 5 or 25 mM D-glucose for a period of 24 h. The immunoprecipitation assay was carried out according to standard methods known in the art, using pull-down antibody directed to TXNIP and a western blot antibody directed to thioredoxin. After incubation cells were lysed and the protein isolated and quantified. Rabbit anti-VDUP1/TXNIP antibody was incubated with Protein G Sepharose beads then protein lysates added for a 2 h incubation at 4°C. Beads were then washed and the supernatant used for Western blot analysis using a rabbit-anti-TRX1 antibody specific for TRX1. Immunoprecipitation revealed enhanced interaction between TRX/TXNIP by hyperglycemia, consistent with TRX repression by TXNIP.

### EXAMPLE 6 - Silencing TXNIP expression abrogates the deleterious effects of hyperglycemia.

Post transcriptional gene silencing of TRX1 and TXNIP using siRNA in HUVECs cells has been carried out with the results of the Western blot analysis provided in Figure 12. Densitometric analysis using QuantityOne Software indicate that TRX1 siRNA inhibited >90% TRX1 protein expression and >70% repression of TXNIP protein expression. Similar results were also obtained for post transcriptional gene silencing of TRX1 and TXNIP using siRNA in HCAECs (Figure 14), showing that siRNA knock down inhibits TRX-1 activity.

The inventors have studied the effects of TRX/TXNIP modulation on vascular network formation in HCAECs using Matrigel assay. Gene knockdown of TRX1 by siRNA inhibited HCAEC tubulogenesis by 47±5.3% (P<0.01; Figure 13a and Figure 13b). Conversely, knockdown of TXNIP strongly stimulated angiogenesis by 58.9±11.1% (P<0.01) (Figure 13a and Figure 13b). These results demonstrate that Gene-silencing of TXNIP induces angiogenesis in human coronary artery endothelial cells (HCAECs).

Figure 15 shows the glucose concentration dependent HUVEC cell proliferation following TXNIP siRNA gene silencing as a percentage of scrambled controls. TXNIP siRNA significantly improved HUVEC proliferation at both 15 and 25 mM glucose by 114.8 ± 9.2% (p < 0.05) and 116.2 ± 2.9% (p < 0.01), respectively, when compared to scrambled control cells at equivalent glucose concentrations. These results demoristrate that inhibition of TXNIP expression via siRNA abrogates the deleterious effect of hyperglycemia on human umbilical vein endothelial cell (HUVEC) proliferation.

Figure 16 shows the glucose concentration dependent hyperglycemic-impaired HUVEC cell migration following TXNIP siRNA gene silencing, compared to migration of normoglycemic HUVECs transfected with non-specific scrambled ds siRNA control. TXNIP siRNA protected HUVECs from hyperglycemic impairment of cell migration at 20 mM glucose when compared to normoglycemic scrambled control with no significant differences detected. Moreover, TXNIP siRNA significantly improved cell migration under high glucose concentrations to ∼ 200% (P<0.0001) when compared to scrambled control cells also grown at 20 mM glucose. These results demonstrate that inhibition of TXNIP expression via siRNA abrogates the deleterious effect of hyperglycemia on HUVEC migration.

Figure 17 shows TXNIP siRNA rescues tubulogenesis of hyperglycemic-impaired HUVECs. TXNIP siRNA protected HUVECs from hyperglycemic impairment of tubulogenesis at concentrations up to 20 mM glucose, maintaining tubulogenesis at 71.6% ± 10.1% when compared to normoglycemic scrambled control. When compared to scrambled control cells also grown in high glucose, TXNIP siRNA resulted in a significant increase in tubule formation to ∼280% (p<0.001). These results demonstrate that inhibition of TXNIP expression via siRNA abrogates the deleterious effect of hyperglycemia on HUVEC tubulogenesis in a Matrigel assay.

Figure 18 shows TXNIP siRNA rescue of hyperglycemia-induced HUVEC cell death. Hyperglycemia (25 mM) induced cell death by 33.0% in HUVECs. TXNIP siRNA protected HUVECs from hyperglycemic cell death with levels of cell death reduced to 2.2%. The slight increase in apoptotic population at 25 mM concentrations may indicate that the transition from apoptosis to necrosis has been missed as the assay was performed after an incubation of 24 h. Therefore, inhibition of TXNIP expression via siRNA abrogates the deleterious effect of hyperglycemia on HUVECs viability.

Figure 19 shows that gene silencing of TXNIP promotes tubulogenesis at high glucose concentrations under hypoxic (1% O₂) conditions. Cells transfected with TXNIP siRNA and cultured in high glucose, hypoxic conditions showed an approximate 275% increase in tubulogenesis compared to control cells at the same glycemic and O₂ conditions. This experiment has been performed on three occasions with similar results achieved each time. The data suggests that TXNIP inhibition greatly improves tubulogenesis under hyperglycemic and ischemic conditions.

Thus, the data provided in Figures 13-19 hereof demonstrate that in normoglycemic conditions (5mM glucose), knockdown of TRX by siRNA inhibits tubulogenesis, however TXNIP silencing stimulates angiogenesis (53±5.3%, 159±11.1% vs. control; P<0.01). Moreover, TXNIP silencing reverses the inhibitory effects of hyperglycemia on proliferation, migration and tubulogenesis (107.7±4.8%, 65.7±11.9%, 83.9±7.0% respectively; P>0.1 vs. normoglycemic control). Also, TXNIP inhibition greatly improves tubulogenesis under hyperglycemic and ischemic conditions (high glucose concentrations under hypoxic (1% O₂) conditions).

Data provided in Figure 22 show that siRNA-mediated reduction in TXNIP expression also alleviates hyperglycemia-induced reduction in VEGF expression in HUVECs (e.g., as shown in Figure 20). HUVECs were cultured in 5mM, 15mM or 25 mM glucose for 24 h, then transfected with double-stranded siRNA comprising SEQ ID NO: 5 for specific silencing of human *TRX1* mRNA e.g., SEQ ID NO: 1, or double-stranded siRNA comprising SEQ ID NO: 6 for specific silencing of human *TXNIP* mRNA e.g., SEQ ID NO: 3, using Lipofectamine 2000^{®} (Invitrogen) according to the manufacturer's protocol. A non-specific 'scrambled' ds-siRNA (SCR) comprising SEQ ID NO: 7 was used as a negative control. At 24h post-transfection, cells were lysed and the lysates were subjected to an ELISA for detecting human VEGFA165 protein. The data confirm that hyperglycemia reduces VEGF protein in the presence of the negative control SCR siRNA, and demonstrate that siRNA targeting expression of TXNIP significantly alleviates the adverse effect of hyperglycemia on VEGF protein expression (p<0.01). These data support the conclusion that angiogenesis is enhanced by reducing TXNIP expression in hyperglycemic conditions.

In summary, the results demonstrate that in hyperglycemic conditions e.g., 20mM or 25mM glucose, reducing the expression of TXNIP, e.g., by siRNA-mediated silencing, alleviates the inhibition or impairment of angiogenesis as determined by proliferation, migration or tubulogenesis of cells, or by determining a marker of angiogenesis e.g., VEFG.

### EXAMPLE 7 - Reducing TXNIP expression alleviates vascular complications in an animal model of Type 2 diabetes and/or animals having induced vascular complications

### 7.1 Induction of diabetes

Male C57BL/6J mice (e.g., Jackson Laboratory, Bar Harbor, ME, USA) at about 4 weeks of age are fed a high-fat diet to induce Type 2 diabetes. In the high-fat diet, 45% of total calories are from fat, which induces obesity with increased insulin levels and insulin resistance. After about 12 weeks on the high-fat diet, glucose tolerance testing is performed in which mice are fasted overnight, provided water *ad libitum,* and given a glucose load of about 1 mg/kg body weight e.g., by intraperitoneal injection. Blood glucose concentrations are measured before glucose loading and at about 30 min, 60 min and 90 min after glucose loading e.g., using a OneTouch Ultra glucometer (LifeScan, Milpitas, CA). Glucose tolerance is determined e.g., as the area under a curve of blood glucose concentration. Mice maintained on high-fat diets meeting the criteria for diabetes are utilized.

Alternatively, Type-1 diabetes is induced in mice by streptozotocin injection. Streptozotocin is delivered to 5-week old male mice by intraperitoneal injection (165µg/g body wt, in 0.3ml 10mM citrate, pH 4.5) without anaesthesia. Hyperglycemia is confirmed by measuring blood glucose in tail vein blood samples, using an ACCucheck glucometer. Mice meeting the criteria for diabetes are utilized. The diabetic state is confirmed weekly until the animal is sacrificed at 8-9 weeks of age. Experiments show that the diabetic state is fully maintained from 5.5 weeks until sacrifice.

Age-matched wild-type C57BL/6 mice that have been administered citrate buffer lacking streptozotocin and/or fed normal chow diet are used as controls.

### 7.2 Induction of ischemia

Mice are anesthetized e.g., by intraperitoneal injection of ketamine (90 mg/kg) and xylazine (10 mg/kg) and undergo surgical induction of an accepted model of vascular complication of diabetes, in particular the induction of unilateral hindlimb ischemia with ligation and excision of the femoral artery from its origin just above the inguinal ligament to its bifurcation at the origin of the saphenous and popliteal arteries, using known methods. The inferior epigastric, lateral circumflex, and superficial epigastric artery branches are also isolated and ligated.

Alternatively, animals are anesthetized using methoxyflurane an incision is made into the skin overlying the middle portion of the hindlimb to expose the superficial femoral artery, and the distal end of the saphenous artery is ligated to expose the deep femoral artery. The proximal femoral and deep femoral arteries are then ligated and these arteries and their collateral side branches dissected free and excised.

A sham procedure (preparation of arteries without ligations) is also performed on the contralateral leg of each animal.

### 7.3 Administration of siRNAs

The adductor muscles of non-diabetic control animals and diabetic animals, optionally subjected to femoral artery ligation to induce ischemia, are injected at four separate sites with a total volume of 50 µL of plasmid or other vector e.g., TransIT-IN VIVO having nucleic acid encoding siRNA targeting TXNIP expression. In a control group of animals, the adductor muscle is injected at four separate sites during the operation with a total volume of 50 µL of plasmid or other vector e.g., TransIT-IN VIVO having nucleic acid encoding a scrambled sequence of the same siRNA targeting TXNIP expression, wherein the scrambled sequence does not reduce or inhibit TXNIP expression. The incisions are closed. Intramuscular injectionsa are repeated on day 3 and 7. Animals are monitored during a post-operative period for at least up to about 10 days.

For administration to non-diabetic or diabetic animals in which an ischemic event has not been induced surgically, the siRNAs are administered initially on the day that diabetes is induced e.g., using streptozotocin with boosters being provided 3 days and 7 days later. For ischemic hindlimbs, the siRNAs are administered initially on the day of surgery to ligate femoral arteries (if applicable) with boosters being provided on post-operative days e.g., 3 days post-operation and 7 days post-operation. Animals are anesthetized for each intramuscular injection into the adductor bundle.

The siRNAs are administered to control animals not having undergone surgery at the same time as for test animals.

In one example, animals are randomized in a blinded manner into the following treatment groups, or a sub-set thereof depending upon the experimental protocol employed:
- Group 1:: untreated non-diabetic control animals and maintained on normal chow diet;
- Group 2:: untreated streptozotocin-induced diabetic animals without ischemic hindlimbs and maintained on normal chow diet;
- Group 3:: untreated non-diabetic animals with ischemic hindlimbs and maintained on normal chow diet;
- Group 4:: untreated streptozotocin-induced diabetic animals with ischemic hindlimbs and maintained on normal chow diet;
- Group 5:: untreated high-fat diet-induced diabetic animals without ischemic hindlimbs;
- Group 6:: untreated high-fat diet-induced diabetic animals with ischemic hindlimbs;
- Group 7:: untreated streptozotocin-induced and high-fat diet-induced diabetic animals without ischemic hindlimbs;
- Group 8:: untreated streptozotocin-induced and high-fat diet-induced diabetic animals with ischemic hindlimbs, treated with siRNA comprising SEQ ID NO: 5 and targeting TRX1 expression;
- Group 9:: non-diabetic control animals and maintained on normal chow diet, treated with siRNA comprising SEQ ID NO: 5 and targeting TRX1 expression;
- Group 10:: streptozotocin-induced diabetic animals without ischemic hindlimbs and maintained on normal chow diet, treated with siRNA comprising SEQ ID NO: 5 and targeting TRX1 expression;
- Group 11:: non-diabetic animals with ischemic hindlimbs and maintained on normal chow diet, treated with siRNA comprising SEQ ID NO: 5 and targeting TRX1 expression;
- Group 12:: streptozotocin-induced diabetic animals with ischemic hindlimbs and maintained on normal chow diet, treated with siRNA comprising SEQ ID NO: 5 and targeting TRX1 expression;
- Group 13:: high-fat diet-induced diabetic animals without ischemic hindlimbs, treated with siRNA comprising SEQ ID NO: 5 and targeting TRX1 expression;
- Group 14:: high-fat diet-induced diabetic animals with ischemic hindlimbs, treated with siRNA comprising SEQ ID NO: 5 and targeting TRX1 expression;
- Group 15:: streptozotocin-induced and high-fat diet-induced diabetic animals without ischemic hindlimbs, treated with siRNA comprising SEQ ID NO: 5 and targeting TRX1 expression;
- Group 16:: streptozotocin-induced and high-fat diet-induced diabetic animals with ischemic hindlimbs, treated with siRNA comprising SEQ ID NO: 5 and targeting TRX1 expression;
- Group 17:: non-diabetic control animals and maintained on normal chow diet, treated with siRNA comprising SEQ ID NO: 6 and targeting TXNIP expression;
- Group 18:: streptozotocin-induced diabetic animals without ischemic hindlimbs and maintained on normal chow diet, treated with siRNA comprising SEQ ID NO: 6 and targeting TXNIP expression;
- Group 19:: non-diabetic animals with ischemic hindlimbs and maintained on normal chow diet, treated with siRNA comprising SEQ ID NO: 6 and targeting TXNIP expression;
- Group 20:: streptozotocin-induced diabetic animals with ischemic hindlimbs and maintained on normal chow diet, treated with siRNA comprising SEQ ID NO: 6 and targeting TXNIP expression;
- Group 21:: high-fat diet-induced diabetic animals without ischemic hindlimbs, treated with siRNA comprising SEQ ID NO: 6 and targeting TXNIP expression;
- Group 22:: high-fat diet-induced diabetic animals with ischemic hindlimbs, treated with siRNA comprising SEQ ID NO: 6 and targeting TXNIP expression;
- Group 23:: streptozotocin-induced and high-fat diet-induced diabetic animals without ischemic hindlimbs, treated with siRNA comprising SEQ ID NO: 6 and targeting TXNIP expression;
- Group 24:: streptozotocin-induced and high-fat diet-induced diabetic animals with ischemic hindlimbs, treated with siRNA comprising SEQ ID NO: 6 and targeting TXNIP expression;
- Group 25:: non-diabetic control animals and maintained on normal chow diet, treated with negative control siRNA comprising SEQ ID NO: 7 and not capable of targeting TRX1 or TXNIP expression;
- Group 26: streptozotocin-induced diabetic animals without ischemic hindlimbs and maintained on normal chow diet, treated with negative control siRNA comprising SEQ ID NO: 7 and not capable of targeting TRX1 or TXNIP expression;
- Group 27:: non-diabetic animals with ischemic hindlimbs and maintained on normal chow diet, treated with negative control siRNA comprising SEQ ID NO: 7 and not capable of targeting TRX1 or TXNIP expression;
- Group 28:: streptozotocin-induced diabetic animals with ischemic hindlimbs and maintained on normal chow diet, treated with negative control siRNA comprising SEQ ID NO: 7 and not capable of targeting TRX1 or TXNIP expression;
- Group 29:: high-fat diet-induced diabetic animals without ischemic hindlimbs, treated with negative control siRNA comprising SEQ ID NO: 7 and not capable of targeting TRX1 or TXNIP expression;
- Group 30:: high-fat diet-induced diabetic animals with ischemic hindlimbs, treated with negative control siRNA comprising SEQ ID NO: 7 and not capable of targeting TRX1 or TXNIP expression;
- Group 31:: streptozotocin-induced and high-fat diet-induced diabetic animals without ischemic hindlimbs, treated with negative control siRNA comprising SEQ ID NO: 7 and not capable of targeting TRX1 or TXNIP expression; and
- Group 32:: streptozotocin-induced and high-fat diet-induced diabetic animals with ischemic hindlimbs, treated with negative control siRNA comprising SEQ ID NO: 7 and not capable of targeting TRX1 or TXNIP expression.

### 7.4 Analyses of treatment groups

A small incision is made in the hindlimb and the adductor muscles from both legs are excised and divided into portions for immunological and histological analyses, and for molecular analyses (RNA, protein and biochemical studies).

For determining angiogenic indicators, animals undergo Laser Doppler blood flow imaging immediately after surgery and at timed intervals until the termination of the study. Blood flow to the muscle/hindlimb is assessed on anesthetized mice prior to, and immediately after introduction of hindlimb ischemia, on post-surgical days 3 and 7, and prior to sacrifice on days 10-14 using a Near-Infrared Laser Doppler imager. Animals are kept warm and at constant temperature on a heat-pad throughout measurements.

Alternatively, or in addition, capillary density is determined by staining for von Willebrand factor e.g., normalized for myocyte density as determined by hematoxylin staining of distal portions of adductor muscles.

Alternatively, or in addition, foot movement of ischemic hindlimbs is determined as indicative of vascular condition e.g., daily, or between days 3 and 5 post-surgery, wherein a score of zero indicates a normal foot movement in response to tail traction, a score of 1 indicates a plantar movement without toe flexion in response to tail traction, a score of 2 indicates absence of plantar movement and toe flexion in response to tail traction, and a score of 3 indicates dragging of the limb.

Alternatively, or in addition, the extent of necrosis in ischemic hindlimbs is recorded e.g., daily, or between days 3 and 5 post-surgery, wherein a score of zero indicates no tissue damage, a score of 1 indicates skin discolouration, a score of 2 indicates loss of one or two toes, and a score of 3 indicates loss of foot or more.

Alternatively, or in addition, the extent of necrosis in ischemic hindlimbs is recorded e.g., daily, or between days 3 and 5 post-surgery, wherein grade 0 indicates no necrosis in ischemic limb; grade I indicates necrosis limited to toes; grade II indicates necrosis extending to dorsum pedis; grade III indicates necrosis extending to crus; and grade IV indicates necrosis extending to thigh.

Alternatively, or in addition, blood is also drawn by cardiac puncture under anaesthesia at the time of animal sacrifice e.g., at about 10-14 days post-operation or when required. Mice are anesthetized by inhalation of methoxyflurane and approximately 1 mL blood is withdrawn e.g., via cardiac puncture. Biochemistry and cell culture assays are performed.

Alternatively, or in addition, the activity and/or level of one or more angiogenic marker proteins and/or one or more mRNAs encoding an angiogenic marker protein is(are) determined using ELISA, Western blotting or quantitative RT-PCR. For example, the activity and/or level of SPP and/or sphingosine kinase and/or VEGF and/or FGF and/or IGF and/or an angiopoietin and/or PD-EGF and/or TGF e.g., TGF-β1 and/or HIF1-α and/or nitric oxide synthase and/or MCP-1 and/or IL-8 and/or an ephrin and/or NAP-2 and/or ENA-78 and/or a fragment of tyrosyl-tRNA synthetase is determined. Alternatively, or in addition, the level of mRNA encoding SPP and/or sphingosine kinase and/or VEGF and/or FGF and/or IGF and/or an angiopoietin and/or PD-EGF and/or TGF e.g., TGF-β1 and/or HIF1-α and/or nitric oxide synthase and/or MCP-1 and/or IL-8 and/or an ephrin and/or NAP-2 and/or ENA-78 and/or a fragment of tyrosyl-tRNA synthetase is determined.

### 7.5 Results

In one particular example, diabetes mellitus was induced by administration of streptozotocin to male C57BL/6J mice and, two weeks following treatment, animals were sacrificed, their adductor muscles were removed, RNA was isolated, and real-time quantitative PCR was performed using oligonucleotide primers for amplifying both murine TXNIP-encoding mRNA transcripts (SEQ ID NOs: 415 and 416). Data presented in Figure 23 show that streptozotocin-induced diabetes mellitus enhances *TXNIP* mRNA expression in skeletal muscle *in vivo,* suggesting a biological role for TXNIP. in vascular complications and/or vascular disease associated with diabetes mellitus.

In another particular example, ischemia was induced in a standard model of hindlimb ischemia, by performing unilateral femoral artery ligation in 7-week old male C57BL/6J mice and, two weeks following treatment, animals were sacrificed, their adductor muscles were removed, RNA was isolated, and real-time quantitative PCR was performed using oligonucleotide primers for amplifying both murine TXNIP-encoding mRNA transcripts (SEQ ID NOs: 415 and 416). Data provided in Figure 24 show a significant increase (p<0.05) in TXNIP transcript levels due to ischemia, relative to β-actin transcript levels, suggesting a biological role for TXNIP in vascular complications and/or vascular disease associated with ischemia. Data provided in Figure 25 demonstrate that siRNA-mediated gene silencing of TXNIP expression alleviates this enhancement of TXNIP expression *in vivo.* In particular, plasmid encoding siRNA targeting murine TXNIP. expression (SEQ ID NO: 417) or encoding a negative scrambled control siRNA (Scr) was administered intramuscularly to adductor muscle of like-treated animals either on the same day as femoral artery ligation was performed (day 0), or 3 days or 7 days post-surgery, animals were sacrificed at day 10 post-surgery, their adductor muscles were removed, RNA was isolated, and real-time quantitative PCR was performed using oligonucleotide primers for amplifying both murine TXNIP-encoding mRNA transcripts (SEQ ID NOs: 415 and 416). Control non-diabetic animals had received empty liposomes i.e., without siRNA.

The beneficial effects of reducing TXNIP expression *in vivo* were exemplified by rendering animals diabetic and optionally inducing ischemia in hindlimbs and administering siRNAs to adductor muscles as described herein above, and then determining the angiogenic indicators of blood flow, capillary density, foot movement, tissue damage, Hifla expression and VEGFa164 expression, and also determining GLUT1 expression as an indicator of glucose intolerance.

With respect to physiological indicators of angiogenesis, the data provided in Figure 26 indicate that diabetes impairs blood flow in the streptozotocin-induction model and that ischemia impairs recovery, however the impaired blood flow following an ischemic event in diabetic animals is alleviated by siRNA-mediated gene silencing of TXNIP expression. Data provided in Figure 27 indicate that siRNA-mediated gene silencing of TXNIP expression alleviates a reduction in capillary density induced by ischemia in both diabetic and non-diabetic animals *in vivo.* Data provided in Figure 28 indicate that siRNA-mediated gene silencing of TXNIP expression alleviates a reduction in physical consequences of impaired blood flow induced by ischemia in both diabetic and non-diabetic animals *in vivo,* by showing that ischemia impairs foot movement in both diabetic and non-diabetic animals, and that recovery from ischemia as determined by improved foot movement in both diabetic and non-diabetic animals is enhanced by siRNA-mediated gene silencing of TXNIP expression. Data provided in Figure 29 indicate that siRNA-mediated gene silencing of TXNIP expression prevents or otherwise alleviates ischemic tissue damage in both diabetic and non-diabetic animals *in vivo,* wherein recovery from mild tissue damage consequential to an ischemic event in both diabetic and non-diabetic animals is enhanced or promoted by siRNA-mediated gene silencing of TXNIP expression, as determined by improved tissue coloration.

With respect of biochemical markers of angiogenesis, the data provided in Figure 30 indicate that siRNA-mediated gene silencing of TXNIP expression enhances *Hif1-α* mRNA expression following an ischemic event in non-diabetic and diabetic animals. In particular, *Hif1-α* mRNA increases in both diabetic and non-diabetic animals exposed to an ischemic event and treated with siRNA targeting TXNIP expression, indicating enhanced angiogenesis following treatment. The data presented in Figure 31 indicate that siRNA-mediated gene silencing of TXNIP expression enhances *YEGFa164* mRNA expression following an ischemic event in non-diabetic and diabetic animals. In particular, *YEGFa164* mRNA increases in both diabetic and non-diabetic animals exposed to an ischemic event and treated with siRNA targeting TXNIP expression, indicating enhanced angiogenesis following treatment.

With respect to indicators of glucose intolerance, the data presented in Figure 32 indicate that siRNA-mediated gene silencing of TXNIP expression leads to reduced *GLUT1* mRNA expression following an ischemic event in non-diabetic and diabetic animals. In particular, those data show that *GLUT1* mRNA increases in diabetic animals compared to non-diabetic animals, and that this enhanced expression is alleviated following treatment with siRNA targeting TXNIP expression, suggesting that glucose intolerance is reduced.

## Claims

1. A composition for use in preventing or alleviating one or more vascular complication(s) in a subject suffering from diabetes and/or impaired glucose tolerance and/or hyperglycemia, wherein the composition comprises siRNA that specifically targets a thioredoxin-interacting protein (TXNIP)-encoding nucleic acid and reduces expression of TXNIP.

2. The composition for use according to claim 1, wherein the siRNA comprises a nucleic acid sequence set forth in SEQ ID NO: 6 or any one of SEQ ID NOs: 8 to 403 set forth in Table 1 or a nucleotide sequence complementary to any one of SEQ ID NOs: 6 or 8 to 403.

3. The composition for use according to claim 2, wherein the siRNA comprises the nucleic acid sequence set forth in SEQ ID NO: 6.

4. The composition for use according to claim 2, wherein the siRNA comprises a nucleic acid sequence set forth in any one of SEQ ID NOs: 57 to 59, 117-121, 175 to 177 and 235 to 239.

5. The composition for use according to any one of claims 1 to 4, wherein the composition enhances the ability of a cell to develop or proliferate into a functional endothelial cell (EC) or contribute to the formation of functional endothelium or blood vessels or otherwise promote vascular repair or enhance vascular repair.

6. The composition for use according to claim 5, wherein the cell is selected from a stem cell, an endothelial progenitor cell and an outgrowth endothelial cell.

7. The composition for use according to any one of claims 1 to 6, wherein the diabetes is Type 1 diabetes mellitus or Type 2 diabetes mellitus.

8. The composition for use according to any one of claims 1 to 7, wherein the one or more vascular complication(s) in the subject is(are) macrovascular complications such as one or more macrovascular complication(s) in the subject selected from extracranial vascular disease, intracranial vascular disease, ischemia, stroke, coronary heart disease and peripheral vascular disease or wherein one or more vascular complication(s) in the subject is(are) microvascular complications such as one or more microvascular complication(s) in the subject selected from neuropathy, retinopathy, nephropathy, impotence, and impaired or slow wound healing.

9. Use of an siRNA that specifically targets a thioredoxin-interacting protein (TXNIP)-encoding nucleic acid and reduces expression of TXNIP in the preparation of a medicament for the treatment of one or more vascular complications of diabetes mellitus and/or impaired glucose tolerance and/or hyperglycemia.

10. The use according to claim 9, wherein the siRNA comprises a nucleic acid sequence set forth in SEQ ID NO: 6 or any one of SEQ ID NOs: 8 to 403 set forth in Table 1 or a nucleotide sequence complementary to any one of SEQ ID NOs: 6 or 8 to 403.

11. The use according to claim 10, wherein the siRNA comprises the nucleic acid sequence set forth in SEQ ID NO: 6.

12. The use according to claim 10, wherein the siRNA comprises a nucleic acid sequence set forth in any one of SEQ ID NOs: 57 to 59, 117-121, 175 to 177 and 235 to 239.

## Patentansprüche

1. Zusammensetzung zur Verwendung bei der Vorbeugung oder Linderung einer oder mehrerer vaskulärer Komplikation(en) bei einem Individuum, das unter Diabetes und/oder gestörter Glucosetoleranz und/oder Hyperglykämie leidet, worin die Zusammensetzung siRNA umfasst, die auf eine für ein Thioredoxin-wechselwirkendes Protein (TXNIP) kodierende Nucleinsäure spezifisch abzielt und die Expression von TXNIP reduziert.

2. Zusammensetzung zur Verwendung nach Anspruch 1, worin die siRNA eine Nucleinsäuresequenz, die in Seq.-ID Nr. 6 oder einer der in Tabelle 1 angeführten Seq.-ID Nr. 8 bis 403 angeführt ist, oder eine Nucleotidsequenz umfasst, die komplementär zu einer der Seq.-ID Nr. 6 oder 8 bis 403 ist.

3. Zusammensetzung zur Verwendung nach Anspruch 2, worin die siRNA die in Seq.-ID Nr. 6 angeführte Nucleinsäuresequenz umfasst.

4. Zusammensetzung zur Verwendung nach Anspruch 2, worin die siRNA eine in einer der Seq.-ID Nr. 57 bis 59, 117-121, 175 bis 177 und 235 bis 239 angeführte Nucleinsäuresequenz umfasst.

5. Zusammensetzung zur Verwendung nach einem der Ansprüche 1 bis 4, worin die Zusammensetzung die Fähigkeit einer Zelle verstärkt, sich in eine funktionale Endothelzelle (EC) zu entwickeln oder zu proliferieren oder zur Bildung eines funktionalen Endothels oder von Blutgefäßen beizutragen oder anderweitig die vaskuläre Reparatur zu fördern oder die vaskuläre Reparatur zu verstärken.

6. Zusammensetzung zur Verwendung nach Anspruch 5, worin die Zelle aus einer Stammzelle, einer Endothelvorläuferzelle und einer Auswuchsendothelzelle ausgewählt ist.

7. Zusammensetzung zur Verwendung nach einem der Ansprüche 1 bis 6, worin der Diabetes Diabetes mellitus Typ 1 oder Diabetes mellitus Typ 2 ist.

8. Zusammensetzung zur Verwendung nach einem der Ansprüche 1 bis 7, worin die eine oder mehreren vaskuläre(n) Komplikation(en) beim Individuum makrovaskuläre Komplikation(en) ist/sind, wie etwa eine oder mehrere makrovaskuläre Komplikation(en) beim Individuum, ausgewählt aus extrakranieller Gefäßkrankheit, intrakranieller Gefäßkrankheit, Ischämie, Schlaganfall, koronarer Herzkrankheit und peripherer Gefäßkrankheit, oder worin eine oder mehrere vaskuläre Komplikation(en) beim Individuum mikrovaskuläre Komplikation(en) ist/sind, wie etwa eine oder mehrere mikrovaskuläre Komplikation(en) beim Individuum, ausgewählt aus Neuropathie, Retinopathie, Nephropathie, Impotenz und gestörter oder langsamer Wundheilung.

9. Verwendung einer siRNA, die auf eine für ein Thioredoxin-wechselwirkendes Protein (TXNIP) kodierende Nucleinsäure spezifisch abzielt und die Expression von TXNIP reduziert, zur Herstellung eines Medikaments zur Behandlung einer oder mehrerer vaskulärer Komplikationen von Diabetes mellitus und/oder gestörter Glucosetoleranz und/oder Hyperglykämie.

10. Verwendung nach Anspruch 9, worin die siRNA eine Nucleinsäuresequenz, die in Seq.-ID Nr. 6 oder einer der in Tabelle 1 angeführten Seq.-ID Nr. 8 bis 403 angeführt ist, oder eine Nucleotidsequenz umfasst, die komplementär zu einer der Seq.-ID Nr. 6 oder 8 bis 403 ist.

11. Verwendung nach Anspruch 10, worin die siRNA die in Seq.-ID Nr. 6 angeführte Nucleinsäuresequenz umfasst.

12. Verwendung nach Anspruch 10, worin die siRNA eine Nucleinsäuresequenz umfasst, die in einer der Seq.-ID Nr. 57 bis 59, 117-121, 175 bis 177 und 235 bis 239 angeführt ist.

## Revendications

1. Composition destinée à être utilisée dans la prévention ou l'atténuation d'une ou de plusieurs complication(s) vasculaire(s) chez un sujet souffrant de diabète et/ou d'intolérance au glucose et/ou d'hyperglycémie, où la composition comprend un ARNsi qui cible spécifiquement un acide nucléique codant pour la protéine d'interaction avec la thiorédoxine (TXNIP) et réduit l'expression de la TXNIP.

2. Composition destinée à être utilisée selon la revendication 1, dans laquelle l'ARNsi comprend une séquence d'acide nucléique décrite dans SEQ ID NO: 6 ou l'une quelconque des SEQ ID NO: 8 à 403 décrites dans le Tableau 1 ou une séquence de nucléotides complémentaire à l'une quelconque de SEQ ID NO: 6 ou 8 à 403.

3. Composition destinée à être utilisée selon la revendication 2, dans laquelle l'ARNsi comprend la séquence d'acide nucléique décrite dans SEQ ID NO: 6.

4. Composition destinée à être utilisée selon la revendication 2, dans laquelle l'ARNsi comprend une séquence d'acide nucléique décrite dans l'une quelconque de SEQ ID NO: 57 à 59, 117-121, 175 à 177 et 235 à 239.

5. Composition destinée à être utilisée selon l'une quelconque des revendications 1 à 4, où la composition améliore l'aptitude d'une cellule à se développer ou à proliférer en une cellule endothéliale (CE) fonctionnelle ou à contribuer à la formation d'un endothélium fonctionnel ou de vaisseaux sanguins ou à autrement promouvoir la réparation vasculaire ou améliorer la réparation vasculaire.

6. Composition destinée à être utilisée selon la revendication 5, dans laquelle la cellule est sélectionnée parmi une cellule souche, une cellule progénitrice endothéliale et une cellule endothéliale d'excroissance.

7. Composition destinée à être utilisée selon l'une quelconque des revendications 1 à 6, où le diabète est le diabète de type 1 ou le diabète de type 2.

8. Composition destinée à être utilisée selon l'une quelconque des revendications 1 à 7, dans laquelle la une ou plusieurs complication(s) vasculaire(s) chez le sujet est(sont) des complications macrovasculaires comme une ou plusieurs complication(s) macrovasculaire(s) chez le sujet sélectionnée(s) parmi une maladie vasculaire extra-crânienne, une maladie vasculaire intracrânienne, une ischémie, un accident vasculaire cérébral, une coronaropathie et une maladie vasculaire périphérique, ou dans laquelle une ou plusieurs complication(s) vasculaire(s) chez le sujet est(sont) des complications microvasculaires comme une ou plusieurs complication(s) microvasculaire(s) chez le sujet sélectionnée(s) parmi une neuropathie, une rétinopathie, une néphropathie, l'impuissance, et une cicatrisation lente ou altérée.

9. Utilisation d'un ARNsi qui cible spécifiquement un acide nucléique codant pour la protéine d'interaction avec la thiorédoxine (TXNIP) et réduit l'expression de la TXNIP dans la préparation d'un médicament destiné au traitement d'une ou de plusieurs complications vasculaires du diabète et/ou de l'intolérance au glucose et/ou de l'hyperglycémie.

10. Utilisation selon la revendication 9, dans laquelle l'ARNsi comprend une séquence d'acide nucléique décrite dans SEQ ID N0: 6 ou l'une quelconque des SEQ ID NO: 8 à 403 décrites dans le Tableau 1 ou une séquence de nucléotides complémentaire à l'une quelconque de SEQ ID NO: 6 ou 8 à 403.

11. Utilisation selon la revendication 10, dans laquelle l'ARNsi comprend la séquence d'acide nucléique décrite dans SEQ ID NO: 6.

12. Utilisation selon la revendication 10, dans laquelle l'ARNsi comprend une séquence d'acide nucléique décrite dans l'une quelconque de SEQ ID NO: 57 à 59, 117-121, 175 à 177 et 235 à 239.


### SEQUENCE LISTING

<110> The Heart Research Institute Ltd
<120> Method of treatment of vascular complications
<130> SPH/FP6837934
<140>
   <141> 2009-06-29
<150> 09768642.2
   <151> 2009-06-29
<150> PCT/AU2009/000840
   <151> 2009-06-29
<150> US61/076,550
   <151> 2008-06-27
<150> US61/077,261
   <151> 2008-07-01
<160> 417
<170> PatentIn version 3.4
<210> 1
   <211> 508
   <212> RNA
   <213> Homo sapiens
<220>
   <221> CDS
   <222> (64)..(378)
<400> 1
   uuuggugcuu uggauccauu uccaucgguc cuuacagccg cucgucagac uccagcagcc      60
   aag aug gug aag cag auc gag agc aag acu gcu uuu cag gaa gcc uug       108
       Met Val Lys Gln Ile Glu Ser Lys Thr Ala Phe Gln Glu Ala Leu
       1               5                   10                  15
   gac gcu gca ggu gau aaa cuu gua gua guu gac uuc uca gcc acg ugg       156
   Asp Ala Ala Gly Asp Lys Leu Val Val Val Asp Phe Ser Ala Thr Trp
                   20                  25                  30
   ugu ggg ccu ugc aaa aug auc aag ccu uuc uuu cau ucc cuc ucu gaa       204
   Cys Gly Pro Cys Lys Met Ile Lys Pro Phe Phe His Ser Leu Ser Glu
               35                  40                  45
   aag uau ucc aac gug aua uuc cuu gaa gua gau gug gau gac ugu cag       252
   Lys Tyr Ser Asn Val Ile Phe Leu Glu Val Asp Val Asp Asp Cys Gln
           50                  55                  60
   gau guu gcu uca gag ugu gaa guc aaa ugc aug cca aca uuc cag uuu       300
   Asp Val Ala Ser Glu Cys Glu Val Lys Cys Met Pro Thr Phe Gln Phe
       65                  70                  75
   uuu aag aag gga caa aag gug ggu gaa uuu ucu gga gcc aau aag gaa       348
   Phe Lys Lys Gly Gln Lys Val Gly Glu Phe Ser Gly Ala Asn Lys Glu
   80                  85                  90                  95
   aag cuu gaa gcc acc auu aau gaa uua guc uaaucauguu uucugaaaau         398
   Lys Leu Glu Ala Thr Ile Asn Glu Leu Val
                   100                 105
   auaaccagcc auuggcuauu uaaaacuugu aauuuuuuua auuuacaaaa auauaaaaua    458
   ugaagacaua aacccaguug ccaucugcgu gacaauaaaa cauuaaugcu              508
<210> 2
   <211> 105
   <212> PRT
   <213> Homo sapiens
<400> 2
   Met Val Lys Gln Ile Glu Ser Lys Thr Ala Phe Gln Glu Ala Leu Asp
   1               5                   10                  15
   Ala Ala Gly Asp Lys Leu Val Val Val Asp Phe Ser Ala Thr Trp Cys
               20                  25                  30
   Gly Pro Cys Lys Met Ile Lys Pro Phe Phe His Ser Leu Ser Glu Lys
           35                  40                  45
   Tyr Ser Asn Val Ile Phe Leu Glu Val Asp Val Asp Asp Cys Gln Asp
       50                  55                  60
   Val Ala Ser Glu Cys Glu Val Lys Cys Met Pro Thr Phe Gln Phe Phe
   65                  70                  75                  80
   Lys Lys Gly Gln Lys Val Gly Glu Phe Ser Gly Ala Asn Lys Glu Lys
                   85                  90                  95
   Leu Glu Ala Thr Ile Asn Glu Leu Val
               100                 105
<210> 3
   <211> 2953
   <212> RNA
   <213> Homo sapiens
<220>
   <221> CDS
   <222> (342)..(1514)
<400> 3
   auauagagac guuuccgccu ccugcuugaa acuaaccccu cuuuuucucc aaaggagugc     60
   uuguggagau cggaucuuuu cuccagcaau ugggggaaag aaggcuuuuu cucugaauuc    120
   gcuuagugua accagcggcg uauauuuuuu aggcgccuuu ucgaaaaccu aguaguuaau    180
   auucauuugu uuaaaucuua uuuuauuuuu aagcucaaac ugcuuaagaa uaccuuaauu    240
   ccuuaaagug aaauaauuuu uugcaaaggg guuuccucga uuuggagcuu uuuuuuucuu    300
   ccaccgucau uucuaacucu uaaaaccaac ucaguuccau c aug gug aug uuc aag    356
                                                   Met Val Met Phe Lys
                                                   1               5
   aag auc aag ucu uuu gag gug guc uuu aac gac ccu gaa aag gug uac      404
   Lys Ile Lys Ser Phe Glu Val Val Phe Asn Asp Pro Glu Lys Val Tyr
                   10                  15                  20
   ggc agu ggc gag aag gug gcu ggc cgg gug aua gug gag gug ugu gaa      452
   Gly Ser Gly Glu Lys Val Ala Gly Arg Val Ile Val Glu Val Cys Glu
               25                  30                  35
   guu acu cgu guc aaa gcc guu agg auc cug gcu ugc gga gug gcu aaa      500
   Val Thr Arg Val Lys Ala Val Arg Ile Leu Ala Cys Gly Val Ala Lys
           40                  45                  50
   gug cuu ugg aug cag gga ucc cag cag ugc aaa cag acu ucg gag uac      548
   Val Leu Trp Met Gln Gly Ser Gln Gln Cys Lys Gln Thr Ser Glu Tyr
       55                  60                  65
   cug cgc uau gaa gac acg cuu cuu cug gaa gac cag cca aca ggu gag      596
   Leu Arg Tyr Glu Asp Thr Leu Leu Leu Glu Asp Gln Pro Thr Gly Glu
   70                  75                  80                  85
   aau gag aug gug auc aug aga ccu gga aac aaa uau gag uac aag uuc      644
   Asn Glu Met Val Ile Met Arg Pro Gly Asn Lys Tyr Glu Tyr Lys Phe
                   90                  95                  100
   ggc uuu gag cuu ccu cag ggg ccu cug gga aca ucc uuc aaa gga aaa      692
   Gly Phe Glu Leu Pro Gln Gly Pro Leu Gly Thr Ser Phe Lys Gly Lys
               105                 110                 115
   uau ggg ugu gua gac uac ugg gug aag gcu uuu cuu gac cgc ccg agc      740
   Tyr Gly Cys Val Asp Tyr Trp Val Lys Ala Phe Leu Asp Arg Pro Ser
           120                 125                 130
   cag cca acu caa gag aca aag aaa aac uuu gaa gua gug gau cug gug      788
   Gln Pro Thr Gln Glu Thr Lys Lys Asn Phe Glu Val Val Asp Leu Val
       135                 140                 145
   gau guc aau acc ccu gau uua aug gca ccu gug ucu gcu aaa aaa gaa      836
   Asp Val Asn Thr Pro Asp Leu Met Ala Pro Val Ser Ala Lys Lys Glu
   150                 155                 160                 165
   aag aaa guu ucc ugc aug uuc auu ccu gau ggg cgg gug ucu guc ucu      884
   Lys Lys Val Ser Cys Met Phe Ile Pro Asp Gly Arg Val Ser Val Ser
                   170                 175                 180
   gcu cga auu gac aga aaa gga uuc ugu gaa ggu gau gag auu ucc auc      932
   Ala Arg Ile Asp Arg Lys Gly Phe Cys Glu Gly Asp Glu Ile Ser Ile
               185                 190                 195
   cau gcu gac uuu gag aau aca ugu ucc cga auu gug guc ccc aaa gcu      980
   His Ala Asp Phe Glu Asn Thr Cys Ser Arg Ile Val Val Pro Lys Ala
           200                 205                 210
   gcc auu gug gcc cgc cac acu uac cuu gcc aau ggc cag acc aag gug     1028
   Ala Ile Val Ala Arg His Thr Tyr Leu Ala Asn Gly Gln Thr Lys Val
       215                 220                 225
   cug acu cag aag uug uca uca guc aga ggc aau cau auu auc uca ggg     1076
   Leu Thr Gln Lys Leu Ser Ser Val Arg Gly Asn His Ile Ile Ser Gly
   230                 235                 240                 245
   aca ugc gca uca ugg cgu ggc aag agc cuu cgg guu cag aag auc agg     1124
   Thr Cys Ala Ser Trp Arg Gly Lys Ser Leu Arg Val Gln Lys Ile Arg
               250                 255                 260
   ccu ucu auc cug ggc ugc aac auc cuu cga guu gaa uau ucc uua cug      1172
   Pro Ser Ile Leu Gly Cys Asn Ile Leu Arg Val Glu Tyr Ser Leu Leu
               265                 270                 275
   auc uau guu agc guu ccu gga ucc aag aag guc auc cuu gac cug ccc      1220
   Ile Tyr Val Ser Val Pro Gly Ser Lys Lys Val Ile Leu Asp Leu Pro
           280                 285                 290
   cug gua auu ggc agc aga uca ggu cua agc agc aga aca ucc agc aug      1268
   Leu Val Ile Gly Ser Arg Ser Gly Leu Ser Ser Arg Thr Ser Ser Met
       295                 300                 305
   gcc agc cga acc agc ucu gag aug agu ugg gua gau cug aac auc ccu      1316
   Ala Ser Arg Thr Ser Ser Glu Met Ser Trp Val Asp Leu Asn Ile Pro
   310                 315                 320                 325
   gau acc cca gaa gcu ccu ccc ugc uau aug gau guc auu ccu gaa gau      1364
   Asp Thr Pro Glu Ala Pro Pro Cys Tyr Met Asp Val Ile Pro Glu Asp
                   330                 335                 340
   cac cga uug gag agc cca acc acu ccu cug cua gau gac aug gau ggc      1412
   His Arg Leu Glu Ser Pro Thr Thr Pro Leu Leu Asp Asp Met Asp Gly
               345                 350                 355
   ucu caa gac agc ccu auc uuu aug uau gcc ccu gag uuc aag uuc aug      1460
   Ser Gln Asp Ser Pro Ile Phe Met Tyr Ala Pro Glu Phe Lys Phe Met
           360                 365                 370
   cca cca ccg acu uau acu gag gug gau ccc ugc auc cuc aac aac aau      1508
   Pro Pro Pro Thr Tyr Thr Glu Val Asp Pro Cys Ile Leu Asn Asn Asn
       375                 380                 385
   gug cag ugagcaugug gaagaaaaga agcagcuuua ccuacuuguu ucuuuuuguc      1564
   Val Gln
   390
   ucucuuccug gacacucacu uuuucagaga cucaacaguc ucugcaaugg aguguggguc      1624
   caccuuagcc ucugacuucc uaauguagga gguggucagc aggcaaucuc cugggccuua      1684
   aaggaugcgg acucauccuc agccagcgcc cauguuguga uacaggggug uuuguuggau      1744
   ggguuuaaaa auaacuagaa aaacucaggc ccauccauuu ucucagaucu ccuugaaaau      1804
   ugaggccuuu ucgauaguuu cgggucaggu aaaaauggcc uccuggcgua agcuuuucaa      1864
   gguuuuuugg aggcuuuuug uaaauuguga uaggaacuuu ggaccuugaa cuuauguauc      1924
   auguggagaa gagccaauuu aacaaacuag gaagaugaaa agggaaauug uggccaaaac      1984
   uuugggaaaa ggagguucuu aaaaucagug uuuccccuuu gugcacuugu agaaaaaaaa      2044
   gaaaaaccuu cuagagcuga uuugauggac aauggagaga gcuuucccug ugauuauaaa      2104
   aaaggaagcu agcugcucua cggucaucuu ugcuuaagag uauacuuuaa ccuggcuuuu      2164
   aaagcaguag uaacugcccc accaaagguc uuaaaagcca uuuuuggagc cuauugcacu      2224
   guguucuccu acugcaaaua uuuucauaug ggaggauggu uuucucuuca uguaaguccu      2284
   uggaauugau ucuaagguga uguucuuagc acuuuaauuc cugucaaauu uuuuguucuc      2344
   cccuucugcc aucuuaaaug uaagcugaaa cuggucuacu gugucucuag gguuaagcca      2404
   aaagacaaaa aaaauuuuac uacuuuugag auugccccaa uguacagaau uauauaauuc 2464
   uaacgcuuaa aucaugugaa aggguugcug cugucagccu ugcccacugu gacuucaaac 2524
   ccaaggagga acucuugauc aagaugccga acccuguguu cagaaccucc aaauacugcc 2584
   augagaaacu agagggcagg ucuucauaaa agcccuuuga acccccuucc ugcccugugu 2644
   uaggagauag ggauauuggc cccucacugc agcugccagc acuuggucag ucacucucag 2704
   ccauagcacu uuguucacug uccuguguca gagcacugag cuccacccuu uucugagagu 2764
   uauuacagcc agaaagugug ggcugaagau gguugguuuc auguuuuugu auuauguauc 2824
   uuuuuguaug guaaagacua uauuuuguac uuaaccagau auauuuuuac cccagauggg 2884
   gauauucuuu guaaaaaaug aaaauaaagu uuuuuuaaug gaaaaaaaaa aaaaaaaaaa 2944
   aaaaaaaaa 2953
<210> 4
   <211> 391
   <212> PRT
   <213> Homo sapiens
<400> 4
   Met Val Met Phe Lys Lys Ile Lys Ser Phe Glu Val Val Phe Asn Asp
   1 5 10 15
   Pro Glu Lys Val Tyr Gly Ser Gly Glu Lys Val Ala Gly Arg Val Ile
   20 25 30
   Val Glu Val Cys Glu Val Thr Arg Val Lys Ala Val Arg Ile Leu Ala
   35 40 45
   Cys Gly Val Ala Lys Val Leu Trp Met Gln Gly Ser Gln Gln Cys Lys
   50 55 60
   Gln Thr Ser Glu Tyr Leu Arg Tyr Glu Asp Thr Leu Leu Leu Glu Asp
   65 70 75 80
   Gln Pro Thr Gly Glu Asn Glu Met Val Ile Met Arg Pro Gly Asn Lys
   85 90 95
   Tyr Glu Tyr Lys Phe Gly Phe Glu Leu Pro Gln Gly Pro Leu Gly Thr
   100 105 110
   Ser Phe Lys Gly Lys Tyr Gly Cys Val Asp Tyr Trp Val Lys Ala Phe
   115 120 125
   Leu Asp Arg Pro Ser Gln Pro Thr Gln Glu Thr Lys Lys Asn Phe Glu
   130 135 140
   Val Val Asp Leu Val Asp Val Asn Thr Pro Asp Leu Met Ala Pro Val
   145                 150                 155                 160
   Ser Ala Lys Lys Glu Lys Lys Val Ser Cys Met Phe Ile Pro Asp Gly
                   165                 170                 175
   Arg Val Ser Val Ser Ala Arg Ile Asp Arg Lys Gly Phe Cys Glu Gly
               180                 185                 190
   Asp Glu Ile Ser Ile His Ala Asp Phe Glu Asn Thr Cys Ser Arg Ile
           195                 200                 205
   Val Val Pro Lys Ala Ala Ile Val Ala Arg His Thr Tyr Leu Ala Asn
       210                 215                 220
   Gly Gln Thr Lys Val Leu Thr Gln Lys Leu Ser Ser Val Arg Gly Asn
   225                 230                 235                 240
   His Ile Ile Ser Gly Thr Cys Ala Ser Trp Arg Gly Lys Ser Leu Arg
                   245                 250                 255
   Val Gln Lys Ile Arg Pro Ser Ile Leu Gly Cys Asn Ile Leu Arg Val
               260                 265                 270
   Glu Tyr Ser Leu Leu Ile Tyr Val Ser Val Pro Gly Ser Lys Lys Val
           275                 280                 285
   Ile Leu Asp Leu Pro Leu Val Ile Gly Ser Arg Ser Gly Leu Ser Ser
       290                 295                 300
   Arg Thr Ser Ser Met Ala Ser Arg Thr Ser Ser Glu Met Ser Trp Val
   305                 310                 315                 320
   Asp Leu Asn Ile Pro Asp Thr Pro Glu Ala Pro Pro Cys Tyr Met Asp
                   325                 330                 335
   Val Ile Pro Glu Asp His Arg Leu Glu Ser Pro Thr Thr Pro Leu Leu
               340                 345                 350
   Asp Asp Met Asp Gly Ser Gln Asp Ser Pro Ile Phe Met Tyr Ala Pro
           355                 360                 365
   Glu Phe Lys Phe Met Pro Pro Pro Thr Tyr Thr Glu Val Asp Pro Cys
       370                 375                 380
   Ile Leu Asn Asn Asn Val Gln
   385                 390
<210> 5
   <211> 19
   <212> RNA
   <213> artificial
<220>
   <223> sequence for preparation of siRNA or shRNA targeting TRX1 expression
<400> 5
   gcagaucgag agcaagacu 19
<210> 6
   <211> 19
   <212> RNA
   <213> artificial
<220>
   <223> sequence for preparation of siRNA or shRNA targeting TXNIP expression
<400> 6
   acagacuucg gaguaccug 19
<210> 7
   <211> 21
   <212> RNA
   <213> artificial
<220>
   <223> "scrambled" sequence for preparation of negative control siRNA or shRNA
<400> 7
   guuggccauu cuacuucgca a 21
<210> 8
   <211> 21
   <212> RNA
   <213> artificial
<220>
   <223> sequence for preparation of siRNA or shRNA targeting TXNIP expression
<400> 8
   aaacuaaccc cucuuuuucu c 21
<210> 9
   <211> 20
   <212> RNA
   <213> artificial
<220>
   <223> sequence for preparing siRNA or shRNA targeting TXNIP expression
<400> 9
   cuaaccccuc uuuuucucuu 20
<210> 10
   <211> 21
   <212> RNA
   <213> artificial
<220>
   <223> sequence for preparation of siRNA or shRNA targeting TXNIP expression
<400> 10
   gagaaaaaga gggguuaguu u 21
<210> 11
   <211> 21
   <212> RNA
   <213> artificial
<220>
   <223> sequence for preparation of siRNA or shRNA targeting TXNIP expression
<400> 11
   aaccccucuu uuucuccaaa g 21
<210> 12
   <211> 21
   <212> RNA
   <213> artificial
<220>
   <223> sequence for preparation of siRNA or shRNA targeting TXNIP expression
<400> 12
   ccccucuuuu ucuccaaagu u 21
<210> 13
   <211> 21
   <212> RNA
   <213> artificial
<220>
   <223> sequence for preparation of siRNA or shRNA targeting TXNIP expression
<400> 13
   cuuuggagaa aaagaggggu u 21
<210> 14
   <211> 21
   <212> RNA
   <213> artificial
<220>
   <223> sequence for preparation of siRNA or shRNA targeting TXNIP expression
<400> 14
   aaaggagugc uuguggagau c 21
<210> 15
   <211> 21
   <212> RNA
   <213> artificial
<220>
   <223> sequence for preparation of siRNA or shRNA targeting TXNIP expression
<400> 15
   aggagugcuu guggagaucu u 21
<210> 16
   <211> 21
   <212> RNA
   <213> artificial
<220>
   <223> sequence for preparation of siRNA or shRNA targeting TXNIP expression
<400> 16
   gaucuccaca agcacuccuu u 21
<210> 17
   <211> 21
   <212> RNA
   <213> artificial
<220>
   <223> sequence for preparation of siRNA or shRNA targeting TXNIP expression
<400> 17
   aauuggggga aagaaggcuu u 21
<210> 18
   <211> 21
   <212> RNA
   <213> artificial
<220>
   <223> sequence for preparation of siRNA or shRNA targeting TXNIP expression
<400> 18
   uugggggaaa gaaggcuuuu u 21
<210> 19
   <211> 21
   <212> RNA
   <213> artificial
<220>
   <223> sequence for preparation of siRNA or shRNA targeting TXNIP expression
<400> 19
   aaagccuucu uucccccaau u 21
<210> 20
   <211> 21
   <212> RNA
   <213> artificial
<220>
   <223> sequence for preparation of siRNA or shRNA targeting TXNIP expression
<400> 20
   aaagaaggcu uuuucucuga a 21
<210> 21
   <211> 21
   <212> RNA
   <213> artificial
<220>
   <223> sequence for preparation of siRNA or shRNA targeting TXNIP expression
<400> 21
   agaaggcuuu uucucugaau u 21
<210> 22
   <211> 21
   <212> RNA
   <213> artificial
<220>
   <223> sequence for preparation of siRNA or shRNA targeting TXNIP expression
<400> 22
   uucagagaaa aagccuucuu u 21
<210> 23
   <211> 21
   <212> RNA
   <213> artificial
<220>
   <223> sequence for preparation of siRNA or shRNA targeting TXNIP expression
<400> 23
   aaggcuuuuu cucugaauuc g 21
<210> 24
   <211> 21
   <212> RNA
   <213> artificial
<220>
   <223> sequence for preparation of siRNA or shRNA targeting TXNIP expression
<400> 24
   ggcuuuuucu cugaauucgu u 21
<210> 25
   <211> 21
   <212> RNA
   <213> artificial
<220>
   <223> sequence for preparation of siRNA or shRNA targeting TXNIP expression
<400> 25
   cgaauucaga gaaaaagccu u 21
<210> 26
   <211> 21
   <212> RNA
   <213> artificial
<220>
   <223> sequence for preparation of siRNA or shRNA targeting TXNIP expression
<400> 26
   aauucgcuua guguaaccag c 21
<210> 27
   <211> 21
   <212> RNA
   <213> artificial
<220>
   <223> sequence for preparation of siRNA or shRNA targeting TXNIP expression
<400> 27
   uucgcuuagu guaaccagcu u 21
<210> 28
   <211> 21
   <212> RNA
   <213> artificial
<220>
   <223> sequence for preparation of siRNA or shRNA targeting TXNIP expression
<400> 28
   gcugguuaca cuaagcgaau u 21
<210> 29
   <211> 21
   <212> RNA
   <213> artificial
<220>
   <223> sequence for preparation of siRNA or shRNA targeting TXNIP expression
<400> 29
   aaccagcggc guauauuuuu u 21
<210> 30
   <211> 21
   <212> RNA
   <213> artificial
<220>
   <223> sequence for preparation of siRNA or shRNA targeting TXNIP expression
<400> 30
   ccagcggcgu auauuuuuuu u 21
<210> 31
   <211> 21
   <212> RNA
   <213> artificial
<220>
   <223> sequence for preparation of siRNA or shRNA targeting TXNIP expression
<400> 31
   aaaaaauaua cgccgcuggu u 21
<210> 32
   <211> 21
   <212> RNA
   <213> artificial
<220>
   <223> sequence for preparation of siRNA or shRNA targeting TXNIP expression
<400> 32
   aaccuaguag uuaauauuca u 21
<210> 33
   <211> 21
   <212> RNA
   <213> artificial
<220>
   <223> sequence for preparation of siRNA or shRNA targeting TXNIP expression
<400> 33
   ccuaguaguu aauauucauu u 21
<210> 34
   <211> 21
   <212> RNA
   <213> artificial
<220>
   <223> sequence for preparation of siRNA or shRNA targeting TXNIP expression
<400> 34
   augaauauua acuacuaggu u 21
<210> 35
   <211> 21
   <212> RNA
   <213> artificial
<220>
   <223> sequence for preparation of siRNA or shRNA targeting TXNIP expression
<400> 35
   aauauucauu uguuuaaauc u 21
<210> 36
   <211> 21
   <212> RNA
   <213> artificial
<220>
   <223> sequence for preparation of siRNA or shRNA targeting TXNIP expression
<400> 36
   uauucauuug uuuaaaucuu u 21
<210> 37
   <211> 21
   <212> RNA
   <213> artificial
<220>
   <223> sequence for preparation of siRNA or shRNA targeting TXNIP expression
<400> 37
   agauuuaaac aaaugaauau u 21
<210> 38
   <211> 21
   <212> RNA
   <213> artificial
<220>
   <223> sequence for preparation of siRNA or shRNA targeting TXNIP expression
<400> 38
   aaaucuuauu uuauuuuuaa g 21
<210> 39
   <211> 21
   <212> RNA
   <213> artificial
<220>
   <223> sequence for preparation of siRNA or shRNA targeting TXNIP expression
<400> 39
   aucuuauuuu auuuuuaagu u 21
<210> 40
   <211> 21
   <212> RNA
   <213> artificial
<220>
   <223> sequence for preparation of siRNA or shRNA targeting TXNIP expression
<400> 40
   cuuaaaaaua aaauaagauu u 21
<210> 41
   <211> 21
   <212> RNA
   <213> artificial
<220>
   <223> sequence for preparation of siRNA or shRNA targeting TXNIP expression
<400> 41
   aagcucaaac ugcuuaagaa u 21
<210> 42
   <211> 21
   <212> RNA
   <213> artificial
<220>
   <223> sequence for preparation of siRNA or shRNA targeting TXNIP expression
<400> 42
   gcucaaacug cuuaagaauu u 21
<210> 43
   <211> 21
   <212> RNA
   <213> artificial
<220>
   <223> sequence for preparation of siRNA or shRNA targeting TXNIP expression
<400> 43
   auucuuaagc aguuugagcu u 21
<210> 44
   <211> 21
   <212> RNA
   <213> artificial
<220>
   <223> sequence for preparation of siRNA or shRNA targeting TXNIP expression
<400> 44
   aaacugcuua agaauaccuu a 21
<210> 45
   <211> 21
   <212> RNA
   <213> artificial
<220>
   <223> sequence for preparation of siRNA or shRNA targeting TXNIP expression
<400> 45
   acugcuuaag aauaccuuau u 21
<210> 46
   <211> 21
   <212> RNA
   <213> artificial
<220>
   <223> sequence for preparation of siRNA or shRNA targeting TXNIP expression
<400> 46
   uaagguauuc uuaagcaguu u 21
<210> 47
   <211> 21
   <212> RNA
   <213> artificial
<220>
   <223> sequence for preparation of siRNA or shRNA targeting TXNIP expression
<400> 47
   aagaauaccu uaauuccuua a 21
<210> 48
   <211> 21
   <212> RNA
   <213> artificial
<220>
   <223> sequence for preparation of siRNA or shRNA targeting TXNIP expression
<400> 48
   gaauaccuua auuccuuaau u 21
<210> 49
   <211> 21
   <212> RNA
   <213> artificial
<220>
   <223> sequence for preparation of siRNA or shRNA targeting TXNIP expression
<400> 49
   uuaaggaauu aagguauucu u 21
<210> 50
   <211> 21
   <212> RNA
   <213> artificial
<220>
   <223> sequence for preparation of siRNA or shRNA targeting TXNIP expression
<400> 50
   aauaccuuaa uuccuuaaag u 21
<210> 51
   <211> 21
   <212> RNA
   <213> artificial
<220>
   <223> sequence for preparation of siRNA or shRNA targeting TXNIP expression
<400> 51
   uaccuuaauu ccuuaaaguu u 21
<210> 52
   <211> 21
   <212> RNA
   <213> artificial
<220>
   <223> sequence for preparation of siRNA or shRNA targeting TXNIP expression
<400> 52
   acuuuaagga auuaagguau u 21
<210> 53
   <211> 21
   <212> RNA
   <213> artificial
<220>
   <223> sequence for preparation of siRNA or shRNA targeting TXNIP expression
<400> 53
   aauuccuuaa agugaaauaa u 21
<210> 54
   <211> 21
   <212> RNA
   <213> artificial
<220>
   <223> sequence for preparation of siRNA or shRNA targeting TXNIP expression
<400> 54
   uuccuuaaag ugaaauaauu u 21
<210> 55
   <211> 21
   <212> RNA
   <213> artificial
<220>
   <223> sequence for preparation of siRNA or shRNA targeting TXNIP expression
<400> 55
   auuauuucac uuuaaggaau u 21
<210> 56
   <211> 21
   <212> RNA
   <213> artificial
<220>
   <223> sequence for preparation of siRNA or shRNA targeting TXNIP expression
<400> 56
   aaagugaaau aauuuuuugc a 21
<210> 57
   <211> 21
   <212> RNA
   <213> artificial
<220>
   <223> sequence for preparation of siRNA or shRNA targeting TXNIP expression
<400> 57
   agugaaauaa uuuuuugcau u 21
<210> 58
   <211> 21
   <212> RNA
   <213> artificial
<220>
   <223> sequence for preparation of siRNA or shRNA targeting TXNIP expression
<400> 58
   ugcaaaaaau uauuucacuu u 21
<210> 59
   <211> 21
   <212> RNA
   <213> artificial
<220>
   <223> sequence for preparation of siRNA or shRNA targeting TXNIP expression
<400> 59
   aaauaauuuu uugcaaaggg g 21
<210> 60
   <211> 21
   <212> RNA
   <213> artificial
<220>
   <223> sequence for preparation of siRNA or shRNA targeting TXNIP expression
<400> 60
   auaauuuuuu gcaaaggggu u 21
<210> 61
   <211> 21
   <212> RNA
   <213> artificial
<220>
   <223> sequence for preparation of siRNA or shRNA targeting TXNIP expression
<400> 61
   ccccuuugca aaaaauuauu u 21
<210> 62
   <211> 21
   <212> RNA
   <213> artificial
<220>
   <223> sequence for preparation of siRNA or shRNA targeting TXNIP expression
<400> 62
   aauuuuuugc aaagggguuu c 21
<210> 63
   <211> 21
   <212> RNA
   <213> artificial
<220>
   <223> sequence for preparation of siRNA or shRNA targeting TXNIP expression
<400> 63
   uuuuuugcaa agggguuucu u 21
<210> 64
   <211> 21
   <212> RNA
   <213> artificial
<220>
   <223> sequence for preparation of siRNA or shRNA targeting TXNIP expression
<400> 64
   gaaaccccuu ugcaaaaaau u 21
<210> 65
   <211> 21
   <212> RNA
   <213> artificial
<220>
   <223> sequence for preparation of siRNA or shRNA targeting TXNIP expression
<400> 65
   aaagggguuu ccucgauuug g 21
<210> 66
   <211> 21
   <212> RNA
   <213> artificial
<220>
   <223> sequence for preparation of siRNA or shRNA targeting TXNIP expression
<400> 66
   agggguuucc ucgauuuggu u 21
<210> 67
   <211> 21
   <212> RNA
   <213> artificial
<220>
   <223> sequence for preparation of siRNA or shRNA targeting TXNIP expression
<400> 67
   ccaaaucgag gaaaccccuu u 21
<210> 68
   <211> 21
   <212> RNA
   <213> artificial
<220>
   <223> sequence for preparation of siRNA or shRNA targeting TXNIP expression
<400> 68
   aaccaacuca guuccaucau g 21
<210> 69
   <211> 21
   <212> RNA
   <213> artificial
<220>
   <223> sequence for preparation of siRNA or shRNA targeting TXNIP expression
<400> 69
   ccaacucagu uccaucaugu u 21
<210> 70
   <211> 21
   <212> RNA
   <213> artificial
<220>
   <223> sequence for preparation of siRNA or shRNA targeting TXNIP expression
<400> 70
   caugauggaa cugaguuggu u 21
<210> 71
   <211> 21
   <212> RNA
   <213> artificial
<220>
   <223> sequence for preparation of siRNA or shRNA targeting TXNIP expression
<400> 71
   aacucaguuc caucauggug a 21
<210> 72
   <211> 21
   <212> RNA
   <213> artificial
<220>
   <223> sequence for preparation of siRNA or shRNA targeting TXNIP expression
<400> 72
   cucaguucca ucauggugau u 21
<210> 73
   <211> 21
   <212> RNA
   <213> artificial
<220>
   <223> sequence for preparation of siRNA or shRNA targeting TXNIP expression
<400> 73
   ucaccaugau ggaacugagu u 21
<210> 74
   <211> 21
   <212> RNA
   <213> artificial
<220>
   <223> sequence for preparation of siRNA or shRNA targeting TXNIP expression
<400> 74
   aagaagauca agucuuuuga g 21
<210> 75
   <211> 21
   <212> RNA
   <213> artificial
<220>
   <223> sequence for preparation of siRNA or shRNA targeting TXNIP expression
<400> 75
   gaagaucaag ucuuuugagu u 21
<210> 76
   <211> 21
   <212> RNA
   <213> artificial
<220>
   <223> sequence for preparation of siRNA or shRNA targeting TXNIP expression
<400> 76
   cucaaaagac uugaucuucu u 21
<210> 77
   <211> 21
   <212> RNA
   <213> artificial
<220>
   <223> sequence for preparation of siRNA or shRNA targeting TXNIP expression
<400> 77
   aagaucaagu cuuuugaggu g 21
<210> 78
   <211> 21
   <212> RNA
   <213> artificial
<220>
   <223> sequence for preparation of siRNA or shRNA targeting TXNIP expression
<400> 78
   gaucaagucu uuugaggugu u 21
<210> 79
   <211> 21
   <212> RNA
   <213> artificial
<220>
   <223> sequence for preparation of siRNA or shRNA targeting TXNIP expression
<400> 79
   caccucaaaa gacuugaucu u 21
<210> 80
   <211> 21
   <212> RNA
   <213> artificial
<220>
   <223> sequence for preparation of siRNA or shRNA targeting TXNIP expression
<400> 80
   aagucuuuug agguggucuu u 21
<210> 81
   <211> 21
   <212> RNA
   <213> artificial
<220>
   <223> sequence for preparation of siRNA or shRNA targeting TXNIP expression
<400> 81
   gucuuuugag guggucuuuu u 21
<210> 82
   <211> 21
   <212> RNA
   <213> artificial
<220>
   <223> sequence for preparation of siRNA or shRNA targeting TXNIP expression
<400> 82
   aaagaccacc ucaaaagacu u 21
<210> 83
   <211> 21
   <212> RNA
   <213> artificial
<220>
   <223> sequence for preparation of siRNA or shRNA targeting TXNIP expression
<400> 83
   aagguguacg gcaguggcga g 21
<210> 84
   <211> 21
   <212> RNA
   <213> artificial
<220>
   <223> sequence for preparation of siRNA or shRNA targeting TXNIP expression
<400> 84
   gguguacggc aguggcgagu u 21
<210> 85
   <211> 21
   <212> RNA
   <213> artificial
<220>
   <223> sequence for preparation of siRNA or shRNA targeting TXNIP expression
<400> 85
   cucgccacug ccguacaccu u 21
<210> 86
   <211> 21
   <212> RNA
   <213> artificial
<220>
   <223> sequence for preparation of siRNA or shRNA targeting TXNIP expression
<400> 86
   aagguggcug gccgggugau a 21
<210> 87
   <211> 21
   <212> RNA
   <213> artificial
<220>
   <223> sequence for preparation of siRNA or shRNA targeting TXNIP expression
<400> 87
   gguggcuggc cgggugauau u 21
<210> 88
   <211> 21
   <212> RNA
   <213> artificial
<220>
   <223> sequence for preparation of siRNA or shRNA targeting TXNIP expression
<400> 88
   uaucacccgg ccagccaccu u 21
<210> 89
   <211> 21
   <212> RNA
   <213> artificial
<220>
   <223> sequence for preparation of siRNA or shRNA targeting TXNIP expression
<400> 89
   aaguuacucg ugucaaagcc g 21
<210> 90
   <211> 21
   <212> RNA
   <213> artificial
<220>
   <223> sequence for preparation of siRNA or shRNA targeting TXNIP expression
<400> 90
   guuacucgug ucaaagccgu u 21
<210> 91
   <211> 21
   <212> RNA
   <213> artificial
<220>
   <223> sequence for preparation of siRNA or shRNA targeting TXNIP expression
<400> 91
   cggcuuugac acgaguaacu u 21
<210> 92
   <211> 21
   <212> RNA
   <213> artificial
<220>
   <223> sequence for preparation of siRNA or shRNA targeting TXNIP expression
<400> 92
   aaagccguua ggauccuggc u 21
<210> 93
   <211> 21
   <212> RNA
   <213> artificial
<220>
   <223> sequence for preparation of siRNA or shRNA targeting TXNIP expression
<400> 93
   agccguuagg auccuggcuu u 21
<210> 94
   <211> 21
   <212> RNA
   <213> artificial
<220>
   <223> sequence for preparation of siRNA or shRNA targeting TXNIP expression
<400> 94
   agccaggauc cuaacggcuu u 21
<210> 95
   <211> 21
   <212> RNA
   <213> artificial
<220>
   <223> sequence for preparation of siRNA or shRNA targeting TXNIP expression
<400> 95
   aaagugcuuu ggaugcaggg a 21
<210> 96
   <211> 21
   <212> RNA
   <213> artificial
<220>
   <223> sequence for preparation of siRNA or shRNA targeting TXNIP expression
<400> 96
   agugcuuugg augcagggau u 21
<210> 97
   <211> 21
   <212> RNA
   <213> artificial
<220>
   <223> sequence for preparation of siRNA or shRNA targeting TXNIP expression
<400> 97
   ucccugcauc caaagcacuu u 21
<210> 98
   <211> 21
   <212> RNA
   <213> artificial
<220>
   <223> sequence for preparation of siRNA or shRNA targeting TXNIP expression
<400> 98
   aaacagacuu cggaguaccu g 21
<210> 99
   <211> 21
   <212> RNA
   <213> artificial
<220>
   <223> sequence for preparation of siRNA or shRNA targeting TXNIP expression
<400> 99
   acagacuucg gaguaccugu u 21
<210> 100
   <211> 21
   <212> RNA
   <213> artificial
<220>
   <223> sequence for preparation of siRNA or shRNA targeting TXNIP expression
<400> 100
   cagguacucc gaagucuguu u 21
<210> 101
   <211> 21
   <212> RNA
   <213> artificial
<220>
   <223> sequence for preparation of siRNA or shRNA targeting TXNIP expression
<400> 101
   aagacacgcu ucuucuggaa g 21
<210> 102
   <211> 21
   <212> RNA
   <213> artificial
<220>
   <223> sequence for preparation of siRNA or shRNA targeting TXNIP expression
<400> 102
   gacacgcuuc uucuggaagu u 21
<210> 103
   <211> 21
   <212> RNA
   <213> artificial
<220>
   <223> sequence for preparation of siRNA or shRNA targeting TXNIP expression
<400> 103
   cuuccagaag aagcgugucu u 21
<210> 104
   <211> 21
   <212> RNA
   <213> artificial
<220>
   <223> sequence for preparation of siRNA or shRNA targeting TXNIP expression
<400> 104
   aagaccagcc aacaggugag a 21
<210> 105
   <211> 21
   <212> RNA
   <213> artificial
<220>
   <223> sequence for preparation of siRNA or shRNA targeting TXNIP expression
<400> 105
   gaccagccaa caggugagau u 21
<210> 106
   <211> 21
   <212> RNA
   <213> artificial
<220>
   <223> sequence for preparation of siRNA or shRNA targeting TXNIP expression
<400> 106
   ucucaccugu uggcuggucu u 21
<210> 107
   <211> 21
   <212> RNA
   <213> artificial
<220>
   <223> sequence for preparation of siRNA or shRNA targeting TXNIP expression
<400> 107
   aacaggugag aaugagaugg u 21
<210> 108
   <211> 21
   <212> RNA
   <213> artificial
<220>
   <223> sequence for preparation of siRNA or shRNA targeting TXNIP expression
<400> 108
   caggugagaa ugagaugguu u 21
<210> 109
   <211> 21
   <212> RNA
   <213> artificial
<220>
   <223> sequence for preparation of siRNA or shRNA targeting TXNIP expression
<400> 109
   accaucucau ucucaccugu u 21
<210> 110
   <211> 21
   <212> RNA
   <213> artificial
<220>
   <223> sequence for preparation of siRNA or shRNA targeting TXNIP expression
<400> 110
   aaugagaugg ugaucaugag a 21
<210> 111
   <211> 21
   <212> RNA
   <213> artificial
<220>
   <223> sequence for preparation of siRNA or shRNA targeting TXNIP expression
<400> 111
   ugagauggug aucaugagau u 21
<210> 112
   <211> 21
   <212> RNA
   <213> artificial
<220>
   <223> sequence for preparation of siRNA or shRNA targeting TXNIP expression
<400> 112
   ucucaugauc accaucucau u 21
<210> 113
   <211> 21
   <212> RNA
   <213> artificial
<220>
   <223> sequence for preparation of siRNA or shRNA targeting TXNIP expression
<400> 113
   aaacaaauau gaguacaagu u 21
<210> 114
   <211> 21
   <212> RNA
   <213> artificial
<220>
   <223> sequence for preparation of siRNA or shRNA targeting TXNIP expression
<400> 114
   acaaauauga guacaaguuu u 21
<210> 115
   <211> 21
   <212> RNA
   <213> artificial
<220>
   <223> sequence for preparation of siRNA or shRNA targeting TXNIP expression
<400> 115
   aacuuguacu cauauuuguu u 21
<210> 116
   <211> 21
   <212> RNA
   <213> artificial
<220>
   <223> sequence for preparation of siRNA or shRNA targeting TXNIP expression
<400> 116
   aaauaugagu acaaguucgg c 21
<210> 117
   <211> 21
   <212> RNA
   <213> artificial
<220>
   <223> sequence for preparation of siRNA or shRNA targeting TXNIP expression
<400> 117
   auaugaguac aaguucggcu u 21
<210> 118
   <211> 21
   <212> RNA
   <213> artificial
<220>
   <223> sequence for preparation of siRNA or shRNA targeting TXNIP expression
<400> 118
   gccgaacuug uacucauauu u 21
<210> 119
   <211> 21
   <212> RNA
   <213> artificial
<220>
   <223> sequence for preparation of siRNA or shRNA targeting TXNIP expression
<400> 119
   aaguucggcu uugagcuucc u 21
<210> 120
   <211> 21
   <212> RNA
   <213> artificial
<220>
   <223> sequence for preparation of siRNA or shRNA targeting TXNIP expression
<400> 120
   guucggcuuu gagcuuccuu u 21
<210> 121
   <211> 21
   <212> RNA
   <213> artificial
<220>
   <223> sequence for preparation of siRNA or shRNA targeting TXNIP expression
<400> 121
   aggaagcuca aagccgaacu u 21
<210> 122
   <211> 21
   <212> RNA
   <213> artificial
<220>
   <223> sequence for preparation of siRNA or shRNA targeting TXNIP expression
<400> 122
   aauaugggug uguagacuac u 21
<210> 123
   <211> 21
   <212> RNA
   <213> artificial
<220>
   <223> sequence for preparation of siRNA or shRNA targeting TXNIP expression
<400> 123
   uaugggugug uagacuacuu u 21
<210> 124
   <211> 21
   <212> RNA
   <213> artificial
<220>
   <223> sequence for preparation of siRNA or shRNA targeting TXNIP expression
<400> 124
   aguagucuac acacccauau u 21
<210> 125
   <211> 21
   <212> RNA
   <213> artificial
<220>
   <223> sequence for preparation of siRNA or shRNA targeting TXNIP expression
<400> 125
   aaggcuuuuc uugaccgccc g 21
<210> 126
   <211> 21
   <212> RNA
   <213> artificial
<220>
   <223> sequence for preparation of siRNA or shRNA targeting TXNIP expression
<400> 126
   ggcuuuucuu gaccgcccgu u 21
<210> 127
   <211> 21
   <212> RNA
   <213> artificial
<220>
   <223> sequence for preparation of siRNA or shRNA targeting TXNIP expression
<400> 127
   cgggcgguca agaaaagccu u 21
<210> 128
   <211> 21
   <212> RNA
   <213> artificial
<220>
   <223> sequence for preparation of siRNA or shRNA targeting TXNIP expression
<400> 128
   aaacuuugaa guaguggauc u 21
<210> 129
   <211> 21
   <212> RNA
   <213> artificial
<220>
   <223> sequence for preparation of siRNA or shRNA targeting TXNIP expression
<400> 129
   acuuugaagu aguggaucuu u 21
<210> 130
   <211> 21
   <212> RNA
   <213> artificial
<220>
   <223> sequence for preparation of siRNA or shRNA targeting TXNIP expression
<400> 130
   agauccacua cuucaaaguu u 21
<210> 131
   <211> 21
   <212> RNA
   <213> artificial
<220>
   <223> sequence for preparation of siRNA or shRNA targeting TXNIP expression
<400> 131
   aaguagugga ucugguggau g 21
<210> 132
   <211> 21
   <212> RNA
   <213> artificial
<220>
   <223> sequence for preparation of siRNA or shRNA targeting TXNIP expression
<400> 132
   guaguggauc ugguggaugu u 21
<210> 133
   <211> 21
   <212> RNA
   <213> artificial
<220>
   <223> sequence for preparation of siRNA or shRNA targeting TXNIP expression
<400> 133
   cauccaccag auccacuacu u 21
<210> 134
   <211> 21
   <212> RNA
   <213> artificial
<220>
   <223> sequence for preparation of siRNA or shRNA targeting TXNIP expression
<400> 134
   aauaccccug auuuaauggc a 21
<210> 135
   <211> 21
   <212> RNA
   <213> artificial
<220>
   <223> sequence for preparation of siRNA or shRNA targeting TXNIP expression
<400> 135
   uaccccugau uuaauggcau u 21
<210> 136
   <211> 21
   <212> RNA
   <213> artificial
<220>
   <223> sequence for preparation of siRNA or shRNA targeting TXNIP expression
<400> 136
   ugccauuaaa ucagggguau u 21
<210> 137
   <211> 21
   <212> RNA
   <213> artificial
<220>
   <223> sequence for preparation of siRNA or shRNA targeting TXNIP expression
<400> 137
   aauggcaccu gugucugcua a 21
<210> 138
   <211> 21
   <212> RNA
   <213> artificial
<220>
   <223> sequence for preparation of siRNA or shRNA targeting TXNIP expression
<400> 138
   uggcaccugu gucugcuaau u 21
<210> 139
   <211> 21
   <212> RNA
   <213> artificial
<220>
   <223> sequence for preparation of siRNA or shRNA targeting TXNIP expression
<400> 139
   uuagcagaca caggugccau u 21
<210> 140
   <211> 21
   <212> RNA
   <213> artificial
<220>
   <223> sequence for preparation of siRNA or shRNA targeting TXNIP expression
<400> 140
   aagaaaguuu ccugcauguu c 21
<210> 141
   <211> 21
   <212> RNA
   <213> artificial
<220>
   <223> sequence for preparation of siRNA or shRNA targeting TXNIP expression
<400> 141
   gaaaguuucc ugcauguucu u 21
<210> 142
   <211> 21
   <212> RNA
   <213> artificial
<220>
   <223> sequence for preparation of siRNA or shRNA targeting TXNIP expression
<400> 142
   gaacaugcag gaaacuuucu u 21
<210> 143
   <211> 21
   <212> RNA
   <213> artificial
<220>
   <223> sequence for preparation of siRNA or shRNA targeting TXNIP expression
<400> 143
   aaaguuuccu gcauguucau u 21
<210> 144
   <211> 21
   <212> RNA
   <213> artificial
<220>
   <223> sequence for preparation of siRNA or shRNA targeting TXNIP expression
<400> 144
   aguuuccugc auguucauuu u 21
<210> 145
   <211> 21
   <212> RNA
   <213> artificial
<220>
   <223> sequence for preparation of siRNA or shRNA targeting TXNIP expression
<400> 145
   aaugaacaug caggaaacuu u 21
<210> 146
   <211> 21
   <212> RNA
   <213> artificial
<220>
   <223> sequence for preparation of siRNA or shRNA targeting TXNIP expression
<400> 146
   aaggauucug ugaaggugau g 21
<210> 147
   <211> 21
   <212> RNA
   <213> artificial
<220>
   <223> sequence for preparation of siRNA or shRNA targeting TXNIP expression
<400> 147
   ggauucugug aaggugaugu u 21
<210> 148
   <211> 21
   <212> RNA
   <213> artificial
<220>
   <223> sequence for preparation of siRNA or shRNA targeting TXNIP expression
<400> 148
   caucaccuuc acagaauccu u 21
<210> 149
   <211> 21
   <212> RNA
   <213> artificial
<220>
   <223> sequence for preparation of siRNA or shRNA targeting TXNIP expression
<400> 149
   aaggugauga gauuuccauc c 21
<210> 150
   <211> 21
   <212> RNA
   <213> artificial
<220>
   <223> sequence for preparation of siRNA or shRNA targeting TXNIP expression
<400> 150
   ggugaugaga uuuccauccu u 21
<210> 151
   <211> 21
   <212> RNA
   <213> artificial
<220>
   <223> sequence for preparation of siRNA or shRNA targeting TXNIP expression
<400> 151
   ggauggaaau cucaucaccu u 21
<210> 152
   <211> 21
   <212> RNA
   <213> artificial
<220>
   <223> sequence for preparation of siRNA or shRNA targeting TXNIP expression
<400> 152
   aauacauguu cccgaauugu g 21
<210> 153
   <211> 21
   <212> RNA
   <213> artificial
<220>
   <223> sequence for preparation of siRNA or shRNA targeting TXNIP expression
<400> 153
   uacauguucc cgaauugugu u 21
<210> 154
   <211> 21
   <212> RNA
   <213> artificial
<220>
   <223> sequence for preparation of siRNA or shRNA targeting TXNIP expression
<400> 154
   cacaauucgg gaacauguau u 21
<210> 155
   <211> 21
   <212> RNA
   <213> artificial
<220>
   <223> sequence for preparation of siRNA or shRNA targeting TXNIP expression
<400> 155
   aauugugguc cccaaagcug c 21
<210> 156
   <211> 21
   <212> RNA
   <213> artificial
<220>
   <223> sequence for preparation of siRNA or shRNA targeting TXNIP expression
<400> 156
   uugugguccc caaagcugcu u 21
<210> 157
   <211> 21
   <212> RNA
   <213> artificial
<220>
   <223> sequence for preparation of siRNA or shRNA targeting TXNIP expression
<400> 157
   gcagcuuugg ggaccacaau u 21
<210> 158
   <211> 21
   <212> RNA
   <213> artificial
<220>
   <223> sequence for preparation of siRNA or shRNA targeting TXNIP expression
<400> 158
   aaagcugcca uuguggcccg c 21
<210> 159
   <211> 21
   <212> RNA
   <213> artificial
<220>
   <223> sequence for preparation of siRNA or shRNA targeting TXNIP expression
<400> 159
   agcugccauu guggcccgcu u 21
<210> 160
   <211> 21
   <212> RNA
   <213> artificial
<220>
   <223> sequence for preparation of siRNA or shRNA targeting TXNIP expression
<400> 160
   gcgggccaca auggcagcuu u 21
<210> 161
   <211> 21
   <212> RNA
   <213> artificial
<220>
   <223> sequence for preparation of siRNA or shRNA targeting TXNIP expression
<400> 161
   aauggccaga ccaaggugcu g 21
<210> 162
   <211> 21
   <212> RNA
   <213> artificial
<220>
   <223> sequence for preparation of siRNA or shRNA targeting TXNIP expression
<400> 162
   uggccagacc aaggugcugu u 21
<210> 163
   <211> 21
   <212> RNA
   <213> artificial
<220>
   <223> sequence for preparation of siRNA or shRNA targeting TXNIP expression
<400> 163
   cagcaccuug gucuggccau u 21
<210> 164
   <211> 21
   <212> RNA
   <213> artificial
<220>
   <223> sequence for preparation of siRNA or shRNA targeting TXNIP expression
<400> 164
   aaggugcuga cucagaaguu g 21
<210> 165
   <211> 21
   <212> RNA
   <213> artificial
<220>
   <223> sequence for preparation of siRNA or shRNA targeting TXNIP expression
<400> 165
   ggugcugacu cagaaguugu u 21
<210> 166
   <211> 21
   <212> RNA
   <213> artificial
<220>
   <223> sequence for preparation of siRNA or shRNA targeting TXNIP expression
<400> 166
   caacuucuga gucagcaccu u 21
<210> 167
   <211> 21
   <212> RNA
   <213> artificial
<220>
   <223> sequence for preparation of siRNA or shRNA targeting TXNIP expression
<400> 167
   aaguugucau cagucagagg c 21
<210> 168
   <211> 21
   <212> RNA
   <213> artificial
<220>
   <223> sequence for preparation of siRNA or shRNA targeting TXNIP expression
<400> 168
   guugucauca gucagaggcu u 21
<210> 169
   <211> 21
   <212> RNA
   <213> artificial
<220>
   <223> sequence for preparation of siRNA or shRNA targeting TXNIP expression
<400> 169
   gccucugacu gaugacaacu u 21
<210> 170
   <211> 21
   <212> RNA
   <213> artificial
<220>
   <223> sequence for preparation of siRNA or shRNA targeting TXNIP expression
<400> 170
   aaucauauua ucucagggac a 21
<210> 171
   <211> 21
   <212> RNA
   <213> artificial
<220>
   <223> sequence for preparation of siRNA or shRNA targeting TXNIP expression
<400> 171
   ucauauuauc ucagggacau u 21
<210> 172
   <211> 21
   <212> RNA
   <213> artificial
<220>
   <223> sequence for preparation of siRNA or shRNA targeting TXNIP expression
<400> 172
   ugucccugag auaauaugau u 21
<210> 173
   <211> 21
   <212> RNA
   <213> artificial
<220>
   <223> sequence for preparation of siRNA or shRNA targeting TXNIP expression
<400> 173
   aagagccuuc ggguucagaa g 21
<210> 174
   <211> 21
   <212> RNA
   <213> artificial
<220>
   <223> sequence for preparation of siRNA or shRNA targeting TXNIP expression
<400> 174
   gagccuucgg guucagaagu u 21
<210> 175
   <211> 21
   <212> RNA
   <213> artificial
<220>
   <223> sequence for preparation of siRNA or shRNA targeting TXNIP expression
<400> 175
   cuucugaacc cgaaggcucu u 21
<210> 176
   <211> 21
   <212> RNA
   <213> artificial
<220>
   <223> sequence for preparation of siRNA or shRNA targeting TXNIP expression
<400> 176
   aagaucaggc cuucuauccu g 21
<210> 177
   <211> 21
   <212> RNA
   <213> artificial
<220>
   <223> sequence for preparation of siRNA or shRNA targeting TXNIP expression
<400> 177
   gaucaggccu ucuauccugu u 21
<210> 178
   <211> 21
   <212> RNA
   <213> artificial
<220>
   <223> sequence for preparation of siRNA or shRNA targeting TXNIP expression
<400> 178
   caggauagaa ggccugaucu u 21
<210> 179
   <211> 21
   <212> RNA
   <213> artificial
<220>
   <223> sequence for preparation of siRNA or shRNA targeting TXNIP expression
<400> 179
   aacauccuuc gaguugaaua u 21
<210> 180
   <211> 21
   <212> RNA
   <213> artificial
<220>
   <223> sequence for preparation of siRNA or shRNA targeting TXNIP expression
<400> 180
   cauccuucga guugaauauu u 21
<210> 181
   <211> 21
   <212> RNA
   <213> artificial
<220>
   <223> sequence for preparation of siRNA or shRNA targeting TXNIP expression
<400> 181
   auauucaacu cgaaggaugu u 21
<210> 182
   <211> 21
   <212> RNA
   <213> artificial
<220>
   <223> sequence for preparation of siRNA or shRNA targeting TXNIP expression
<400> 182
   aauauuccuu acugaucuau g 21
<210> 183
   <211> 21
   <212> RNA
   <213> artificial
<220>
   <223> sequence for preparation of siRNA or shRNA targeting TXNIP expression
<400> 183
   uauuccuuac ugaucuaugu u 21
<210> 184
   <211> 21
   <212> RNA
   <213> artificial
<220>
   <223> sequence for preparation of siRNA or shRNA targeting TXNIP expression
<400> 184
   cauagaucag uaaggaauau u 21
<210> 185
   <211> 21
   <212> RNA
   <213> artificial
<220>
   <223> sequence for preparation of siRNA or shRNA targeting TXNIP expression
<400> 185
   aagaagguca uccuugaccu g 21
<210> 186
   <211> 21
   <212> RNA
   <213> artificial
<220>
   <223> sequence for preparation of siRNA or shRNA targeting TXNIP expression
<400> 186
   gaaggucauc cuugaccugu u 21
<210> 187
   <211> 21
   <212> RNA
   <213> artificial
<220>
   <223> sequence for preparation of siRNA or shRNA targeting TXNIP expression
<400> 187
   caggucaagg augaccuucu u 21
<210> 188
   <211> 21
   <212> RNA
   <213> artificial
<220>
   <223> sequence for preparation of siRNA or shRNA targeting TXNIP expression
<400> 188
   aaggucaucc uugaccugcc c 21
<210> 189
   <211> 21
   <212> RNA
   <213> artificial
<220>
   <223> sequence for preparation of siRNA or shRNA targeting TXNIP expression
<400> 189
   ggucauccuu gaccugcccu u 21
<210> 190
   <211> 21
   <212> RNA
   <213> artificial
<220>
   <223> sequence for preparation of siRNA or shRNA targeting TXNIP expression
<400> 190
   gggcagguca aggaugaccu u 21
<210> 191
   <211> 21
   <212> RNA
   <213> artificial
<220>
   <223> sequence for preparation of siRNA or shRNA targeting TXNIP expression
<400> 191
   aauuggcagc agaucagguc u 21
<210> 192
   <211> 21
   <212> RNA
   <213> artificial
<220>
   <223> sequence for preparation of siRNA or shRNA targeting TXNIP expression
<400> 192
   uuggcagcag aucaggucuu u 21
<210> 193
   <211> 21
   <212> RNA
   <213> artificial
<220>
   <223> sequence for preparation of siRNA or shRNA targeting TXNIP expression
<400> 193
   agaccugauc ugcugccaau u 21
<210> 194
   <211> 21
   <212> RNA
   <213> artificial
<220>
   <223> sequence for preparation of siRNA or shRNA targeting TXNIP expression
<400> 194
   aagcagcaga acauccagca u 21
<210> 195
   <211> 21
   <212> RNA
   <213> artificial
<220>
   <223> sequence for preparation of siRNA or shRNA targeting TXNIP expression
<400> 195
   gcagcagaac auccagcauu u 21
<210> 196
   <211> 21
   <212> RNA
   <213> artificial
<220>
   <223> sequence for preparation of siRNA or shRNA targeting TXNIP expression
<400> 196
   augcuggaug uucugcugcu u 21
<210> 197
   <211> 21
   <212> RNA
   <213> artificial
<220>
   <223> sequence for preparation of siRNA or shRNA targeting TXNIP expression
<400> 197
   aacauccagc auggccagcc g 21
<210> 198
   <211> 21
   <212> RNA
   <213> artificial
<220>
   <223> sequence for preparation of siRNA or shRNA targeting TXNIP expression
<400> 198
   cauccagcau ggccagccgu u 21
<210> 199
   <211> 21
   <212> RNA
   <213> artificial
<220>
   <223> sequence for preparation of siRNA or shRNA targeting TXNIP expression
<400> 199
   cggcuggcca ugcuggaugu u 21
<210> 200
   <211> 21
   <212> RNA
   <213> artificial
<220>
   <223> sequence for preparation of siRNA or shRNA targeting TXNIP expression
<400> 200
   aaccagcucu gagaugaguu g 21
<210> 201
   <211> 21
   <212> RNA
   <213> artificial
<220>
   <223> sequence for preparation of siRNA or shRNA targeting TXNIP expression
<400> 201
   ccagcucuga gaugaguugu u 21
<210> 202
   <211> 21
   <212> RNA
   <213> artificial
<220>
   <223> sequence for preparation of siRNA or shRNA targeting TXNIP expression
<400> 202
   caacucaucu cagagcuggu u 21
<210> 203
   <211> 21
   <212> RNA
   <213> artificial
<220>
   <223> sequence for preparation of siRNA or shRNA targeting TXNIP expression
<400> 203
   aacaucccug auaccccaga a 21
<210> 204
   <211> 21
   <212> RNA
   <213> artificial
<220>
   <223> sequence for preparation of siRNA or shRNA targeting TXNIP expression
<400> 204
   caucccugau accccagaau u 21
<210> 205
   <211> 21
   <212> RNA
   <213> artificial
<220>
   <223> sequence for preparation of siRNA or shRNA targeting TXNIP expression
<400> 205
   uucuggggua ucagggaugu u 21
<210> 206
   <211> 21
   <212> RNA
   <213> artificial
<220>
   <223> sequence for preparation of siRNA or shRNA targeting TXNIP expression
<400> 206
   aagcuccucc cugcuauaug g 21
<210> 207
   <211> 21
   <212> RNA
   <213> artificial
<220>
   <223> sequence for preparation of siRNA or shRNA targeting TXNIP expression
<400> 207
   gcuccucccu gcuauauggu u 21
<210> 208
   <211> 21
   <212> RNA
   <213> artificial
<220>
   <223> sequence for preparation of siRNA or shRNA targeting TXNIP expression
<400> 208
   ccauauagca gggaggagcu u 21
<210> 209
   <211> 21
   <212> RNA
   <213> artificial
<220>
   <223> sequence for preparation of siRNA or shRNA targeting TXNIP expression
<400> 209
   aagaucaccg auuggagagc c 21
<210> 210
   <211> 21
   <212> RNA
   <213> artificial
<220>
   <223> sequence for preparation of siRNA or shRNA targeting TXNIP expression
<400> 210
   gaucaccgau uggagagccu u 21
<210> 211
   <211> 21
   <212> RNA
   <213> artificial
<220>
   <223> sequence for preparation of siRNA or shRNA targeting TXNIP expression
<400> 211
   ggcucuccaa ucggugaucu u 21
<210> 212
   <211> 21
   <212> RNA
   <213> artificial
<220>
   <223> sequence for preparation of siRNA or shRNA targeting TXNIP expression
<400> 212
   aaccacuccu cugcuagaug a 21
<210> 213
   <211> 21
   <212> RNA
   <213> artificial
<220>
   <223> sequence for preparation of siRNA or shRNA targeting TXNIP expression
<400> 213
   ccacuccucu gcuagaugau u 21
<210> 214
   <211> 21
   <212> RNA
   <213> artificial
<220>
   <223> sequence for preparation of siRNA or shRNA targeting TXNIP expression
<400> 214
   ucaucuagca gaggaguggu u 21
<210> 215
   <211> 21
   <212> RNA
   <213> artificial
<220>
   <223> sequence for preparation of siRNA or shRNA targeting TXNIP expression
<400> 215
   aagacagccc uaucuuuaug u 21
<210> 216
   <211> 21
   <212> RNA
   <213> artificial
<220>
   <223> sequence for preparation of siRNA or shRNA targeting TXNIP expression
<400> 216
   gacagcccua ucuuuauguu u 21
<210> 217
   <211> 21
   <212> RNA
   <213> artificial
<220>
   <223> sequence for preparation of siRNA or shRNA targeting TXNIP expression
<400> 217
   acauaaagau agggcugucu u 21
<210> 218
   <211> 21
   <212> RNA
   <213> artificial
<220>
   <223> sequence for preparation of siRNA or shRNA targeting TXNIP expression
<400> 218
   aaguucaugc caccaccgac u 21
<210> 219
   <211> 21
   <212> RNA
   <213> artificial
<220>
   <223> sequence for preparation of siRNA or shRNA targeting TXNIP expression
<400> 219
   guucaugcca ccaccgacuu u 21
<210> 220
   <211> 21
   <212> RNA
   <213> artificial
<220>
   <223> sequence for preparation of siRNA or shRNA targeting TXNIP expression
<400> 220
   agucgguggu ggcaugaacu u 21
<210> 221
   <211> 21
   <212> RNA
   <213> artificial
<220>
   <223> sequence for preparation of siRNA or shRNA targeting TXNIP expression
<400> 221
   aacaacaaug ugcagugagc a 21
<210> 222
   <211> 21
   <212> RNA
   <213> artificial
<220>
   <223> sequence for preparation of siRNA or shRNA targeting TXNIP expression
<400> 222
   caacaaugug cagugagcau u 21
<210> 223
   <211> 21
   <212> RNA
   <213> artificial
<220>
   <223> sequence for preparation of siRNA or shRNA targeting TXNIP expression
<400> 223
   ugcucacugc acauuguugu u 21
<210> 224
   <211> 21
   <212> RNA
   <213> artificial
<220>
   <223> sequence for preparation of siRNA or shRNA targeting TXNIP expression
<400> 224
   aacaaugugc agugagcaug u 21
<210> 225
   <211> 21
   <212> RNA
   <213> artificial
<220>
   <223> sequence for preparation of siRNA or shRNA targeting TXNIP expression
<400> 225
   caaugugcag ugagcauguu u 21
<210> 226
   <211> 21
   <212> RNA
   <213> artificial
<220>
   <223> sequence for preparation of siRNA or shRNA targeting TXNIP expression
<400> 226
   acaugcucac ugcacauugu u 21
<210> 227
   <211> 21
   <212> RNA
   <213> artificial
<220>
   <223> sequence for preparation of siRNA or shRNA targeting TXNIP expression
<400> 227
   aaugugcagu gagcaugugg a 21
<210> 228
   <211> 21
   <212> RNA
   <213> artificial
<220>
   <223> sequence for preparation of siRNA or shRNA targeting TXNIP expression
<400> 228
   ugugcaguga gcauguggau u 21
<210> 229
   <211> 21
   <212> RNA
   <213> artificial
<220>
   <223> sequence for preparation of siRNA or shRNA targeting TXNIP expression
<400> 229
   uccacaugcu cacugcacau u 21
<210> 230
   <211> 21
   <212> RNA
   <213> artificial
<220>
   <223> sequence for preparation of siRNA or shRNA targeting TXNIP expression
<400> 230
   aagaagcagc uuuaccuacu u 21
<210> 231
   <211> 21
   <212> RNA
   <213> artificial
<220>
   <223> sequence for preparation of siRNA or shRNA targeting TXNIP expression
<400> 231
   gaagcagcuu uaccuacuuu u 21
<210> 232
   <211> 21
   <212> RNA
   <213> artificial
<220>
   <223> sequence for preparation of siRNA or shRNA targeting TXNIP expression
<400> 232
   aaguagguaa agcugcuucu u 21
<210> 233
   <211> 21
   <212> RNA
   <213> artificial
<220>
   <223> sequence for preparation of siRNA or shRNA targeting TXNIP expression
<400> 233
   aagcagcuuu accuacuugu u 21
<210> 234
   <211> 21
   <212> RNA
   <213> artificial
<220>
   <223> sequence for preparation of siRNA or shRNA targeting TXNIP expression
<400> 234
   gcagcuuuac cuacuuguuu u 21
<210> 235
   <211> 21
   <212> RNA
   <213> artificial
<220>
   <223> sequence for preparation of siRNA or shRNA targeting TXNIP expression
<400> 235
   aacaaguagg uaaagcugcu u 21
<210> 236
   <211> 21
   <212> RNA
   <213> artificial
<220>
   <223> sequence for preparation of siRNA or shRNA targeting TXNIP expression
<400> 236
   aacagucucu gcaauggagu g 21
<210> 237
   <211> 21
   <212> RNA
   <213> artificial
<220>
   <223> sequence for preparation of siRNA or shRNA targeting TXNIP expression
<400> 237
   cagucucugc aauggagugu u 21
<210> 238
   <211> 21
   <212> RNA
   <213> artificial
<220>
   <223> sequence for preparation of siRNA or shRNA targeting TXNIP expression
<400> 238
   cacuccauug cagagacugu u 21
<210> 239
   <211> 21
   <212> RNA
   <213> artificial
<220>
   <223> sequence for preparation of siRNA or shRNA targeting TXNIP expression
<400> 239
   aauggagugu ggguccaccu u 21
<210> 240
   <211> 21
   <212> RNA
   <213> artificial
<220>
   <223> sequence for preparation of siRNA or shRNA targeting TXNIP expression
<400> 240
   uggagugugg guccaccuuu u 21
<210> 241
   <211> 21
   <212> RNA
   <213> artificial
<220>
   <223> sequence for preparation of siRNA or shRNA targeting TXNIP expression
<400> 241
   aagguggacc cacacuccau u 21
<210> 242
   <211> 21
   <212> RNA
   <213> artificial
<220>
   <223> sequence for preparation of siRNA or shRNA targeting TXNIP expression
<400> 242
   aauguaggag guggucagca g 21
<210> 243
   <211> 21
   <212> RNA
   <213> artificial
<220>
   <223> sequence for preparation of siRNA or shRNA targeting TXNIP expression
<400> 243
   uguaggaggu ggucagcagu u 21
<210> 244
   <211> 21
   <212> RNA
   <213> artificial
<220>
   <223> sequence for preparation of siRNA or shRNA targeting TXNIP expression
<400> 244
   cugcugacca ccuccuacau u 21
<210> 245
   <211> 21
   <212> RNA
   <213> artificial
<220>
   <223> sequence for preparation of siRNA or shRNA targeting TXNIP expression
<400> 245
   aaucuccugg gccuuaaagg a 21
<210> 246
   <211> 21
   <212> RNA
   <213> artificial
<220>
   <223> sequence for preparation of siRNA or shRNA targeting TXNIP expression
<400> 246
   ucuccugggc cuuaaaggau u 21
<210> 247
   <211> 21
   <212> RNA
   <213> artificial
<220>
   <223> sequence for preparation of siRNA or shRNA targeting TXNIP expression
<400> 247
   uccuuuaagg cccaggagau u 21
<210> 248
   <211> 21
   <212> RNA
   <213> artificial
<220>
   <223> sequence for preparation of siRNA or shRNA targeting TXNIP expression
<400> 248
   aaaggaugcg gacucauccu c 21
<210> 249
   <211> 21
   <212> RNA
   <213> artificial
<220>
   <223> sequence for preparation of siRNA or shRNA targeting TXNIP expression
<400> 249
   aggaugcgga cucauccucu u 21
<210> 250
   <211> 21
   <212> RNA
   <213> artificial
<220>
   <223> sequence for preparation of siRNA or shRNA targeting TXNIP expression
<400> 250
   gaggaugagu ccgcauccuu u 21
<210> 251
   <211> 21
   <212> RNA
   <213> artificial
<220>
   <223> sequence for preparation of siRNA or shRNA targeting TXNIP expression
<400> 251
   aaacucaggc ccauccauuu u 21
<210> 252
   <211> 21
   <212> RNA
   <213> artificial
<220>
   <223> sequence for preparation of siRNA or shRNA targeting TXNIP expression
<400> 252
   acucaggccc auccauuuuu u 21
<210> 253
   <211> 21
   <212> RNA
   <213> artificial
<220>
   <223> sequence for preparation of siRNA or shRNA targeting TXNIP expression
<400> 253
   aaaauggaug ggccugaguu u 21
<210> 254
   <211> 21
   <212> RNA
   <213> artificial
<220>
   <223> sequence for preparation of siRNA or shRNA targeting TXNIP expression
<400> 254
   aauugaggcc uuuucgauag u 21
<210> 255
   <211> 21
   <212> RNA
   <213> artificial
<220>
   <223> sequence for preparation of siRNA or shRNA targeting TXNIP expression
<400> 255
   uugaggccuu uucgauaguu u 21
<210> 256
   <211> 21
   <212> RNA
   <213> artificial
<220>
   <223> sequence for preparation of siRNA or shRNA targeting TXNIP expression
<400> 256
   acuaucgaaa aggccucaau u 21
<210> 257
   <211> 21
   <212> RNA
   <213> artificial
<220>
   <223> sequence for preparation of siRNA or shRNA targeting TXNIP expression
<400> 257
   aaauggccuc cuggcguaag c 21
<210> 258
   <211> 21
   <212> RNA
   <213> artificial
<220>
   <223> sequence for preparation of siRNA or shRNA targeting TXNIP expression
<400> 258
   auggccuccu ggcguaagcu u 21
<210> 259
   <211> 21
   <212> RNA
   <213> artificial
<220>
   <223> sequence for preparation of siRNA or shRNA targeting TXNIP expression
<400> 259
   gcuuacgcca ggaggccauu u 21
<210> 260
   <211> 21
   <212> RNA
   <213> artificial
<220>
   <223> sequence for preparation of siRNA or shRNA targeting TXNIP expression
<400> 260
   aagcuuuuca agguuuuuug g 21
<210> 261
   <211> 21
   <212> RNA
   <213> artificial
<220>
   <223> sequence for preparation of siRNA or shRNA targeting TXNIP expression
<400> 261
   gcuuuucaag guuuuuuggu u 21
<210> 262
   <211> 21
   <212> RNA
   <213> artificial
<220>
   <223> sequence for preparation of siRNA or shRNA targeting TXNIP expression
<400> 262
   ccaaaaaacc uugaaaagcu u 21
<210> 263
   <211> 21
   <212> RNA
   <213> artificial
<220>
   <223> sequence for preparation of siRNA or shRNA targeting TXNIP expression
<400> 263
   aagguuuuuu ggaggcuuuu u 21
<210> 264
   <211> 21
   <212> RNA
   <213> artificial
<220>
   <223> sequence for preparation of siRNA or shRNA targeting TXNIP expression
<400> 264
   gguuuuuugg aggcuuuuuu u 21
<210> 265
   <211> 21
   <212> RNA
   <213> artificial
<220>
   <223> sequence for preparation of siRNA or shRNA targeting TXNIP expression
<400> 265
   aaaaagccuc caaaaaaccu u 21
<210> 266
   <211> 21
   <212> RNA
   <213> artificial
<220>
   <223> sequence for preparation of siRNA or shRNA targeting TXNIP expression
<400> 266
   aaauugugau aggaacuuug g 21
<210> 267
   <211> 21
   <212> RNA
   <213> artificial
<220>
   <223> sequence for preparation of siRNA or shRNA targeting TXNIP expression
<400> 267
   auugugauag gaacuuuggu u 21
<210> 268
   <211> 21
   <212> RNA
   <213> artificial
<220>
   <223> sequence for preparation of siRNA or shRNA targeting TXNIP expression
<400> 268
   ccaaaguucc uaucacaauu u 21
<210> 269
   <211> 21
   <212> RNA
   <213> artificial
<220>
   <223> sequence for preparation of siRNA or shRNA targeting TXNIP expression
<400> 269
   aacuuuggac cuugaacuua u 21
<210> 270
   <211> 21
   <212> RNA
   <213> artificial
<220>
   <223> sequence for preparation of siRNA or shRNA targeting TXNIP expression
<400> 270
   cuuuggaccu ugaacuuauu u 21
<210> 271
   <211> 21
   <212> RNA
   <213> artificial
<220>
   <223> sequence for preparation of siRNA or shRNA targeting TXNIP expression
<400> 271
   auaaguucaa gguccaaagu u 21
<210> 272
   <211> 21
   <212> RNA
   <213> artificial
<220>
   <223> sequence for preparation of siRNA or shRNA targeting TXNIP expression
<400> 272
   aacuuaugua ucauguggag a 21
<210> 273
   <211> 21
   <212> RNA
   <213> artificial
<220>
   <223> sequence for preparation of siRNA or shRNA targeting TXNIP expression
<400> 273
   cuuauguauc auguggagau u 21
<210> 274
   <211> 21
   <212> RNA
   <213> artificial
<220>
   <223> sequence for preparation of siRNA or shRNA targeting TXNIP expression
<400> 274
   ucuccacaug auacauaagu u 21
<210> 275
   <211> 21
   <212> RNA
   <213> artificial
<220>
   <223> sequence for preparation of siRNA or shRNA targeting TXNIP expression
<400> 275
   aagagccaau uuaacaaacu a 21
<210> 276
   <211> 21
   <212> RNA
   <213> artificial
<220>
   <223> sequence for preparation of siRNA or shRNA targeting TXNIP expression
<400> 276
   gagccaauuu aacaaacuau u 21
<210> 277
   <211> 21
   <212> RNA
   <213> artificial
<220>
   <223> sequence for preparation of siRNA or shRNA targeting TXNIP expression
<400> 277
   uaguuuguua aauuggcucu u 21
<210> 278
   <211> 21
   <212> RNA
   <213> artificial
<220>
   <223> sequence for preparation of siRNA or shRNA targeting TXNIP expression
<400> 278
   aauuuaacaa acuaggaaga u 21
<210> 279
   <211> 21
   <212> RNA
   <213> artificial
<220>
   <223> sequence for preparation of siRNA or shRNA targeting TXNIP expression
<400> 279
   uuuaacaaac uaggaagauu u 21
<210> 280
   <211> 21
   <212> RNA
   <213> artificial
<220>
   <223> sequence for preparation of siRNA or shRNA targeting TXNIP expression
<400> 280
   aucuuccuag uuuguuaaau u 21
<210> 281
   <211> 21
   <212> RNA
   <213> artificial
<220>
   <223> sequence for preparation of siRNA or shRNA targeting TXNIP expression
<400> 281
   aaucaguguu uccccuuugu g 21
<210> 282
   <211> 21
   <212> RNA
   <213> artificial
<220>
   <223> sequence for preparation of siRNA or shRNA targeting TXNIP expression
<400> 282
   ucaguguuuc cccuuugugu u 21
<210> 283
   <211> 21
   <212> RNA
   <213> artificial
<220>
   <223> sequence for preparation of siRNA or shRNA targeting TXNIP expression
<400> 283
   cacaaagggg aaacacugau u 21
<210> 284
   <211> 21
   <212> RNA
   <213> artificial
<220>
   <223> sequence for preparation of siRNA or shRNA targeting TXNIP expression
<400> 284
   aaaccuucua gagcugauuu g 21
<210> 285
   <211> 21
   <212> RNA
   <213> artificial
<220>
   <223> sequence for preparation of siRNA or shRNA targeting TXNIP expression
<400> 285
   accuucuaga gcugauuugu u 21
<210> 286
   <211> 21
   <212> RNA
   <213> artificial
<220>
   <223> sequence for preparation of siRNA or shRNA targeting TXNIP expression
<400> 286
   caaaucagcu cuagaagguu u 21
<210> 287
   <211> 21
   <212> RNA
   <213> artificial
<220>
   <223> sequence for preparation of siRNA or shRNA targeting TXNIP expression
<400> 287
   aauggagaga gcuuucccug u 21
<210> 288
   <211> 21
   <212> RNA
   <213> artificial
<220>
   <223> sequence for preparation of siRNA or shRNA targeting TXNIP expression
<400> 288
   uggagagagc uuucccuguu u 21
<210> 289
   <211> 21
   <212> RNA
   <213> artificial
<220>
   <223> sequence for preparation of siRNA or shRNA targeting TXNIP expression
<400> 289
   acagggaaag cucucuccau u 21
<210> 290
   <211> 21
   <212> RNA
   <213> artificial
<220>
   <223> sequence for preparation of siRNA or shRNA targeting TXNIP expression
<400> 290
   aaggaagcua gcugcucuac g 21
<210> 291
   <211> 21
   <212> RNA
   <213> artificial
<220>
   <223> sequence for preparation of siRNA or shRNA targeting TXNIP expression
<400> 291
   ggaagcuagc ugcucuacgu u 21
<210> 292
   <211> 21
   <212> RNA
   <213> artificial
<220>
   <223> sequence for preparation of siRNA or shRNA targeting TXNIP expression
<400> 292
   cguagagcag cuagcuuccu u 21
<210> 293
   <211> 21
   <212> RNA
   <213> artificial
<220>
   <223> sequence for preparation of siRNA or shRNA targeting TXNIP expression
<400> 293
   aagcuagcug cucuacgguc a 21
<210> 294
   <211> 21
   <212> RNA
   <213> artificial
<220>
   <223> sequence for preparation of siRNA or shRNA targeting TXNIP expression
<400> 294
   gcuagcugcu cuacggucau u 21
<210> 295
   <211> 21
   <212> RNA
   <213> artificial
<220>
   <223> sequence for preparation of siRNA or shRNA targeting TXNIP expression
<400> 295
   ugaccguaga gcagcuagcu u 21
<210> 296
   <211> 21
   <212> RNA
   <213> artificial
<220>
   <223> sequence for preparation of siRNA or shRNA targeting TXNIP expression
<400> 296
   aagaguauac uuuaaccugg c 21
<210> 297
   <211> 21
   <212> RNA
   <213> artificial
<220>
   <223> sequence for preparation of siRNA or shRNA targeting TXNIP expression
<400> 297
   gaguauacuu uaaccuggcu u 21
<210> 298
   <211> 21
   <212> RNA
   <213> artificial
<220>
   <223> sequence for preparation of siRNA or shRNA targeting TXNIP expression
<400> 298
   gccagguuaa aguauacucu u 21
<210> 299
   <211> 21
   <212> RNA
   <213> artificial
<220>
   <223> sequence for preparation of siRNA or shRNA targeting TXNIP expression
<400> 299
   aaccuggcuu uuaaagcagu a 21
<210> 300
   <211> 21
   <212> RNA
   <213> artificial
<220>
   <223> sequence for preparation of siRNA or shRNA targeting TXNIP expression
<400> 300
   ccuggcuuuu aaagcaguau u 21
<210> 301
   <211> 21
   <212> RNA
   <213> artificial
<220>
   <223> sequence for preparation of siRNA or shRNA targeting TXNIP expression
<400> 301
   uacugcuuua aaagccaggu u 21
<210> 302
   <211> 21
   <212> RNA
   <213> artificial
<220>
   <223> sequence for preparation of siRNA or shRNA targeting TXNIP expression
<400> 302
   aaagcaguag uaacugcccc a 21
<210> 303
   <211> 21
   <212> RNA
   <213> artificial
<220>
   <223> sequence for preparation of siRNA or shRNA targeting TXNIP expression
<400> 303
   agcaguagua acugccccau u 21
<210> 304
   <211> 21
   <212> RNA
   <213> artificial
<220>
   <223> sequence for preparation of siRNA or shRNA targeting TXNIP expression
<400> 304
   uggggcaguu acuacugcuu u 21
<210> 305
   <211> 21
   <212> RNA
   <213> artificial
<220>
   <223> sequence for preparation of siRNA or shRNA targeting TXNIP expression
<400> 305
   aacugcccca ccaaaggucu u 21
<210> 306
   <211> 21
   <212> RNA
   <213> artificial
<220>
   <223> sequence for preparation of siRNA or shRNA targeting TXNIP expression
<400> 306
   cugccccacc aaaggucuuu u 21
<210> 307
   <211> 21
   <212> RNA
   <213> artificial
<220>
   <223> sequence for preparation of siRNA or shRNA targeting TXNIP expression
<400> 307
   aagaccuuug guggggcagu u 21
<210> 308
   <211> 21
   <212> RNA
   <213> artificial
<220>
   <223> sequence for preparation of siRNA or shRNA targeting TXNIP expression
<400> 308
   aagccauuuu uggagccuau u 21
<210> 309
   <211> 21
   <212> RNA
   <213> artificial
<220>
   <223> sequence for preparation of siRNA or shRNA targeting TXNIP expression
<400> 309
   gccauuuuug gagccuauuu u 21
<210> 310
   <211> 21
   <212> RNA
   <213> artificial
<220>
   <223> sequence for preparation of siRNA or shRNA targeting TXNIP expression
<400> 310
   aauaggcucc aaaaauggcu u 21
<210> 311
   <211> 21
   <212> RNA
   <213> artificial
<220>
   <223> sequence for preparation of siRNA or shRNA targeting TXNIP expression
<400> 311
   aaauauuuuc auaugggagg a 21
<210> 312
   <211> 21
   <212> RNA
   <213> artificial
<220>
   <223> sequence for preparation of siRNA or shRNA targeting TXNIP expression
<400> 312
   auauuuucau augggaggau u 21
<210> 313
   <211> 21
   <212> RNA
   <213> artificial
<220>
   <223> sequence for preparation of siRNA or shRNA targeting TXNIP expression
<400> 313
   uccucccaua ugaaaauauu u 21
<210> 314
   <211> 21
   <212> RNA
   <213> artificial
<220>
   <223> sequence for preparation of siRNA or shRNA targeting TXNIP expression
<400> 314
   aaguccuugg aauugauucu a 21
<210> 315
   <211> 21
   <212> RNA
   <213> artificial
<220>
   <223> sequence for preparation of siRNA or shRNA targeting TXNIP expression
<400> 315
   guccuuggaa uugauucuau u 21
<210> 316
   <211> 21
   <212> RNA
   <213> artificial
<220>
   <223> sequence for preparation of siRNA or shRNA targeting TXNIP expression
<400> 316
   uagaaucaau uccaaggacu u 21
<210> 317
   <211> 21
   <212> RNA
   <213> artificial
<220>
   <223> sequence for preparation of siRNA or shRNA targeting TXNIP expression
<400> 317
   aauugauucu aaggugaugu u 21
<210> 318
   <211> 21
   <212> RNA
   <213> artificial
<220>
   <223> sequence for preparation of siRNA or shRNA targeting TXNIP expression
<400> 318
   uugauucuaa ggugauguuu u 21
<210> 319
   <211> 21
   <212> RNA
   <213> artificial
<220>
   <223> sequence for preparation of siRNA or shRNA targeting TXNIP expression
<400> 319
   aacaucaccu uagaaucaau u 21
<210> 320
   <211> 21
   <212> RNA
   <213> artificial
<220>
   <223> sequence for preparation of siRNA or shRNA targeting TXNIP expression
<400> 320
   aaggugaugu ucuuagcacu u 21
<210> 321
   <211> 21
   <212> RNA
   <213> artificial
<220>
   <223> sequence for preparation of siRNA or shRNA targeting TXNIP expression
<400> 321
   ggugauguuc uuagcacuuu u 21
<210> 322
   <211> 21
   <212> RNA
   <213> artificial
<220>
   <223> sequence for preparation of siRNA or shRNA targeting TXNIP expression
<400> 322
   aagugcuaag aacaucaccu u 21
<210> 323
   <211> 21
   <212> RNA
   <213> artificial
<220>
   <223> sequence for preparation of siRNA or shRNA targeting TXNIP expression
<400> 323
   aauuccuguc aaauuuuuug u 21
<210> 324
   <211> 21
   <212> RNA
   <213> artificial
<220>
   <223> sequence for preparation of siRNA or shRNA targeting TXNIP expression
<400> 324
   uuccugucaa auuuuuuguu u 21
<210> 325
   <211> 21
   <212> RNA
   <213> artificial
<220>
   <223> sequence for preparation of siRNA or shRNA targeting TXNIP expression
<400> 325
   acaaaaaauu ugacaggaau u 21
<210> 326
   <211> 21
   <212> RNA
   <213> artificial
<220>
   <223> sequence for preparation of siRNA or shRNA targeting TXNIP expression
<400> 326
   aaauuuuuug uucuccccuu c 21
<210> 327
   <211> 21
   <212> RNA
   <213> artificial
<220>
   <223> sequence for preparation of siRNA or shRNA targeting TXNIP expression
<400> 327
   auuuuuuguu cuccccuucu u 21
<210> 328
   <211> 21
   <212> RNA
   <213> artificial
<220>
   <223> sequence for preparation of siRNA or shRNA targeting TXNIP expression
<400> 328
   gaaggggaga acaaaaaauu u 21
<210> 329
   <211> 21
   <212> RNA
   <213> artificial
<220>
   <223> sequence for preparation of siRNA or shRNA targeting TXNIP expression
<400> 329
   aaauguaagc ugaaacuggu c 21
<210> 330
   <211> 21
   <212> RNA
   <213> artificial
<220>
   <223> sequence for preparation of siRNA or shRNA targeting TXNIP expression
<400> 330
   auguaagcug aaacuggucu u 21
<210> 331
   <211> 21
   <212> RNA
   <213> artificial
<220>
   <223> sequence for preparation of siRNA or shRNA targeting TXNIP expression
<400> 331
   gaccaguuuc agcuuacauu u 21
<210> 332
   <211> 21
   <212> RNA
   <213> artificial
<220>
   <223> sequence for preparation of siRNA or shRNA targeting TXNIP expression
<400> 332
   aagcugaaac uggucuacug u 21
<210> 333
   <211> 21
   <212> RNA
   <213> artificial
<220>
   <223> sequence for preparation of siRNA or shRNA targeting TXNIP expression
<400> 333
   gcugaaacug gucuacuguu u 21
<210> 334
   <211> 21
   <212> RNA
   <213> artificial
<220>
   <223> sequence for preparation of siRNA or shRNA targeting TXNIP expression
<400> 334
   acaguagacc aguuucagcu u 21
<210> 335
   <211> 21
   <212> RNA
   <213> artificial
<220>
   <223> sequence for preparation of siRNA or shRNA targeting TXNIP expression
<400> 335
   aaacuggucu acugugucuc u 21
<210> 336
   <211> 21
   <212> RNA
   <213> artificial
<220>
   <223> sequence for preparation of siRNA or shRNA targeting TXNIP expression
<400> 336
   acuggucuac ugugucucuu u 21
<210> 337
   <211> 21
   <212> RNA
   <213> artificial
<220>
   <223> sequence for preparation of siRNA or shRNA targeting TXNIP expression
<400> 337
   agagacacag uagaccaguu u 21
<210> 338
   <211> 21
   <212> RNA
   <213> artificial
<220>
   <223> sequence for preparation of siRNA or shRNA targeting TXNIP expression
<400> 338
   aauuuuacua cuuuugagau u 21
<210> 339
   <211> 21
   <212> RNA
   <213> artificial
<220>
   <223> sequence for preparation of siRNA or shRNA targeting TXNIP expression
<400> 339
   uuuuacuacu uuugagauuu u 21
<210> 340
   <211> 21
   <212> RNA
   <213> artificial
<220>
   <223> sequence for preparation of siRNA or shRNA targeting TXNIP expression
<400> 340
   aaucucaaaa guaguaaaau u 21
<210> 341
   <211> 21
   <212> RNA
   <213> artificial
<220>
   <223> sequence for preparation of siRNA or shRNA targeting TXNIP expression
<400> 341
   aauguacaga auuauauaau u 21
<210> 342
   <211> 21
   <212> RNA
   <213> artificial
<220>
   <223> sequence for preparation of siRNA or shRNA targeting TXNIP expression
<400> 342
   uguacagaau uauauaauuu u 21
<210> 343
   <211> 21
   <212> RNA
   <213> artificial
<220>
   <223> sequence for preparation of siRNA or shRNA targeting TXNIP expression
<400> 343
   aauuauauaa uucuguacau u 21
<210> 344
   <211> 21
   <212> RNA
   <213> artificial
<220>
   <223> sequence for preparation of siRNA or shRNA targeting TXNIP expression
<400> 344
   aauuauauaa uucuaacgcu u 21
<210> 345
   <211> 21
   <212> RNA
   <213> artificial
<220>
   <223> sequence for preparation of siRNA or shRNA targeting TXNIP expression
<400> 345
   uuauauaauu cuaacgcuuu u 21
<210> 346
   <211> 21
   <212> RNA
   <213> artificial
<220>
   <223> sequence for preparation of siRNA or shRNA targeting TXNIP expression
<400> 346
   aagcguuaga auuauauaau u 21
<210> 347
   <211> 21
   <212> RNA
   <213> artificial
<220>
   <223> sequence for preparation of siRNA or shRNA targeting TXNIP expression
<400> 347
   aauucuaacg cuuaaaucau g 21
<210> 348
   <211> 21
   <212> RNA
   <213> artificial
<220>
   <223> sequence for preparation of siRNA or shRNA targeting TXNIP expression
<400> 348
   uucuaacgcu uaaaucaugu u 21
<210> 349
   <211> 21
   <212> RNA
   <213> artificial
<220>
   <223> sequence for preparation of siRNA or shRNA targeting TXNIP expression
<400> 349
   caugauuuaa gcguuagaau u 21
<210> 350
   <211> 21
   <212> RNA
   <213> artificial
<220>
   <223> sequence for preparation of siRNA or shRNA targeting TXNIP expression
<400> 350
   aacgcuuaaa ucaugugaaa g 21
<210> 351
   <211> 21
   <212> RNA
   <213> artificial
<220>
   <223> sequence for preparation of siRNA or shRNA targeting TXNIP expression
<400> 351
   cgcuuaaauc augugaaagu u 21
<210> 352
   <211> 21
   <212> RNA
   <213> artificial
<220>
   <223> sequence for preparation of siRNA or shRNA targeting TXNIP expression
<400> 352
   cuuucacaug auuuaagcgu u 21
<210> 353
   <211> 21
   <212> RNA
   <213> artificial
<220>
   <223> sequence for preparation of siRNA or shRNA targeting TXNIP expression
<400> 353
   aaaucaugug aaaggguugc u 21
<210> 354
   <211> 21
   <212> RNA
   <213> artificial
<220>
   <223> sequence for preparation of siRNA or shRNA targeting TXNIP expression
<400> 354
   aucaugugaa aggguugcuu u 21
<210> 355
   <211> 21
   <212> RNA
   <213> artificial
<220>
   <223> sequence for preparation of siRNA or shRNA targeting TXNIP expression
<400> 355
   agcaacccuu ucacaugauu u 21
<210> 356
   <211> 21
   <212> RNA
   <213> artificial
<220>
   <223> sequence for preparation of siRNA or shRNA targeting TXNIP expression
<400> 356
   aaaggguugc ugcugucagc c 21
<210> 357
   <211> 21
   <212> RNA
   <213> artificial
<220>
   <223> sequence for preparation of siRNA or shRNA targeting TXNIP expression
<400> 357
   aggguugcug cugucagccu u 21
<210> 358
   <211> 21
   <212> RNA
   <213> artificial
<220>
   <223> sequence for preparation of siRNA or shRNA targeting TXNIP expression
<400> 358
   ggcugacagc agcaacccuu u 21
<210> 359
   <211> 21
   <212> RNA
   <213> artificial
<220>
   <223> sequence for preparation of siRNA or shRNA targeting TXNIP expression
<400> 359
   aaacccaagg aggaacucuu g 21
<210> 360
   <211> 21
   <212> RNA
   <213> artificial
<220>
   <223> sequence for preparation of siRNA or shRNA targeting TXNIP expression
<400> 360
   acccaaggag gaacucuugu u 21
<210> 361
   <211> 21
   <212> RNA
   <213> artificial
<220>
   <223> sequence for preparation of siRNA or shRNA targeting TXNIP expression
<400> 361
   caagaguucc uccuuggguu u 21
<210> 362
   <211> 21
   <212> RNA
   <213> artificial
<220>
   <223> sequence for preparation of siRNA or shRNA targeting TXNIP expression
<400> 362
   aaggaggaac ucuugaucaa g 21
<210> 363
   <211> 21
   <212> RNA
   <213> artificial
<220>
   <223> sequence for preparation of siRNA or shRNA targeting TXNIP expression
<400> 363
   ggaggaacuc uugaucaagu u 21
<210> 364
   <211> 21
   <212> RNA
   <213> artificial
<220>
   <223> sequence for preparation of siRNA or shRNA targeting TXNIP expression
<400> 364
   cuugaucaag aguuccuccu u 21
<210> 365
   <211> 21
   <212> RNA
   <213> artificial
<220>
   <223> sequence for preparation of siRNA or shRNA targeting TXNIP expression
<400> 365
   aacucuugau caagaugccg a 21
<210> 366
   <211> 21
   <212> RNA
   <213> artificial
<220>
   <223> sequence for preparation of siRNA or shRNA targeting TXNIP expression
<400> 366
   cucuugauca agaugccgau u 21
<210> 367
   <211> 21
   <212> RNA
   <213> artificial
<220>
   <223> sequence for preparation of siRNA or shRNA targeting TXNIP expression
<400> 367
   ucggcaucuu gaucaagagu u 21
<210> 368
   <211> 21
   <212> RNA
   <213> artificial
<220>
   <223> sequence for preparation of siRNA or shRNA targeting TXNIP expression
<400> 368
   aagaugccga acccuguguu c 21
<210> 369
   <211> 21
   <212> RNA
   <213> artificial
<220>
   <223> sequence for preparation of siRNA or shRNA targeting TXNIP expression
<400> 369
   gaugccgaac ccuguguucu u 21
<210> 370
   <211> 21
   <212> RNA
   <213> artificial
<220>
   <223> sequence for preparation of siRNA or shRNA targeting TXNIP expression
<400> 370
   gaacacaggg uucggcaucu u 21
<210> 371
   <211> 21
   <212> RNA
   <213> artificial
<220>
   <223> sequence for preparation of siRNA or shRNA targeting TXNIP expression
<400> 371
   aacccugugu ucagaaccuc c 21
<210> 372
   <211> 21
   <212> RNA
   <213> artificial
<220>
   <223> sequence for preparation of siRNA or shRNA targeting TXNIP expression
<400> 372
   cccuguguuc agaaccuccu u 21
<210> 373
   <211> 21
   <212> RNA
   <213> artificial
<220>
   <223> sequence for preparation of siRNA or shRNA targeting TXNIP expression
<400> 373
   ggagguucug aacacagggu u 21
<210> 374
   <211> 21
   <212> RNA
   <213> artificial
<220>
   <223> sequence for preparation of siRNA or shRNA targeting TXNIP expression
<400> 374
   aaccuccaaa uacugccaug a 21
<210> 375
   <211> 21
   <212> RNA
   <213> artificial
<220>
   <223> sequence for preparation of siRNA or shRNA targeting TXNIP expression
<400> 375
   ccuccaaaua cugccaugau u 21
<210> 376
   <211> 21
   <212> RNA
   <213> artificial
<220>
   <223> sequence for preparation of siRNA or shRNA targeting TXNIP expression
<400> 376
   ucauggcagu auuuggaggu u 21
<210> 377
   <211> 21
   <212> RNA
   <213> artificial
<220>
   <223> sequence for preparation of siRNA or shRNA targeting TXNIP expression
<400> 377
   aaauacugcc augagaaacu a 21
<210> 378
   <211> 21
   <212> RNA
   <213> artificial
<220>
   <223> sequence for preparation of siRNA or shRNA targeting TXNIP expression
<400> 378
   auacugccau gagaaacuau u 21
<210> 379
   <211> 21
   <212> RNA
   <213> artificial
<220>
   <223> sequence for preparation of siRNA or shRNA targeting TXNIP expression
<400> 379
   uaguuucuca uggcaguauu u 21
<210> 380
   <211> 21
   <212> RNA
   <213> artificial
<220>
   <223> sequence for preparation of siRNA or shRNA targeting TXNIP expression
<400> 380
   aaacuagagg gcaggucuuc a 21
<210> 381
   <211> 21
   <212> RNA
   <213> artificial
<220>
   <223> sequence for preparation of siRNA or shRNA targeting TXNIP expression
<400> 381
   acuagagggc aggucuucau u 21
<210> 382
   <211> 21
   <212> RNA
   <213> artificial
<220>
   <223> sequence for preparation of siRNA or shRNA targeting TXNIP expression
<400> 382
   ugaagaccug cccucuaguu u 21
<210> 383
   <211> 21
   <212> RNA
   <213> artificial
<220>
   <223> sequence for preparation of siRNA or shRNA targeting TXNIP expression
<400> 383
   aagcccuuug aacccccuuc c 21
<210> 384
   <211> 21
   <212> RNA
   <213> artificial
<220>
   <223> sequence for preparation of siRNA or shRNA targeting TXNIP expression
<400> 384
   gcccuuugaa cccccuuccu u 21
<210> 385
   <211> 21
   <212> RNA
   <213> artificial
<220>
   <223> sequence for preparation of siRNA or shRNA targeting TXNIP expression
<400> 385
   ggaagggggu ucaaagggcu u 21
<210> 386
   <211> 21
   <212> RNA
   <213> artificial
<220>
   <223> sequence for preparation of siRNA or shRNA targeting TXNIP expression
<400> 386
   aacccccuuc cugcccugug u 21
<210> 387
   <211> 21
   <212> RNA
   <213> artificial
<220>
   <223> sequence for preparation of siRNA or shRNA targeting TXNIP expression
<400> 387
   cccccuuccu gcccuguguu u 21
<210> 388
   <211> 21
   <212> RNA
   <213> artificial
<220>
   <223> sequence for preparation of siRNA or shRNA targeting TXNIP expression
<400> 388
   acacagggca ggaagggggu u 21
<210> 389
   <211> 21
   <212> RNA
   <213> artificial
<220>
   <223> sequence for preparation of siRNA or shRNA targeting TXNIP expression
<400> 389
   aaaguguggg cugaagaugg u 21
<210> 390
   <211> 21
   <212> RNA
   <213> artificial
<220>
   <223> sequence for preparation of siRNA or shRNA targeting TXNIP expression
<400> 390
   agugugggcu gaagaugguu u 21
<210> 391
   <211> 21
   <212> RNA
   <213> artificial
<220>
   <223> sequence for preparation of siRNA or shRNA targeting TXNIP expression
<400> 391
   accaucuuca gcccacacuu u 21
<210> 392
   <211> 21
   <212> RNA
   <213> artificial
<220>
   <223> sequence for preparation of siRNA or shRNA targeting TXNIP expression
<400> 392
   aagaugguug guuucauguu u 21
<210> 393
   <211> 21
   <212> RNA
   <213> artificial
<220>
   <223> sequence for preparation of siRNA or shRNA targeting TXNIP expression
<400> 393
   gaugguuggu uucauguuuu u 21
<210> 394
   <211> 21
   <212> RNA
   <213> artificial
<220>
   <223> sequence for preparation of siRNA or shRNA targeting TXNIP expression
<400> 394
   aaacaugaaa ccaaccaucu u 21
<210> 395
   <211> 21
   <212> RNA
   <213> artificial
<220>
   <223> sequence for preparation of siRNA or shRNA targeting TXNIP expression
<400> 395
   aaagacuaua uuuuguacuu a 21
<210> 396
   <211> 21
   <212> RNA
   <213> artificial
<220>
   <223> sequence for preparation of siRNA or shRNA targeting TXNIP expression
<400> 396
   agacuauauu uuguacuuau u 21
<210> 397
   <211> 21
   <212> RNA
   <213> artificial
<220>
   <223> sequence for preparation of siRNA or shRNA targeting TXNIP expression
<400> 397
   uaaguacaaa auauagucuu u 21
<210> 398
   <211> 21
   <212> RNA
   <213> artificial
<220>
   <223> sequence for preparation of siRNA or shRNA targeting TXNIP expression
<400> 398
   aaccagauau auuuuuaccc c 21
<210> 399
   <211> 21
   <212> RNA
   <213> artificial
<220>
   <223> sequence for preparation of siRNA or shRNA targeting TXNIP expression
<400> 399
   ccagauauau uuuuaccccu u 21
<210> 400
   <211> 21
   <212> RNA
   <213> artificial
<220>
   <223> sequence for preparation of siRNA or shRNA targeting TXNIP expression
<400> 400
   gggguaaaaa uauaucuggu u 21
<210> 401
   <211> 21
   <212> RNA
   <213> artificial
<220>
   <223> sequence for preparation of siRNA or shRNA targeting TXNIP expression
<400> 401
   aauaaaguuu uuuuaaugga a 21
<210> 402
   <211> 21
   <212> RNA
   <213> artificial
<220>
   <223> sequence for preparation of siRNA or shRNA targeting TXNIP expression
<400> 402
   uaaaguuuuu uuaauggaau u 21
<210> 403
   <211> 21
   <212> RNA
   <213> artificial
<220>
   <223> sequence for preparation of siRNA or shRNA targeting TXNIP expression
<400> 403
   uuccauuaaa aaaacuuuau u 21
<210> 404
   <211> 3
   <212> DNA
   <213> artificial
<220>
   <223> sequence for preparation of siRNA or shRNA targeting TXNIP expression
<400> 404
   ccc 3
<210> 405
   <211> 4
   <212> DNA
   <213> artificial
<220>
   <223> sequence for preparation of siRNA or shRNA targeting TXNIP expression
<400> 405
   ttcg 4
<210> 406
   <211> 5
   <212> DNA
   <213> artificial
<220>
   <223> sequence for preparation of siRNA or shRNA targeting TXNIP expression
<400> 406
   ccacc 5
<210> 407
   <211> 6
   <212> DNA
   <213> artificial
<220>
   <223> sequence for preparation of siRNA or shRNA targeting TXNIP expression
<400> 407
   ctcgag 6
<210> 408
   <211> 6
   <212> DNA
   <213> artificial
<220>
   <223> sequence for preparation of siRNA or shRNA targeting TXNIP expression
<400> 408
   aagctt 6
<210> 409
   <211> 7
   <212> DNA
   <213> artificial
<220>
   <223> sequence for preparation of siRNA or shRNA targeting TXNIP expression
<400> 409
   ccacacc 7
<210> 410
   <211> 9
   <212> DNA
   <213> artificial
<220>
   <223> sequence for preparation of siRNA or shRNA targeting TXNIP expression
<400> 410
   ttcaagaga 9
<210> 411
   <211> 9
   <212> DNA
   <213> artificial
<220>
   <223> sequence for preparation of siRNA or shRNA targeting TXNIP expression
<400> 411
   aagttctct 9
<210> 412
   <211> 9
   <212> DNA
   <213> artificial
<220>
   <223> sequence for preparation of siRNA or shRNA targeting TXNIP expression
<400> 412
   tttgtgtag 9
<210> 413
   <211> 10
   <212> DNA
   <213> artificial
<220>
   <223> sequence for preparation of siRNA or shRNA targeting TXNIP expression
<400> 413
   cttcctgtca 10
<210> 414
   <211> 13
   <212> DNA
   <213> artificial
<220>
   <223> sequence for preparation of siRNA or shRNA targeting TXNIP expression
<400> 414
   cttgatatcc gag 13
<210> 415
   <211> 2801
   <212> DNA
   <213> Mus musculus
<400> 415
   gacactctcc tcctctggtc tcggggtttc cagagtttct ccagttgcgg aagacagctg      60
   ttatttttct cctgaaagct tttggcacag ccggcaggct gaaacttcca ggcacctttt     120
   ggaaaagttg ttagggtttg tttgaagctt tctttacatt ttcgtttggg ttttcaagcc     180
   ctgactttac ggaggcgagc tcttcgtttg ctttgaaggg ttcttaaaga tttttttcct    240
   ctccggcttt cgtttttctt gaacccactc ggctcaatca tggtgatgtt caagaagatc     300
   aagtcttttg aggtggtctt caacgacccc gagaaggtgt acggcagcgg ggagaaggtg     360
   gccggacggg taatagtgga agtgtgtgaa gttacccgag tcaaagccgt caggatcctg     420
   gcttgcggcg tggccaaggt cctgtggatg caagggtctc agcagtgcaa acagactttg     480
   gactacttgc gctatgaaga cacacttctc ctagaagagc agcctacagc aggtgagaac     540
   gagatggtga tcatgaggcc tggaaacaaa tatgagtaca agttcggctt cgagcttcct     600
   caagggcccc tgggaacatc ctttaaagga aaatatggtt gcgtagacta ctgggtgaag     660
   gcttttctcg atcgccccag ccagccaact caagaggcaa agaaaaactt cgaagtgatg     720
   gatctagtgg atgtcaatac ccctgaccta atggcaccag tgtctgccaa aaaggagaag     780
   aaagtttcct gcatgttcat tcctgatgga cgtgtgtcag tctctgctcg aattgacaga     840
   aaaggattct gtgaaggtga tgacatctcc atccatgctg actttgagaa cacgtgttcc     900
   cgaatcgtgg tccccaaagc ggctattgtg gcccgacaca cttaccttgc caatggccag     960
   accaaagtgt tcactcagaa gctgtcctca gtcagaggca atcacattat ctcagggact    1020
   tgcgcatcgt ggcgtggcaa gagcctcaga gtgcagaaga tcagaccatc catcctgggc    1080
   tgcaacatcc tcaaagtcga atactccttg ctgatctacg tcagtgtccc tggctccaag    1140
   aaagtcatcc ttgatctgcc cctagtgatt ggcagcaggt ctggtctgag cagccggaca    1200
   tccagcatgg ccagccggac gagctctgag atgagctgga tagacctaaa catcccagat    1260
   accccagaag ctcctccttg ctatatggac atcattcctg aagatcacag actagagagc    1320
   cccaccaccc ctctgctgga cgatgtggac gactctcaag acagccctat ctttatgtac    1380
   gcccctgagt tccagttcat gcccccaccc acttacactg aggtggatcc gtgcgtcctt    1440
   aacaacaaca acaacaacaa caacgtgcag tgagcctgca ggaaatgaag catctgtatt    1500
   agcgcatttc tttctgcctc tctgcttgaa ctccagtgtt tcagagactc agtctctaca    1560
   gcggggaacg ggtacacccc agccgctgac tcctcaagat gggtggcaat cagtaggcgg    1620
   gtctccggct tcaagtggtg cagaccagtg cccgcactgt ggcataggag tgtttgctgg    1680
   gtggatgtca gaacactctt agaaaaattg agacctgacc actttctcgg atgttggaaa    1740
   tgaagaactt gtttgtgttg actgagtcag ggcactgctg accttctggc gttgtctttc    1800
   caaggttttt gttttaaagg gacttttaaa ttgtctaaaa tatcagtaga ccatcatctg    1860
   tgccatgggg gacagagcca atttcaagtc atggccaaaa ttttgtaaga ggagtgtttt 1920
   tgtgtgtttt ttaaagtcag tgttcctttt ttatatcttt acaaagaaaa gaccttccac 1980
   ggctggtgag cacgcagcct gtgaaattcg gggcagctgc tccaagttga cttcaccctg 2040
   ggagcagtag tagctgtgcc cactgacggc cataaaagcc attttacagc cagttgcact   2100
   gtgttctctt gtaagcataa tcagatggga gaatctgtta tttccctgta accccttgga   2160
   attgattcta aggtgatgtt cttagcactt tagcttgtca attttgtttt agtctccgtt 2220
   atagatgtaa gctccaccag tctcttaagg attaagccca gtgacttgga gggtgggggt 2280
   tagggtctct atccctgaac attgtagacc caggctggcc tgagagatcc acctgcctct 2340
   gcctcctgag tgctgcgatc aaaggcccag cttggttatt gcttttgagg ctttctccca 2400
   acgcacagac ttgtgtaatt ctaacactaa tcctgtgaag ggttgtggtt gacagctgga 2460
   gcctgggtga cattctacat tgagatgccc cagcactgat cggggcacag aagcccccag 2520
   accccatttc ctgtccagtg ttgggagaaa gtgctgcttt cactgtggcc tcagccctgg 2580
   ctcggaagct cactaagcct tagcactttg tcctgtgtca gctccacctg agaactgtgc 2640
   agccagaatg tctgcgagct gatggaggtt tcggttttgt tgtttttgta ttttgtgtat 2700
   ctttttgtat gattaaaaac tatattttct acttatccaa atatattttc accccaaagt 2760
   ggggttatcc tttgtaaaaa aaaataaagt tttttaatga c 2801
<210> 416
   <211> 2806
   <212> DNA
   <213> Mus Musculus
<400> 416
   acactctcct cctctggtct cggggtttcc agagtttctc cagttgcgga agacagctgt     60
   tatttttctc ctgaaagctt ttggcacagc cggcaggctg aaacttccag gcaccttttg    120
   gaaaagttgt tagggtttgt ttgaagcttt ctttacattt tcgtttgggt tttcaagccc    180
   tgactttacg gaggcgagct cttcgtttgc tttgaagggt tcttaaagat ttttttcctc    240
   tccggctttc gtttttcttg aacccactcg gctcaatcat ggtgatgttc aagaagatca    300
   agtcttttga ggtggtcttc aacgaccccg agaaggtgta cggcagcggg gagaaggtgg    360
   ccggacgggt aatagtggaa gtgtgtgaag ttacccgagt caaagccgtc aggatcctgg    420
   cttgcggcgt ggccaaggtc ctgtggatgc aagggtctca gcagtgcaaa cagactttgg    480
   actacttgcg ctatgaagac acacttctcc tagaagagca gcctacaggt gagaacgaga    540
   tggtgatcat gaggcctgga aacaaatatg agtacaagtt cggcttcgag cttcctcaag    600
   ggcccctggg aacatccttt aaaggaaaat atggttgcgt agactactgg gtgaaggctt    660
   ttctcgatcg tcccagccag ccaactcaag aggcaaagaa aaacttcgaa gtgatggatc    720
   tagtggatgt caatacccct gacctaatgg caccagtgtc tgccaaaaag gagaagaaag    780
   tttcctgcat gttcattcct gatggacgtg tgtcagtctc tgctcgaatt gacagaaaag 840
   gattctgtga aggtgatgac atctccatcc atgctgactt tgagaacacg tgttcccgaa 900
   tcgtggtccc caaagcggct attgtggccc gacacactta ccttgccaat ggccagacca 960
   aagtgttcac tcagaagctg tcctcagtca gaggcaatca cattatctca gggacttgcg 1020
   catcgtggcg tggcaagagc ctcagagtgc agaagatcag accatccatc ctgggctgca 1080
   acatcctcaa agtcgaatac tccttgctga tctacgtcag tgtccctggc tccaagaaag 1140
   tcatccttga tctgccccta gtgattggca gcaggtctgg tctgagcagc cggacattca 1200
   gcatggccag ccggacgagc tctgagatga gctggataga cctaaacatc ccagataccc 1260
   cagaagctcc tccttgctat atggacatca ttcctgaaga tcacagacta gagagcccca 1320
   ccacccctct gctggacgat gtggacgact ctcaagacag tcctatcttt atgtacgccc 1380
   ctgagttcca gttcatgccc ccacccactt acactgaggt ggatccgtgc gtccttaaca 1440
   acaacaacaa caacaacaac gtgcagtgag cctgcaggaa atgaagcatc tgtattagcg 1500
   catttctttc tgcctctctg cttgaactcc agtgtttcag agactcagtc tctacagcgg 1560
   ggaacgggta caccccagcc gctgactcct caagatgggt ggcaatcagt aggcgggtct 1620
   ccggcttcaa gtggtgcaga ccagtgcccg cactgtggca taggagtgtt tgctgggtgg 1680
   atgtcagaac actcttagaa aaattgagac ctgaccactt tctcggatgt tggaaatgaa 1740
   gaacttgttt gtgttgactg agtcagggca ctgctgacct tctggcgttg tctttccaag 1800
   gtttttgttt taaagggact tttaaattgt ctaaaatatc agtagaccat catctgtgcc 1860
   atgggggaca gagccaattt caagtcatgg ccaaaatttt gtaagaggag tgtttttgtg 1920
   tgttttttaa agtcagtgtt ccttttttat atctttacaa agaaaagacc ttccacggct 1980
   ggtgagcacg cagcctgtga aattcggggc agctgctcca agttgacttc accctgggag 2040
   cagtagtagc tgtgcccact gacggccata aaagccattt tacagccagt tgcactgtgt 2100
   tctcttgtaa gcataatcag atgggagaat ctgttatttc cctgtaaccc cttggaattg 2160
   attctaaggt gatgttctta gcactttagc ttgtcaattt tgttttagtc tccgttatag 2220
   atgtaagctc caccagtctc ttaaggatta agcccagtga cttggagggt gggggttagg 2280
   gtctctatcc ctgaacattg tagacccagg ctggcctgag agatccacct gcctctgcct 2340
   cctgagtgct gcgatcaaag gcccagcttg gttattgctt ttgaggcttt ctcccaacgc 2400
   acagacttgt gtaattctaa cactaatcct gtgaagggtt gtggttgaca gctggagcct 2460
   gggtgacatt ctacattgag atgccccagc actgatcggg gcacagaagc ccccagaccc 2520
   catttcctgt ccagtgttgg gagaaagtgc tgctttcact gtggcctcag ccctggctcg 2580
   gaagctcact aagccttagc actttgtcct gtgtcagctc cacctgagaa ctgtgcagcc 2640
   agaatgtctg cgagctgatg gaggtttcgg ttttgttgtt tttgtatttt gtgtatcttt 2700
   ttgtatgatt aaaaactata ttttctactt atccaaatat attttcaccc caaagtgggg   2760
   ttatcctttg taaaaaaaaa taaagttttt taatgacaaa aataaa                  2806
<210> 417
   <211> 75
   <212> DNA
   <213> artificial
<220>
   <223> sequence for preparation of siRNA or shRNA targeting TXNIP expression
<400> 417
   ggatcccaca tgcaggaaac tttcttcttt gatatccgag aagaaagttt cctgcatgtt     60
   tttttccaaa agctt                                                      75